# EUROPEAN PATENT SPECIFICATION

(11) **EP 3 448 992 B1**
(45) Date of publication and mention of the grant of the patent: **29.09.2021**
(21) Application number: 17723031.5
(22) Date of filing: 28.04.2017
(51) Int. Cl.: C12N 9/50, C12N 9/36, C07K 14/46, C12N 15/62

(54) **ANTIMICROBIAL AGENTS AGAINST SALMONELLA BACTERIA**
ANTIMIKROBIELLE MITTEL GEGEN SALMONELLENBAKTERIEN
AGENTS ANTIMICROBIENS CONTRE DES BACTÉRIES SALMONELLA

(30) Priority: 28.04.2016 EP 16167543
(43) Date of publication of application: 06.03.2019
(73) Proprietor: Lysando AG, 9497 Triesenberg (LI)
(72) Inventor: GRIESSL, Martin, 93155 Hohenschambach (DE); BIEBL, Manfred, 93083 Obertraubling (DE); SCHIRMEIER, Eva, 93053 Regensburg (DE)
(74) Representative: Rückerl, Florian
(86) International application number: PCT/EP2017/060264
(87) International publication number: WO 2017/186942

(56) References cited:
- WO-A1-2015/155244
- RU-C2- 2 547 584
- DATABASE GENPEPT [Online] NCBI; 8 December 2008 (2008-12-08), "lysozyme [Escherichia phage T5]", XP002762494, retrieved from NCBI accession no. GENPEPT:YP_006868 Database accession no. YP_006868
- CLAUDIA MONTEIRO ET AL: "A 17-mer Membrane-Active MSI-78 Derivative with Improved Selectivity toward Bacterial Cells", MOLECULAR PHARMACEUTICS, vol. 12, no. 8, 11 June 2015 (2015-06-11), pages 2904-2911, XP055307285, US ISSN: 1543-8384, DOI: 10.1021/acs.molpharmaceut.5b00113
- LEGOTSKY SERGEY A ET AL: "Peptidoglycan degrading activity of the broad-range Salmonella bacteriophage S-394 recombinant endolysin", BIOCHIMIE, MASSON, PARIS, FR, vol. 107, 18 September 2014 (2014-09-18), pages 293-299, XP029103941, ISSN: 0300-9084, DOI: 10.1016/J.BIOCHI.2014.09.017
- BRIERS YVES ET AL: "Breaking barriers: expansion of the use of endolysins as novel antibacterials against Gram-negative bacteria", FUTURE MICROBIOLOGY, LONDON : FUTURE MEDICINE LTD, 2006, GB, vol. 10, no. 3, 1 January 2015 (2015-01-01), pages 377-390, XP008180525, ISSN: 1746-0921, DOI: 10.2217/FMB.15.8
- Y. BRIERS ET AL: "Engineered Endolysin-Based "Artilysins" To Combat Multidrug-Resistant Gram-Negative Pathogens", MBIO, vol. 5, no. 4, 1 July 2014 (2014-07-01), pages e01379-14, XP055307172, US ISSN: 2150-7511, DOI: 10.1128/mBio.01379-14

## Description

The present invention relates to the field of antimicrobial agents active against *Salmonella* bacteria. In particular, the present invention relates to polypeptides comprising a first and a second amino acid sequence, wherein the first amino acid sequence is an endolysin, and wherein the second amino acid sequence is an antimicrobial peptide, cationic peptide, hydrophobic peptide, amphipathic peptide or sushi peptide, and wherein the polypeptide comprises at least one sequence selected from the following group of sequences: i) an amino acid sequence according to SEQ ID NO:1, and ii) derivatives of SEQ ID NO: 1 exhibiting at least 80% sequence identity with SEQ ID NO:1, with the proviso that the polypeptide may not comprise the sequence of SEQ ID NO:5, if the polypeptide comprises ii), but none of a) an amino acid sequence according to SEQ ID NO:3; b) a derivative of SEQ ID NO:3 exhibiting at least 77% sequence identity with SEQ ID NO:3; c) an amino acid sequence according to SEQ ID NO:4; with the proviso that the polypeptide may in this case not comprise the sequence of SEQ ID NO:6 in parallel; and d) a derivative of SEQ ID NO:4 exhibiting at least 80% sequence identity with SEQ ID NO:4, and wherein the polypeptide degrades the peptidoglycan of *Salmonella* bacteria. In addition, the present invention relates to nucleic acids encoding such polypeptides, vectors comprising such nucleic acids, and corresponding host cells. Finally, the present invention relates to applications of the inventive polypeptides, nucleic acids, vectors, and/or host cells, in particular in the pharmaceutical field and in the field of food and feed.

Bacterial pathogens represent a significant threat for human health. Although various types of agents having bactericidal or bacteriostatic activity are known in the art (e.g. antibiotics), microbial resistance to these, in particular to antibiotics, is steadily increasing. One of the pathogens representing a health concern are bacteria of the genus *Salmonella.* A large number of infections caused by *Salmonella* bacteria are due to ingestion of contaminated food. Since increasing resistance renders the use of antibiotics in, e.g., feed additives problematic, there is a constant demand for new antimicrobial agents to control the number of *Salmonella* bacteria, e.g. in food and feed.

Bacteria of the genus *Salmonella* are Gram-negative bacteria. Gram-negative bacteria possess an outer membrane, with its characteristic asymmetric bilayer as a hallmark. The outer membrane bilayer consists of an inner monolayer containing phospholipids (primarily phosphatidyl ethanolamine) and an outer monolayer that is mainly composed of a single glycolipid, lipopolysaccharide (LPS). There is an immense diversity of LPS structures in the bacterial kingdom and the LPS structure may be modified in response to prevailing environmental conditions. The stability of the LPS layer and interaction between different LPS molecules is mainly achieved by the electrostatic interaction of divalent ions (Mg²⁺, Ca²⁺) with the anionic components of the LPS molecule (phosphate groups in the lipid A and the inner core and carboxyl groups of KDO). Furthermore, the dense and ordered packing of the hydrophobic moiety of lipid A, favoured by the absence of unsaturated fatty acids, forms a rigid structure with high viscosity. This makes it less permeable for lipophilic molecules and confers additional stability to the outer membrane (OM).

The present invention makes use of endolysins. Endolysins are peptidoglycan hydrolases typically encoded by bacteriophages (or bacterial viruses). They are synthesized during late gene expression in the lytic cycle of phage multiplication and mediate the release of progeny virions from infected cells through degradation of the bacterial peptidoglycan. They are either β(1,4)-glycosyl hydrolases (lysozymes), transglycosylases, amidases or endopeptidases. Antimicrobial application of endolysins was already suggested in 1991 by Gasson (GB2243611). Although the killing capacity of endolysins has been known for a long time, the use of these enzymes as antibacterials was ignored due to the success and dominance of antibiotics. Only after the appearance of multiple antibiotic resistant bacteria this simple concept of combating human pathogens with endolysins received interest. A compelling need to develop totally new classes of antibacterial agents emerged and endolysins used as 'enzybiotics' - a hybrid term of 'enzymes' and 'antibiotics' - perfectly met this need. In 2001, Fischetti and coworkers demonstrated for the first time the therapeutic potential of bacteriophage Cl endolysin towards group A streptococci (Nelson et al., 2001, Proc. Natl. Acad. Sci. U. S. A. 98:4107-4112). Since then many publications have established endolysins as an attractive and complementary alternative to control bacterial infections of Gram-positive bacteria. Subsequently different endolysins against other Gram-positive pathogens such as *Streptococcus pneumoniae* (Loeffler et al., 2001, Science 294:2170-2172), *Bacillus anthracis* (Schuch et al., 2002; Nature 418:884-889), *S. agalactiae* (Cheng et al., 2005; Antimicrob Agents Chemother. 2005 Jan;49(1):111-117) and *Staphylococcus aureus* (Rashel et al, 2007; J Infect Dis. 2007 Oct 15;196(8):1237-1247) have proven their efficacy as enzybiotics. Legotsky et al. (Biochimie, 2014, 107, 293-299) disclose the peptidoglycan degrading activity of the broad-range *Salmonella* bacteriophage S-394 recombinant endolysin. Nowadays, the most important challenge of endolysin therapy lies in the insensitivity of Gram-negative bacteria towards the exogenous action of endolysins, since the outer membrane shields the access of endolysins from the peptidoglycan. This currently prevents the expansion of the range of effective endolysins to important Gram-negative pathogens, such as bacteria of the genus *Salmonella.*

In the art combinations of endolysins with further amino acid sequence stretches have been described to create new antimicrobial agents. WO 2015/071436 discloses fusions of peptides with derivatives of endolysin KZ144, which show activity towards *E. coli, P. aeruginosa,* and *C. jejuni.* Briers et al. (MBio; 2014;5(4):e01379-14) report that fusion proteins of the endolysins OBPgp279 and PVP-SE1gp146 with certain peptides yield antimicrobial agents with moderate activity against *Salmonella* Typhimurium LT 2 bacteria. WO 2015/155244 discloses also various of these fusion proteins for the treatment of chronic bacterial infections.

Nonetheless, there is still a constant need for new antibacterial agents active bacteria of the Genus *Salmonella.* Preferably, said agents are active against a diverse set of *Salmonella* strains, exhibit an increased activity and/or are sufficiently (heat) stable for technical applications.

This object is solved by the subject matter defined in the claims and set forth below.

The term "polypeptide" as used herein refers in particular to a polymer of amino acids linked by peptide bonds in a specific sequence. The amino acid residues of a polypeptide may be modified by e.g. covalent attachments of various groups such as carbohydrates and phosphate. Other substances may be more loosely associated with the polypeptide, such as heme or lipid, giving rise to conjugated polypeptides which are also comprised by the term "polypeptide" as used herein. The term as used herein is intended to encompass also proteins. Thus, the term "polypeptide" also encompasses for example complexes of two or more amino acid polymer chains. The term "polypeptide" does encompass embodiments of polypeptides which exhibit optionally modifications typically used in the art, e.g. biotinylation, acetylation, pegylation, chemical changes of the amino-, SH- or carboxyl-groups (e.g. protecting groups) etc.. As will become apparent from the description below, the polypeptide according to the present invention may also be a fusion protein, i.e. linkage of at least two amino acid sequences which do not occur in this combination in nature. The term "polypeptide", as used herein, is not limited to a specific length of the amino acid polymer chain, but typically the polypeptide will exhibit a length of more than about 50 amino acids, more than about 100 amino acids or even more than about 150 amino acids. Usually, but not necessarily, a typical polypeptide of the present invention will not exceed about 750 amino acids in length.

The term "endolysin" as used herein refers to a bacteriophage-derived enzyme which is suitable to hydrolyse bacterial cell walls. Endolysins comprise at least one "enzymatically active domain" (EAD) having at least one of the following activities: endopeptidase, chitinase, T4 like muraminidase, lambda like muraminidase, N-acetyl-muramoyl-L-alanine-amidase (amidase), muramoyl-L-alanine-amidase, muramidase , lytic transglycosylase (C), lytic transglycosylase (M), N-acetyl-muramidase (lysozyme), N-acetyl-glucosaminidase or transglycosylases as e.g. KZ144. In addition, the endolysins may contain also regions which are enzymatically inactive, and bind to the cell wall of the host bacteria, the so-called CBDs (cell wall binding domains). The term "endolysin" also encompasses enzymes which comprise modifications and/or alterations vis-a-vis naturally occurring endolysins. Such alterations and/or modifications may comprise mutations such as deletions, insertions and additions, substitutions or combinations thereof and/or chemical changes of the amino acid residues. Particularly preferred chemical changes are biotinylation, acetylation, pegylation, chemical changes of the amino-, SH- or carboxyl- groups. Said endolysins exhibit on a general level the lytic activity of the respective wild-type endolysin. However, said activity can be the same, higher or lower as the activity of the respective wild-type endolysin. Said activity can be for example at least about 50, 60, 70, 80, 90, 100, 110, 120, 130, 140, 150, 160, 170, 180, 190 or at least about 200 % of the activity of the respective wild-type endolysin or even more. The activity can be measured by assays well known in the art by a person skilled in the art as e.g. the plate lysis assay or the liquid lysis assay which are e.g. described in Briers et al. (J. Biochem. Biophys Methods; 2007; 70: 531-533) or Donovan et al. (J. FEMS Microbiol Lett. 2006 Dec;265(1).

The term "fragment" as used herein refers to an amino acid sequence which is N-terminally, C-terminally, and/or on both termini truncated with respect to the respective reference sequence, for example a given SEQ ID NO. Thus, a fragment of an amino acid sequence as used herein is an amino acid sequence which is at least one amino acid shorter than the respective reference sequence. A fragment of an amino acid sequence as used herein is preferably an amino acid sequence which is at most 20, more preferably at most 19, more preferably at most 18, more preferably at most 17, more preferably at most 16, more preferably at most 15, more preferably at most 14, more preferably at most 13, more preferably at most 12, more preferably at most 11, more preferably at most 10, more preferably at most 9, more preferably at most 8, more preferably at most 7, more preferably at most 6, more preferably at most 5, more preferably at most 4, more preferably at most 3, more preferably at most 2, most preferably 1 amino acid residue shorter than the respective reference amino acid sequence. The fragment may for example exhibit vis-a-vis the reference sequence a truncation of 1, 2, 3, 4, 5, 6, 7, 8, 9, 10, 11, 12, 13, 14, 15, 16, 17, 18, 19 or 20 amino acids at the N-terminus, the C-terminus or both. It is understood that a polypeptide comprising a fragment of a given amino acid sequence does not comprise the full length of said reference amino acid sequence.

The term "derivative", as used herein, refers to an amino acid sequence which exhibits, in comparison to the respective reference sequence, one or more additions, deletions, insertions, and/or substitutions and combinations thereof. This includes for example combinations of deletions/insertions, insertions/deletions, deletions/additions, additions/deletions, insertion/ additions, additions/insertions etc. A person skilled in the art will however understand that the presence of an amino acid residue at a certain position of the derivative sequence which is different from the one that is present at the respective same position in the reference sequence is not a combination of, for example, a deletion and a subsequent insertion at the same position but is a substitution as defined herein. Rather, if reference is made herein to combinations of one or more of additions, deletions, insertions, and substitutions, then combination of changes at distinct positions in the sequence are intended, e.g. an addition at the N-terminus and an intrasequential deletion. Such derived sequence will exhibit a certain level of sequence identity with the respective reference sequence, for example a given SEQ ID NO, which is preferably at least 60%, such as at least 75%, at least 80%, at least 85%, at least 90%, at least 95%, at least 96%, at least 97%, at least 98% or at least 99%. Preferred derivatives are fragments of the parent molecule, for example a given SEQ ID NO, retaining the activity of the parent molecule, i.e. exhibiting on a general level same activity as the respective parent molecule. However, said activity can be the same, higher or lower as the respective parent molecule. Also preferred derivatives are those resulting from conservative amino acid substitutions within the parent sequence, for example a given SEQ ID NO, again retaining the activity of the parent molecule on a general level.

As used herein, the term "% sequence identity", has to be understood as follows: Two sequences to be compared are aligned to give a maximum correlation between the sequences. This may include inserting "gaps" in either one or both sequences, to enhance the degree of alignment. A % identity may then be determined over the whole length of each of the sequences being compared (so-called global alignment), that is particularly suitable for sequences of the same or similar length, or over shorter, defined lengths (so-called local alignment), that is more suitable for sequences of unequal length. In the above context, an amino acid sequence having a "sequence identity" of at least, for example, 95% to a query amino acid sequence, is intended to mean that the sequence of the subject amino acid sequence is identical to the query sequence except that the subject amino acid sequence may include up to five amino acid alterations per each 100 amino acids of the query amino acid sequence. In other words, to obtain an amino acid sequence having a sequence of at least 95% identity to a query amino acid sequence, up to 5% (5 of 100) of the amino acid residues in the subject sequence may be inserted or substituted with another amino acid or deleted. Methods for comparing the identity and homology of two or more sequences are well known in the art. The percentage to which two sequences are identical can for example be determined by using a mathematical algorithm. A preferred, but not limiting, example of a mathematical algorithm which can be used is the algorithm of Karlin et al. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et al. (1997), Nucleic Acids Res, 25:3389-3402), accessible through the home page of the NCBI at world wide web site ncbi.nlm.nih.gov) and FASTA (Pearson (1990), Methods Enzymol. 83, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al, 1984, Nucleic Acids Res., 387-395), for example the programs BESTFIT and GAP, may be used to determine the % identity between two polypeptide sequences. BESTFIT uses the "local homology" algorithm of (Smith and Waterman (1981), J. Mol. Biol. 147, 195-197.) and finds the best single region of similarity between two sequences. If herein reference is made to an amino acid sequence sharing a particular extent of sequence identity to a reference sequence, then said difference in sequence is preferably due to conservative amino acid substitutions. Preferably, such sequence retains the activity of the reference sequence, e.g. albeit maybe at a slower rate. In addition, if reference is made herein to a sequence sharing "at least" at certain percentage of sequence identity, then 100% sequence identity are preferably not encompassed.

"Conservative amino acid substitutions", as used herein, may occur within a group of amino acids which have sufficiently similar physicochemical properties, so that a substitution between members of the group will preserve the biological activity of the molecule (see e.g. Grantham, R. (1974), Science 185, 862-864). Particularly, conservative amino acid substitutions are preferably substitutions in which the amino acids originate from the same class of amino acids (e.g. basic amino acids, acidic amino acids, polar amino acids, amino acids with aliphatic side chains, amino acids with positively or negatively charged side chains, amino acids with aromatic groups in the side chains, amino acids the side chains of which can enter into hydrogen bridges, e.g. side chains which have a hydroxyl function, etc.). Conservative substitutions are in the present case for example substituting a basic amino acid residue (Lys, Arg, His) for another basic amino acid residue (Lys, Arg, His), substituting an aliphatic amino acid residue (Gly, Ala, Val, Leu, lie) for another aliphatic amino acid residue, substituting an aromatic amino acid residue (Phe, Tyr, Trp) for another aromatic amino acid residue, substituting threonine by serine or leucine by isoleucine. Further conservative amino acid exchanges will be known to the person skilled in the art.

The term "deletion" as used herein refers preferably to the absence of 1, 2, 3, 4, 5 (or even more than 5) continuous amino acid residues in the derivative sequence in comparison to the respective reference sequence, either intrasequentially or at the N- or C-terminus. A derivative of the present invention may exhibit one, two or more of such deletions.

The term "insertion" as used herein refers preferably to the additional intrasequential presence of 1, 2, 3, 4, 5 (or even more than 5) continuous amino acid residues in the derivative sequence in comparison to the respective reference sequence. A derivative of the present invention may exhibit one, two or more of such insertions.

The term "addition" as used herein refers preferably to the additional presence of 1, 2, 3, 4, 5 (or even more than 5) continuous amino acid residues at the N- and/or C-terminus of the derivative sequence in comparison to the respective reference sequence.

The term "substitution" as used herein refers to the presence of an amino acid residue at a certain position of the derivative sequence which is different from the amino acid residue which is present or absent at the corresponding position in the reference sequence. A derivative of the present invention may exhibit 1, 2, 3, 4, 5, 6, 7, 8, 9, 10, 11, 12, 13, 14, 15, 16, 17, 18, 19, 20, or more of such substitutions. As mentioned above, preferably such substitutions are conservative substitutions.

The term "cell wall" as used herein refers to all components that form the outer cell enclosure of Gram-negative bacteria and thus guarantee their integrity. In particular, the term "cell wall" as used herein refers to peptidoglycan, the outer membrane of the Gram-negative bacteria with the lipopolysaccharide, the bacterial cell membrane, but also to additional layers deposited on the peptidoglycan as e.g. capsules, outer protein layers or slimes.

The term "second amino acid sequence", as used herein refers an amino acid subsequence within the amino acid sequence of the polypeptide of the invention. Said sequence may be the sequence of a cationic peptide, a polycationic peptide, an amphipathic peptide, a hydrophobic peptide, a sushi peptide and/or an antimicrobial peptide. The term does not refer to conventional tags like His-tags, such as His5-tags, His6-tags, His7-tags, His8-tags, His9-tags, His10-tags, His11-tags, His12-tags, His16-tags and His20-tags, Strep-tags, Avi-tags, Myc-tags, Gst-tags, JS-tags, cystein-tags, FLAG-tags or other tags known in the art, thioredoxin or maltose binding proteins (MBP). Preferably, the second amino acid sequence has as a length of at least about 6 to at most about 50, preferably at most about 39 amino acid residues.

The terms "first amino acid sequence" and "second amino acid sequence", as used herein, do not imply an inherent order of the sequences within the inventive polypeptide, i.e. the second amino acid sequence may be N-terminal of the first amino acid sequence or C-terminal of the first amino acid sequence.

As used herein, the term "cationic peptide" refers preferably to a peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides, but also includes cationic peptides which comprise for example less than 20%, preferably less than 10% positively charged amino acid residues.

The term "polycationic peptide" as used herein refers preferably to a peptide composed of mostly positively charged amino acid residues, in particular lysine and/or arginine residues. A peptide is composed of mostly positively charged amino acid residues if at least about 20, 30, 40, 50, 60, 70, 75, 80, 85, 90, 95 or about 100 % of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term, "antimicrobial peptide" (AMP) as used herein refers preferably to any naturally occurring peptide that has microbicidal and/or microbistatic activity on, for example, bacteria, viruses, fungi, yeasts, mycoplasma and protozoa. Thus, the term "antimicrobial peptide" as used herein refers in particular to any peptide having anti-bacterial, anti-fungal, anti-mycotic, anti-parasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties. Preferred are anti-bacterial peptides. The antimicrobial peptide may be a member of the RNase A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in radish, silk moth, wolf spider, frog, preferably in Xenopus laevis, Rana frogs, more preferably in Rana catesbeiana, toad, preferably Asian toad Bufo bufo gargarizans, fly, preferably in Drosophila, more preferably in Drosophila melanogaster, in Aedes aegypti, in honey bee, bumblebee, preferably in Bombus pascuorum, flesh fly, preferably in Sarcophaga peregrine, scorpion, horseshoe crab, catfish, preferably in Parasilurus asotus, cow, pig, sheep, porcine, bovine, monkey and human. As used herein, an "antimicrobial peptide" (AMP) may in particular be a peptide which is not a cationic peptide, polycationic peptide, amphipathic peptide, sushi peptide, defensins, and hydrophobic peptide, but nevertheless exhibits antimicrobial activity.

The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "defensin" as used herein refers to a peptide present within animals, preferably mammals, more preferably humans, wherein the defensin plays a role in the innate host defense system as the destruction of foreign substances such as infectious bacteria and/or infectious viruses and/or fungi. A defensin is a non-antibody microbicidal and/or tumoricidal protein, peptide or polypeptide. Examples for "defensins" are "mammalian defensins," alpha-defensins, beta-defensins, indolicidin and magainins. The term "defensins" as used herein refers both to an isolated form from animal cells or to a synthetically produced form, and refers also to variants which substantially retain the cytotoxic activities of their parent proteins, but whose sequences have been altered by insertion or deletion of one or more amino acid residues.

The term "amphipathic peptide" as used herein refers to synthetic peptides having both hydrophilic and hydrophobic functional groups. Preferably, the term "amphipathic peptide" as used herein refers to a peptide having a defined arrangement of hydrophilic and hydrophobic groups e.g. amphipathic peptides may be e.g. alpha helical, having predominantly non polar side chains along one side of the helix and polar residues along the rest of its surface.

The term "hydrophobic group" as used herein refers preferably to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a non-aqueous environment. Examples of amino acid residues with hydrophobic side chains are valine, isoleucine, leucine, methionine, phenylalanine, tryptophan, cysteine, alanine, tyrosine, and proline residues

The term "hydrophobic peptide" as used herein refers to a hydrophobic peptide, which is preferably composed of mostly amino acid residues with hydrophobic groups. Such peptide is preferably composed of mostly hydrophobic amino acid residues, i.e. at least about 20, 30, 40, 50, 60, 70, 75, 80, 85, 90, 95 or at least about 100 % of the amino acid residues are hydrophobic amino acid residues. The amino acid residues being not hydrophobic are preferably neutral and preferably not hydrophilic.

As used herein, the term "tag" refers to an amino acid sequence, which is typically in the art fused to or included in another amino acid sequence for a) improving expression of the overall amino acid sequence or polypeptide, b) facilitating purification of the overall amino acid sequence or polypeptide, c) facilitating immobilisation of the overall amino acid sequence or polypeptide, and/or d) facilitating detection of the overall amino acid sequence or polypeptide. Examples for tags are His tags, such as His5-tags, His6-tags, His7-tags, His8-tags, His9-tags, His10-tags, His11-tags, His12-tags, His16-tags and His20-tags, Strep-tags, Avi-tags, Myc-tags, GST-tags, JS-tags, cystein-tags, FLAG-tags, HA-tags, thioredoxin or maltose binding proteins (MBP), CAT, GFP, YFP, etc. The person skilled in the art will know a vast number of tags suitable for different technical applications. The tag may for example make such tagged polypeptide suitable for e.g. antibody binding in different ELISA assay formats or other technical applications.

The term "comprising" as used herein shall not be construed as being limited to the meaning "consisting of' (i.e. excluding the presence of additional other matter). Rather, "comprising" implies that optionally additional matter may be present. The term "comprising" encompasses as particularly envisioned embodiments falling within its scope "consisting of' (i.e. excluding the presence of additional other matter) and "comprising but not consisting of' (i.e. requiring the presence of additional other matter), with the former being more preferred.

The inventors of the present invention have surprisingly found that the endolysin according to SEQ ID NO:1, and derivatives thereof as defined in claim 1, are extremely useful components when designing antimicrobial agents against bacteria of the Genus *Salmonella.* Such compounds show increased activity, for example against *Salmonella* Typhimurium bacteria.

Therefore, the present invention relates in a first aspect to a polypeptide comprising a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1;
ii) a derivative of SEQ ID NO:1 exhibiting at least 80% sequence identity with SEQ ID NO:1, with the proviso that the polypeptide may not comprise the sequence of SEQ ID NO:5 if the polypeptide comprises ii), but none of:
   a) an amino acid sequence according to SEQ ID NO:3;
   b) a derivative of SEQ ID NO:3 exhibiting at least 77% sequence identity with SEQ ID NO:3;
   c) an amino acid sequence according to SEQ ID NO:4, with the proviso that the polypeptide may in this case not comprise the sequence of SEQ ID NO:6 in parallel; and
   d) a derivative of SEQ ID NO:4 exhibiting at least 80% sequence identity with SEQ ID NO:4, and
   wherein the polypeptide degrades the peptidoglycan of *Salmonella* bacteria.

The amino acid sequence according to SEQ ID NO:1 and derivatives thereof are examples for the first amino acid sequence, i.e. the endolysin, SEQ ID NO:3, SEQ ID NO:4 and respective derivatives are examples for the second amino acid sequence.

Derivatives of SEQ ID NO:1 exhibit preferably at least 85%, at least 87,5%, at least 90%, at least 91%, at least 92%, at least 93%, at least 94%, at least 95%, at least 96%, at least 97%, at least 98%, at least 99%, or more than 99% sequence identity with SEQ ID NO:1. An example for a derivative of SEQ ID NO:1 is the amino acid sequence according to SEQ ID NO:7. In other embodiments, the derivative is not an amino acid sequence according to SEQ ID NO:7. Particularly preferred derivatives exhibit at least 98%, at least 99%, or more than 99% sequence identity with SEQ ID NO:1. As mentioned before, a polypeptide of the present invention comprising a derivative of SEQ ID NO:1 may not comprise the sequence of SEQ ID NO:5, if the polypeptide of the invention does not comprise in parallel an amino acid sequence according to SEQ ID NO:3, a derivative of SEQ ID NO:3 as defined herein, an amino acid sequence according to SEQ ID NO:4, and/or a derivative of SEQ ID NO:4 as defined herein. This proviso applies for the polypeptides of the invention per se, but not necessarily for other aspects of the present invention, e.g. methods and uses employing such polypeptide, in particular when dealing with Salmonella bacteria. However, said proviso may of course also apply to the other aspects, e.g. the methods and uses of such polypeptide.

A suitable sequences for the first amino acid sequence of the polypeptide according to the present invention is for example SEQ ID NO:31. A further example is the amino acid sequence of SEQ ID NO:7. In certain embodiments of the invention, in particular where the polypeptide comprises an amino acid sequence according to SEQ ID NO:3, a derivative of SEQ ID NO:3 as defined herein, an amino acid sequence according to SEQ ID NO:4, and/or a derivative of SEQ ID NO:4 as defined herein, the amino acid sequence according to SEQ ID NO:5 is also a suitable sequence for the first amino acid sequence of the polypeptide according to the present invention.

In preferred embodiments of the invention the the second amino acid sequence is selected from the group consisting of:
i) an amino acid sequence according to SEQ ID NO:3;
ii) a derivative of SEQ ID NO:3 exhibiting at least 77% sequence identity with SEQ ID NO:3;
iii) an amino acid sequence according to SEQ ID NO:4; and
iv) a derivative of SEQ ID NO:4 exhibiting at least 80% sequence identity with SEQ ID NO:4.

Derivatives of SEQ ID NO:3 exhibit preferably at least 81%, at least 86%, at least 90%, or at least 95% sequence identity with SEQ ID NO:3. Derivatives of SEQ ID NO:3 are preferably fragments of SEQ ID NO:3 and/or exhibit conservative amino acid substitutions vis-a-vis SEQ ID NO:3. Particularly preferred derivatives of SEQ ID NO:3 are fragments of SEQ ID NO:3. For example, a 17-mer fragment (SEQ ID NO:37) of SEQ ID NO:3 has been described in the art (Monteiro et al, Mol Pharm., 2015; 12(8):2904-11) which exhibits improved selectivity.

Derivatives of SEQ ID NO:4 exhibit preferably at least 83%, at least 86%, at least 90%, at least 93%, or at least 96% sequence identity with SEQ ID NO:4. Derivatives of SEQ ID NO:4 are preferably fragments of SEQ ID NO:4 and/or exhibit conservative amino acid substitutions vis-a-vis SEQ ID NO:4.

Preferred combinations of first and second amino acid sequence in the polypeptide according to the present invention are polypeptides, wherein the first amino acid sequence is SEQ ID NO:1 or said derivative thereof, and wherein the second amino acid sequence is SEQ ID NO:3 or said derivative thereof. A preferred combination is also wherein the first amino acid sequence is SEQ ID NO:1 or said derivative thereof, and wherein the second amino acid sequence is SEQ ID NO:4 or said derivative thereof.

Particularly preferred combinations are wherein
i) the first amino acid sequence is SEQ ID NO:1 and the second amino acid sequence is SEQ ID NO:3, and
ii) the first amino acid sequence is SEQ ID NO:1 and the second amino acid sequence is SEQ ID NO:4.

In other embodiments of the invention, where the second amino acid sequence of the inventive polypeptide is neither SEQ ID NO:3 nor SEQ ID NO:4, the second amino acid sequence is selected from the group consisting of an antimicrobial peptide, amphiphatic peptide, cationic peptide, polycationic peptide, hydrophobic peptide, sushi peptide or defensin.

Examples for cationic/ polycationic amino acid sequences are listed in the following table.

**Table 1:**

| **Amino acid sequence** | **Length** | **SEQ ID NO:** |
|---|---|---|
| KRKKRK | 6 | 38 |
| KRXKR | 5 | 39 |
| KRSKR | 5 | 40 |
| KRGSG | 5 | 41 |
| KRKKRKKRK | 9 | 42 |
| RRRRRRRRR | 9 | 43 |
| KKKKKKKK | 8 | 44 |
| KRKKRKKRKK | 10 | 45 |
| KRKKRKKRKKRK | 12 | 46 |
| KRKKRKKRKKRKKR | 14 | 47 |
| KKKKKKKKKKKKKKKK | 16 | 48 |
| KRKKRKKRKKRKKRKKRK | 18 | 49 |
| KRKKRKKRKKRKKRKKRKK | 19 | 50 |
| RRRRRRRRRRRRRRRRRRR | 19 | 51 |
| KKKKKKKKKKKKKKKKKKK | 19 | 52 |
| KRKKRKKRKRSKRKKRKKRK | 20 | 53 |
| KRKKRKKRKRSKRKKRKKRKK | 21 | 54 |
| KRKKRKKRKKRKKRKKRKKRK | 21 | 55 |
| KRKKRKKRKRGSGKRKKRKKRK | 22 | 56 |
| KRKKRKKRKRGSGSGKRKKRKKRK | 24 | 57 |
| KRKKRKKRKKRKKRKKRKKRKKRKK | 25 | 58 |
| KRKKRKKRKRSKRKKRKKRKRSKRKKRKKRK | 31 | 59 |
| KRKKRKKRKRGSGSGKRKKRKKRKGSGSGKRKKRKKRK | 38 | 60 |
| KRKKRKKRKKRKKRKKRKKRKKRKKRKKRKKRKKRKKRK | 39 | 61 |
| KRKKRKKRKRSKRKKRKKRKRSKRKKRKKRKRSKRKKRKKRK | 42 | 62 |

Examples for antimicrobial amino acid sequences which may be used in carrying out the present invention are listed in the following table.

**Table 2:**

| **Peptide** | **Sequence** | **SEQ ID NO** |
|---|---|---|
| LL-37 | | 63 |
| SMAP-29 | RGLRRLGRKIAHGVKKYGPTVLRIIRIAG | 64 |
| Indolicidin | ILPWKWPWWPWRR | 65 |
| Protegrin | RGGRLCYCRRRFCVCVGR | 66 |
| Cecropin P1 | SWLSKTAKKLENSAKKRISEGIAIAIQGGPR | 67 |
| Magainin | GIGKFLHSAKKFGKAFVGEIMNS | 68 |
| Pleurocidin | GWGSFFKKAAHVGKHVGKAALTHYL | 69 |
| Cecropin A (A.aegypti) | | 70 |
| Cecropin A (D. melanogaster) | | 71 |
| Buforin II | TRSSRAGLQFPVGRVHRLLRK | 72 |
| Sarcotoxin IA | | 73 |
| Apidaecin | ANRPVYIPPPRPPHPRL | 74 |
| Ascaphine 5 | GIKDWIKGAAKKLIKTVASHIANQ | 75 |
| Nigrocine 2 | GLLSKVLGVGKKVLCGVSGLVC | 76 |
| Pseudin 1 | GLNTLKKVFQGLHEAIKLINNHVQ | 77 |
| Ranalexin | FLGGLIVPAMICAVTKKC | 78 |
| Melittin | GIGAVLKVLTTGLPALISWIKRKRQQ | 79 |
| Lycotoxin 1 | IWLTALKFLGKHAAKKLAKQQLSKL | 80 |
| Parasin 1 | KGRGKQGGKVRAKAKTRSS | 81 |
| Buforin I | | 82 |
| Dermaseptin 1 | ALWKTMLKKLGTMALHAGKAALGAAADTISQGTQ | 83 |
| Bactenecin 1 | RLCRIVVIRVCR | 84 |
| Thanatin | GSKKPVPIIYCNRRTGKCQRM | 85 |
| Brevinin 1T | VNPIILGVLPKVCLITKKC | 86 |
| Ranateurin 1 | SMLSVLKNLGKVGLGFVACKINIKQC | 87 |
| Esculentin 1 | | 88 |
| Tachyplesin | RWCFRVCYRGICYRKCR | 89 |
| Androctonin | RSVCRQIKICRRRGGCYYKCTNRPY | 90 |
| alpha-defensin | DCYCRIPACIAGERRYGTCIYQGRLWAFCC | 91 |
| beta-defensin | | 92 |
| theta-defensin | GFCRCLCRRGVCRCICTR | 93 |
| defensin (sapecin A) | | 94 |
| Thionin (crambin) | | 95 |
| defensin from radish | | 96 |
| Drosomycin | | 97 |
| Hepcidin | DTHFPICIFCCGCCHRSKCGMCCKT | 98 |
| Bac 5 | | 99 |
| PR-39 | | 100 |
| Pyrrhocoricin | VDKGSYLPRPTPPRPIYNRN | 101 |
| Histatin 5 | DSHAKRHHGYKRKFHEKHHSHRGY | 102 |
| ECP19 | RPPQFTRAQWFAIQHISLN | 103 |
| MSI-594 | GIGKFLKKAKKGIGAVLKVLTTG | 104 |
| TL-ColM | | 105 |
| SBO | KLKKIAQKIKNFFAKLVA | 106 |
| Macedocin | GKNGVFKTISHECHLNTWAFLATCCS | 107 |
| Macedocin (Trunc) | GKNGVFKTISHECHLNTWAFLA | 108 |
| D16 | ACKLKSLLKTLSKAKKKKLKTLLKALSK | 109 |
| CPF-C1 | GFGSLLGKALRLGANVL | 110 |
| TL-ColM(-Met) | ETLTVHAPSPSTNLPSYGNGAFSLSAPHVPGAGP | 111 |
| TM-174E | LISKGWPYLLVVVLGATIYFWGNSNG | 112 |
| ECP45 | | 113 |
| ColicinE3_1-51 (S37F) | | 114 |
| ColicinE3_1-69 (S37F) | | 115 |
| ColicinD_1-53 | | 116 |
| MSI-78 | GIGKFLKKAKKFGKAFVKILKK | 3 |
| Cathelicidin-BF | KFFRKLKKSVKKRAKEFFKKPRVIGVSIPF | 4 |

The second amino acid sequence stretch may be a sushi peptide which is described by Ding JL, Li P, Ho B Cell Mol Life Sci. 2008 Apr;65(7-8):1202-19. The Sushi peptides: structural characterization and mode of action against Gram-negative bacteria. Especially preferred is the sushi 1 peptide according to SEQ ID NO: 117. Other preferred sushi peptides are sushi peptides S1 and S3 and multiples thereof (Tan et al, FASEB J. 2000 Sep;14(12):1801-13).

Preferred hydrophobic peptides are Walmagh1 having the amino acid sequence according to SEQ ID NO: 118 and the hydrophobic peptide having the amino acid sequence Phe-Phe-Val-Ala-Pro (SEQ ID NO: 119).

Preferred amphipathic peptides are α4-helix of T4 lysozyme according to SEQ ID NO: 120 and WLBU2-Variant having the amino acid sequence according to SEQ ID NO: 121 and Walmagh 2 according to SEQ ID NO: 122.

With respect to the arrangement of first and second amino acid sequence within the inventive polypeptide it is preferred if the second amino acid sequence is situated N-terminal of the first amino acid sequence. Preferably, the first and second amino acid sequence are linked to each other directly or via a short linker of 1 to 10 amino acid residues, preferably 1 to 5 amino acid residues, even more preferably 1 to 2 amino acids. Linker sequences are preferably flexible sequences, comprising one or more glycine residues. An example for such linker is the sequence GGGGS (SEQ ID NO: 123).

In particular in cases where the inventive polypeptide is to be recombinantly expressed by a host cell, it is preferred if the inventive polypeptide comprises a methionine residue at the N-terminus.

The inventive polypeptide may comprise additionally one or more tag sequences. Such tag sequence may for example be located at the N- or C-terminus of the inventive polypeptide. In a preferred embodiment, the one or more tag sequence is located on the C-terminal side of the polypeptide, e.g. directly at the C-terminus. The one or more tag sequences may be linked for example directly or via a short linker to the rest of the inventive polypeptide (see above). Numerous examples for tags are known in the art, some of which have already been mentioned above. In the context of the present invention a particularly preferred tag sequence is a His-tag, preferably a His tag according to SEQ ID NO: 124.

The length of the polypeptide according to present invention is in principle not limited, but preferably the length will not be excessively large. Preferably, a polypeptide according to the present invention has an overall length not exceeding about 320 amino acids, preferably not exceeding about 310 amino acids.

A preferred polypeptide according to the invention comprises SEQ ID NO: 125, such as a polypeptide comprising SEQ ID NO: 126 or comprising SEQ ID NO: 127. Also contemplated are derivatives of SEQ ID NO: 125, SEQ ID NO: 126 and SEQ ID NO: 127, e.g. each exhibiting at least 80% sequence identity to the respective reference sequence.

A further preferred polypeptide according to the invention comprises SEQ ID NO: 131, such as a polypeptide comprising SEQ ID NO: 132 or comprising SEQ ID NO: 133. Also contemplated are derivatives of SEQ ID NO: 131, SEQ ID NO: 132 and SEQ ID NO: 133, e.g. each exhibiting at least 80% sequence identity to the respective reference sequence.

Further examples of inventive polypeptides are polypeptides comprising any of the sequences selected from the group consisting of SEQ ID NO: 143, SEQ ID NO: 144, SEQ ID NO: 145, and derivatives of these sequences, e.g. each exhibiting at least 80% sequence identity to the respective reference sequence

A polypeptide according to the present invention is characterized by the ability to degrade the peptidoglycan of *Salmonella* bacteria. The peptidoglycan degrading activity can be measured by assays well known in the art, e.g. by muralytic assays in which the outer membrane of gram negative bacteria such as *Salmonella* bacteria is permeabilized or removed (e.g. with chloroform) to allow the putative enzyme access to the peptidoglycan layer. If the enzyme is active, degradation of the peptidoglycan layer will lead to a drop of turbidity, which can be measured photometrically (see for example Briers et al., J. Biochem. Biophys Methods 70: 531-533, (2007).

The present invention does also relate to nucleic acids encoding one or more inventive polypeptides of the present invention. The inventive nucleic acid may take all forms conceivable for a nucleic acid. In particular the nucleic acids according to the present invention may be RNA, DNA or hybrids thereof. They may be single-stranded or doublestranded. The may have the size of small transcripts or of entire genomes, such as a bacteriophage genome. As used herein, a nucleic acid encoding one or more inventive polypeptides of the present invention may be a nucleic acid reflecting the sense strand. Likewise, the antisense strand is also encompassed. The nucleic acid may encompass a heterologous promotor for expression of the inventive polypeptide.

A nucleic acid according to the present invention encodes a polypeptide according to the invention. It comprises a first nucleic acid sequence encoding an endolysin, and a second nucleic acid sequence, wherein the second nucleic acid sequence encodes an antimicrobial peptide, cationic peptide, hydrophobic peptide, amphiphatic peptide or sushi peptide. Preferably, the nucleic acid according to the present invention comprises at least one sequence selected from the following group of sequences:
i) a nucleic acid sequence according to SEQ ID NO:149;
ii) a derivative of SEQ ID NO:149 encoding the same polypeptide as SEQ ID NO:149,
iii) a nucleic acid sequence according to SEQ ID NO:150;
iv) a derivative of SEQ ID NO:150 encoding the same polypeptide as SEQ ID NO:150;
v) a nucleic acid sequence according to SEQ ID NO:151; and
vi) a derivative of SEQ ID NO:151 encoding the same polypeptide as SEQ ID NO:151.

Preferred nucleic acids according to the present invention comprise the nucleic acid sequence according to SEQ ID NO:149 as first nucleic acid sequence and SEQ ID NO:150 as second nucleic acid sequence. Particularly preferred nucleic acid sequences comprise SEQ ID NO:153.

In a further aspect, the present invention relates to a vector, which comprises a nucleic acid according to the present invention. Such vector may for example be an expression vector allowing for expression of an inventive polypeptide. Said expression may be constitutive or inducible. The vector may also be a cloning vector comprising the nucleic acid sequence of an inventive polypeptide for cloning purposes.

In a further aspect, the present invention relates to a host cell comprising a polypeptide according to the present invention, a nucleic acid according to the present invention, and/or a vector according to the present invention. The host cells may be selected in particular from the group consisting of bacterial cells and yeast cells. Particularly preferred host cells are *E. coli* cells.

In a further aspect, the present invention relates to composition comprising a polypeptide according to the present invention, a nucleic acid according to the present invention, a vector according to the present invention, and/or a host cell according to the present invention. Preferred compositions comprise the polypeptide according to the present invention. Preferably, a composition according to the present invention comprises a pharmaceutical acceptable diluent, excipient or carrier. Such composition may be a pharmaceutical composition. A composition according to the present invention may also be a feed additive.

In a further aspect the present invention relates to a polypeptide according to the present invention, a nucleic acid according to the present invention, a vector according to the present invention, a host cell according to the present invention, and/or a composition according the present invention for use in a method for treatment of the human or animal body by surgery or therapy or in diagnostic methods practiced on the human or animal body.

The present invention also relates to a polypeptide (and likewise a nucleic acid encoding such polypeptide, a vector comprising such nucleic acid, a host cell comprising such nucleic acid or vector, and/or a composition comprising such polypeptide, nucleic acid, vector, and/or host cell) for use in a method of treatment or prevention of infections caused by bacteria of the genus *Salmonella,* wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1; and
ii) a derivative of SEQ ID NO:1 exhibiting at least 80% sequence identity with SEQ ID NO:1.

Disclosure set out above for the inventive polypeptide is contemplated for the polypeptide (and the nucleic acid encoding such polypeptide, the vector comprising such nucleic acid, the host cell comprising such nucleic acid or vector, and/or the composition comprising such polypeptide, nucleic acid, vector, and/or host cell) for use in a method of treatment or prevention of infections caused by bacteria of the genus *Salmonella* as well. As mentioned previously, the provisos regarding the polypeptide of the invention (polypeptide may not comprise the sequence of SEQ ID NO:5, if the polypeptide of the invention does not comprise in parallel an amino acid sequence according to SEQ ID NO:3, a derivative of SEQ ID NO:3 as defined herein, an amino acid sequence according to SEQ ID NO:4, and/or a derivative of SEQ ID NO:4 as defined herein; polypeptide may not comprise in parallel the sequence of SEQ ID NO:6, if the polypeptide comprises SEQ ID NO:4) do not necessarily but preferably apply for this aspect of the present invention. Thus, in some embodiments, the polypeptide for use is an inventive polypeptide as set forth above, i.e. the present invention relates to a polypeptide according to the present invention, a nucleic acid according to the present invention, a vector according to the present invention, a host cell according to the present invention, and/or a composition according the present invention for use in a method of treatment or prevention of infections caused by bacteria of the genus *Salmonella.*

In a further aspect the present invention relates to the use of a polypeptide (or likewise a nucleic acid encoding such polypeptide, a vector comprising such nucleic acid, a host cell comprising such nucleic acid or vector, and/or a composition comprising such polypeptide, nucleic acid, vector, and/or host cell) as an antimicrobial in food, as an antimicrobial in feed, as an antimicrobial in cosmetics, or as disinfecting agent, wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1; and
ii) a derivative of SEQ ID NO:1 exhibiting at least 80% sequence identity with SEQ ID NO:1.

Again, the provisos regarding the polypeptide of the invention (see above) do not necessarily but preferably apply for this aspect of the present invention. Thus, the present invention does also relate to a polypeptide according to the present invention, a nucleic acid according the present invention, a vector according to the present invention, a host cell according to the present invention, and/or a composition according to the present invention as an antimicrobial in food, as an antimicrobial in feed, as an antimicrobial in cosmetics, or as disinfecting agent.

In a further aspect the present invention relates to the use of a polypeptide (and likewise a nucleic acid encoding such polypeptide, a vector comprising such nucleic acid, a host cell comprising such nucleic acid or vector, and/or a composition comprising such polypeptide, nucleic acid, vector, and/or host cell) for controlling (e.g. non-therapeutically) the growth of bacteria of the genus *Salmonella* in animals, in particular in livestock, companion animal and/or aquaculture, wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1; and
ii) a derivative of SEQ ID NO:1 exhibiting at least 80% sequence identity with SEQ ID NO:1.

The provisos regarding the polypeptide of the invention (see above) do not necessarily but preferably apply for this aspect of the present invention as well. Disclosure set out above for the inventive polypeptide is contemplated for the polypeptide (and the nucleic acid encoding such polypeptide, the vector comprising such nucleic acid, the host cell comprising such nucleic acid or vector, and/or the composition comprising such polypeptide, nucleic acid, vector, and/or host cell) to be used for controlling (e.g. non-therapeutically) the growth of bacteria of the genus *Salmonella* in animals (in particular in livestock, companion animal and/or aquaculture) as well. In some embodiments, the polypeptide to be used for controlling (e.g. non-therapeutically) the growth of bacteria of the genus *Salmonella* in animals is an inventive polypeptide as set forth above, i.e. the present invention relates to the use of a polypeptide according to the present invention, a nucleic acid according to the present invention, a vector according to the present invention, a host cell according to the present invention, and/or a composition according the present invention for controlling (e.g. non-therapeutically) the growth of bacteria of the genus *Salmonella* in animals, in particular in livestock, companion animal and/or aquaculture.

In a further aspect the present invention relates to a method of controlling the growth of bacteria of the genus *Salmonella* in animals, in particular in livestock, companion animal and/or aquaculture, the method comprising contacting livestock, companion animal and/or aquaculture with a polypeptide (or a nucleic acid encoding such polypeptide, a vector comprising such nucleic acid, a host cell comprising such nucleic acid or vector, and/or a composition comprising such polypeptide, nucleic acid, vector, and/or host cell), wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1; and
ii) a derivative of SEQ ID NO:1 exhibiting at least 80% sequence identity with SEQ ID NO:1.

The provisos regarding the polypeptide of the invention (see above) do not necessarily but preferably apply for this aspect of the present invention as well. Again, disclosure set out above for the inventive polypeptide is contemplated for the polypeptide (and the nucleic acid encoding such polypeptide, the vector comprising such nucleic acid, the host cell comprising such nucleic acid or vector, and/or the composition comprising such polypeptide, nucleic acid, vector, and/or host cell) to be used in said method of controlling the growth of bacteria of the genus *Salmonella* in animals, in particular in livestock, companion animal and/or aquaculture, as well. In some embodiments, the polypeptide to be used in the inventive method of treatment is an inventive polypeptide as set forth above, i.e. the present invention relates to a method of controlling the growth of bacteria of the genus *Salmonella* in animals, e.g. in livestock, companion animal and/or aquaculture, the method comprising contacting the animal (e.g. livestock, companion animal and/or aquaculture) with a polypeptide according to the present invention, a nucleic acid according to the present invention, a vector according to the present invention, a host cell according to the present invention, and/or the composition according to the present invention. Said inventive method of controlling the growth of bacteria of the genus *Salmonella* is preferably a non-therapeutic method, in particular where bacteria of the genus *Salmonella* are non-pathogenic for the respective animal (e.g. livestock, companion animal and/or aquaculture) or are only present in (for the animal) sub-pathogenic concentration, i.e. would not require medical treatment of said animal (i.e. livestock, companion animal and/or aquaculture).

### Examples

In the following, specific examples illustrating various embodiments and aspects of the invention are presented.

### Example 1: Data on antibacterial activity on S. Typhimurium LT2 from prior art polypeptides published in Briers et al, MBio. 2014 Jul 1;5(4):e01379-14; Comparative example

Briers et al. published antibacterial activity of various fusion proteins for S. Typhimurium LT2 (see supplemental table 5). The data are summarized in table 3 (fusion variants of endolysin OBPgp279) and in table 4 (fusion variants of endolysin PVP-SE1gp146).

**Table 3: Fusion variants of endolysin OBPgp279**

| **Fusion** | **Polypeptide** | **w/o EDTA** | **0.5 mM EDTA** |
|---|---|---|---|
| | | | 0.05 ± 0.05 |
| **PCNP** | **LoGT-001** | 0.23 ± 0.03 | 0.91 ± 0.04 |
| **MW1** | **LoGT-002** | 0.15 ± 0.07 | 0.41 ± 0.08 |
| **HPP** | **LoGT-003** | 0.09 ± 0.01 | 0.52 ± 0.04 |
| **MW2** | **LoGT-004** | 0.12 ± 0.06 | 0.36 ± 0.07 |
| **α₄** | **LoGT-005** | 0.17 ± 0.08 | 0.43 ± 0.09 |
| **Parasinl** | **LoGT-006** | 0.20 ± 0.14 | 0.60 ± 0.05 |
| **Lycotoxinl** | **LoGT-007** | 0.01 ± 0.11 | 0.51 ± 0.04 |

**Table 4: Fusion variants of endolysin PVP-SE1gp146**

| **Fusion** | **Polypeptide** | ***S.* Typhimurium LT2** | **0.5 mM EDTA** |
|---|---|---|---|
| | | | 0.05 ± 0.05 |
| **PCNP** | **LoGT-008** | 0.30 ± 0.10 | 0.73 ± 0.30 |
| **MW1** | **LoGT-009** | 0.10 ± 0.07 | 0.27 ± 0.15 |
| **HPP** | **LoGT-010** | 0.24 ± 0.16 | 0.47 ± 0.07 |
| **MW2** | **LoGT-011** | 0.12 ± 0.12 | 0.41 ± 0.25 |
| **α₄** | **LoGT-012** | 0.14 ± 0.12 | 0.47 ± 0.24 |
| **Parasinl** | **LoGT-013** | 0.04 ± 0.03 | 0.59 ± 0.38 |
| **Lycotoxinl** | **LoGT-014** | 0.20 ± 0.22 | 0.87 ± 0.28 |

### Example 2: Data on antibacterial activity of selected polypeptides on S. Typhimurium LT2

The inventors of the present invention have tested antibacterial activity of polypeptides comprising SEQ ID NO: 126 (a polypeptide according to the present invention) or SEQ ID NO: 129 (comparative example) (and further comprising a His-tag for purification reasons) on S. Typhimurium LT2. A first set of experiments was conducted in PBS buffer.

Briefly, exponentially growing cells of *Salmonella* Typhimurium LT2 were diluted 1:100 in 5 mM HEPES pH 7.4. Subsequently, 100 µl of cells were incubated for 30 min at room temperature with 50 µl protein solution dissolved in 1 × PBS (170 mM NaCl, 3 mM KCl, 12.7 mM Na₂HPO₄, 2.2 mM KH₂PO₄, pH 7.4) and 50 µl of 5 mM HEPES pH 7.4 or 50 µL of EDTA (final concentration 0.5 mM) yielding a final protein concentration of 1.313 µM. Cells were serially diluted in 1 × PBS after incubation and plated on LB agar. As a negative control, cells were incubated in the same buffer without any protein and additionally plated. Cell colonies were counted after incubation over night at 37 °C and the antibacterial activity was calculated in logarithmic units (=log10No/Ni with N0 = number of untreated colonies and Ni = number of treated colonies).

The results are shown in table 5 below.

**Table 5: Antibacterial activity of selected polypeptides in PBS buffer**

| **SEQ ID NO** | **w/o EDTA** | **0.5 mM EDTA** |
|---|---|---|
| SEQ ID NO: 126 | 2.75 | ≥ 4.00 |
| SEQ ID NO: 129 | 1.61 | 2.29 |

In a further set of experiments, antibacterial activity in HEPES-buffer on S. Typhimurium LT2 was determined.

Briefly, exponentially growing cells of *Salmonella* Typhimurium LT2 were diluted 1:100 in 5 mM HEPES pH 7.4. Subsequently, 100 µl of cells were incubated for 30 min at room temperature with 50 µl protein solution dissolved in 20 mM HEPES, 500 mM NaCl pH 7.4 and 50 µl of 5 mM HEPES pH 7.4 or 50 µL of EDTA (final concentration 0.5 mM) yielding a final protein concentration of 1.313 µM. Cells were serially diluted in 1 × PBS after incubation and plated on LB agar. As a negative control, cells were incubated in the same buffer without any protein and additionally plated. Cell colonies were counted after incubation over night at 37 °C and the antibacterial activity was calculated in logarithmic units (=log10No/Ni with N0 = number of untreated colonies and Ni = number of treated colonies).

The results are shown in table 6 below.

**Table 6: Antibacterial activity of selected polypeptides in HEPES buffer**

| **SEQ ID NO** | **w/o EDTA** | **0.5 mM EDTA** |
|---|---|---|
| SEQ ID NO: 126 | 0.95 | 3.62 |
| SEQ ID NO: 129 (comparative example) | 1.24 | 1.61 |

In summary, antibacterial activity of the polypeptides comprising the amino acids sequence of SEQ ID NO: 126 (inventive polypeptide) or SEQ ID NO: 129 (comparative example) on S. Typhimurium LT2 surprisingly exceeded the antibacterial activity achieved with similar compounds in the art significantly.

### Example 3: Data on antibacterial activity of further selected polypeptides on S. Typhimurium LT2

In another set of experiments, the inventors of the present invention have also tested antibacterial activity of further fusion proteins. The polypeptides tested did comprise the sequences of SEQ ID NO: 138, SEQ ID NO: 141, SEQ ID NO: 132, SEQ ID NO: 144, and SEQ ID NO: 147 (and further comprising a His-tag for purification reasons) and were tested on S. Typhimurium LT2. The tested compounds comprising SEQ ID NO: 132, SEQ ID NO: 144, and SEQ ID NO: 147 all comprise a component which can be advantageously used according to the present invention (SEQ ID NO:1) for treating in particular *Salmonella* bacteria.

The experiment was done as set out in Example 2.

The results are shown in table 7 below.

**Table 7: Antibacterial activity of selected polypeptides in HEPES buffer**

| **SEQ ID NO** | **w/o EDTA** | **0.5 mM EDTA** |
|---|---|---|
| SEQ ID NO: 138 (comparative example) | 0.76 | 0.88 |
| SEQ ID NO: 141 (comparative example) | 1.71 | 1.47 |
| SEQ ID NO: 132 | 2.74 | >5 |
| SEQ ID NO: 144 | 0.15 | 3.24 |
| SEQ ID NO: 147 (comparative example) | 0.33 | 0.98 |

In summary, antibacterial activity of the inventive polypeptides comprising the amino acids sequence of SEQ ID NO: 132, SEQ ID NO: 144, and SEQ ID NO: 147 on S. Typhimurium LT2 reliably exceeded the antibacterial activity achieved with similar compounds in the art, indicating that the component used (SEQ ID NO:1) can provide for superior activity against Salmonella bacteria in comparison to the compounds of the prior art. Noteworthy, the polypeptide of SEQ ID NO: 132 yielded again by far the best results.

### Example 4: Data on antibacterial activity of selected polypeptide on S. Typhimurium (DSM 17058) (Comparative Example)

The inventors of the present invention have tested antibacterial activity of a polypeptide comprising SEQ ID NO: 135 (and further comprising a His-tag for purification reasons) on S. Typhimurium (DSM 17058).

The experiment was done as set out in Example 2.

The results are shown in table 8 below.

**Table 8: Antibacterial activity of polypeptide in HEPES buffer**

| **SEQ ID NO** | **w/o EDTA** | **0.5 mM EDTA** |
|---|---|---|
| SEQ ID NO: 135 | 3.91 | 3.88 |

Hence, antibacterial activity of the polypeptide comprising the amino acid sequence of SEQ ID NO: 135 on S. Typhimurium (DSM 17058) exceeded the antibacterial activity achieved with similar compounds in the art, indicating that the endolysin component used (SEQ ID NO:13) provides for superior activity against Salmonella bacteria in comparison to the respective components of the prior art polypeptides (i.e. endolysin components OBPgp279 and PVP-SE1gp146).

### Example 5: Data on minimal inhibitory concentration of selected inventive polypeptide on S. Typhimurium (DSM 17058)

The inventors of the present invention have tested antibacterial activity in terms of minimal inhibitory concentration (MIC) of an inventive polypeptide comprising SEQ ID NO: 132 (and further comprising a His-tag for purification reasons) on S. Typhimurium (DSM 17058).

Briefly, Bacteria were grown in (Luria-Bertani) medium and diluted 1:10 in Mueller-Hinton medium. At optical density OD₆₀₀ of about 0.6 bacteria were diluted in the same medium 1:10 followed by a 1:500 dilution in the same medium. Protein solutions were pipetted into a 96 well plate, using different concentrations of proteins and an end volume of 20 µl including 500 µM EDTA final concentration. 180 µl of bacterial cells or a medium (Mueller-Hinton) control were given to the 96 well plate and mixed. The plate was incubated for 18-22 hours at 37°C and the bacterial growth was determined measuring the OD₆₀₀ values of the wells. The MIC which is the protein concentration of the well which showed the same OD₆₀₀ value as determined for the no-bacteria control.

The results in form of minimal inhibitory concentration (MIC) are shown in table 9 below.

**Table 9: Antibacterial activity of inventive polypeptides in PBS buffer**

| **SEQ ID NO** | **0.5 mM EDTA** |
|---|---|
| SEQ ID NO: 132 | ≤5 |

### Example 6: Data on antibacterial activity of selected inventive polypeptide on various Salmonella Enteritidis serovars in presence of salt

The inventors of the present invention have tested antibacterial activity of the polypeptide comprising SEQ ID NO: 132 (and further comprising a His-tag for purification reasons) on various Salmonella serovars in presence of physiological salt levels.

Briefly, exponentially growing cells of Salmonella Enteritidis were diluted 1:10 in 20 mM HEPES pH 7.4, 150 mM NaCl. Subsequently, 50 µl of cells were incubated for 1 h at 37 °C with 50 µl protein solution dissolved in 20 mM HEPES, 150 mM NaCl pH 7.4 yielding a final protein concentration of 100 µg/ml. Cells were serially diluted in 1 × PBS after incubation and plated on LB agar. As a negative control, cells were incubated in the same buffer without any protein and additionally plated. Cell colonies were counted after incubation over night at 37 °C and the antibacterial activity was calculated in logarithmic units (=log10No/Ni with NO = number of untreated colonies and Ni = number of treated colonies).

The results in form of minimal inhibitory concentration (MIC) are shown in table 10 below.

**Table 10: Antibacterial activity of inventive polypeptides under physiological conditions**

| **SEQ ID NO** | **Serovar** | **w/o EDTA** |
|---|---|---|
| SEQ ID NO: 132 | Salmonella Enteritidis RKI 14-00409 | 3.5 |
| SEQ ID NO: 132 | Salmonella Enteritidis LGL-246 | 4.5-5.5 |
| SEQ ID NO: 132 | Salmonella Enteritidis 29/2014 | 6 |

The polypeptide comprising SEQ ID NO: 132 thus shows significant antibacterial activity in presence of physiological salt conditions.

### Example 7: Thermostability

The inventors of the present invention have tested antibacterial activity of the polypeptide comprising SEQ ID NO: 126 on S. Typhimurium (DSM 17058) with increasing temperatures.

Briefly, prior to the determination of the MIC, the protein solution is heated to given temperatures for 20 min. Subsequently, the protein solution is stored on ice to reach a temperature of 4°C. Bacteria were grown in (Luria-Bertani) medium and diluted 1:10 in Mueller-Hinton medium. At optical density OD₆₀₀ of about 0.6 bacteria were diluted in the same medium 1:10 followed by a 1:500 dilution in the same medium. Protein solutions were pipetted into a 96 well plate, using different concentrations of proteins and an end volume of 20 µl including 500 µM EDTA final concentration. 180 µl of bacterial cells or a medium (Mueller-Hinton) control were given to the 96 well plate and mixed. The plate was incubated for 18-22 hours at 37°C and the bacterial growth was determined measuring the OD₆₀₀ values of the wells. The MIC which is the protein concentration of the well which showed the same OD₆₀₀ value as determined for the no-bacteria control.

The results in form of minimal inhibitory concentration (MIC) are shown in table 11 below.

**Table 11: Antibacterial activity of inventive polypeptide with increasing temperature**

| | MIC [µg/ml] |
|---|---|
| Temp. [°C] | SEQ ID NO: 126 |
| RT | 6 |
| 70 | 12 |
| 74 | 10 |
| 78 | 10 |
| 82 | 10 |
| 86 | 10 |
| 90 | 8-10 |
| 94 | 8 |

The polypeptide comprising SEQ ID NO: 126 thus shows extreme thermostability with significant antibacterial activity even at very high temperatures.

Similar results were obtained when using the same endolysin with another peptide or when using a variant of the endolysin (e.g. SEQ ID NO:7) with such peptides.

## Claims

1. Polypeptide comprising a first and a second amino acid sequence, wherein the first amino acid sequence is an endolysin, and wherein the second amino acid sequence is an antimicrobial peptide, cationic peptide, hydrophobic peptide, amphiphatic peptide or sushi peptide, and wherein the polypeptide comprises at least one sequence selected from the following group of sequences:
i) an amino acid sequence according to SEQ ID NO:1;
ii) a derivative of SEQ ID NO:1 exhibiting at least 80*%* sequence identity with SEQ ID NO:1,
with the proviso that the polypeptide may not comprise the sequence of SEQ ID NO:5, if the polypeptide comprises ii), but none of:
a) an amino acid sequence according to SEQ ID NO:3;
b) a derivative of SEQ ID NO:3 exhibiting at least 77*%* sequence identity with SEQ ID NO:3;
c) an amino acid sequence according to SEQ ID NO:4; with the proviso that the polypeptide may in this case not comprise the sequence of SEQ ID NO:6 in parallel; and
d) a derivative of SEQ ID NO:4 exhibiting at least 80*%* sequence identity with SEQ ID NO:4, and
wherein the polypeptide degrades the peptidoglycan of *Salmonella* bacteria.

2. The polypeptide according to claim 1, wherein the derivative of SEQ ID NO:1 exhibits at least 85*%*, at least 87,5*%*, at least 90*%*, at least 91*%*, at least 92*%*, at least 93*%*, at least 94*%*, at least 95*%*, at least 96*%*, at least 97*%*, at least 98*%*, at least 99*%*, or more than 99*%* sequence identity with SEQ ID NO:1, in particular wherein the derivative of SEQ ID NO:1 exhibits at least 98*%*, at least 99*%*, or more than 99*%* sequence identity with SEQ ID NO:1.

3. The polypeptide according to any one of claims 1 to 2, wherein the derivative of SEQ ID NO:1 is an amino acid sequence according to SEQ ID NO:7.

4. The polypeptide according to anyone of the preceding claims, wherein the second amino acid sequence is selected from the group consisting of:
i) an amino acid sequence according to SEQ ID NO:3;
ii) a derivative of SEQ ID NO:3 exhibiting at least 77*%* sequence identity with SEQ ID NO:3;
iii) an amino acid sequence according to SEQ ID NO:4; and
iv) a derivative of SEQ ID NO:4 exhibiting at least 80*%* sequence identity with SEQ ID NO:4.

5. The polypeptide according to claim 4, wherein the derivative of SEQ ID NO:3 exhibits at least 81*%*, at least 86*%*, at least 90*%*, or at least 95*%* sequence identity with SEQ ID NO:3, or wherein the derivative of SEQ ID NO:4 exhibits at least 83*%*, at least 86*%*, at least 90*%*, at least 93*%*, or at least 96*%* sequence identity with SEQ ID NO:4.

6. The polypeptide according to anyone of claims 1 to 5, wherein said derivative of SEQ ID NO:3 is a fragment of SEQ ID NO:3, in particular a fragment according to SEQ ID NO:37.

7. The polypeptide according to claim 1, wherein the polypeptide comprises an amino acid sequence according to SEQ ID NO: 126, SEQ ID NO: 127, SEQ ID NO: 132 or SEQ ID NO: 133.

8. Nucleic acid encoding a polypeptide according to any one of claims 1 to 7.

9. Vector comprising a nucleic acid according to claim 8.

10. Host cell comprising a polypeptide according to any one of claims 1 to 7, a nucleic acid according to claim 8, and/or a vector according to claim 9.

11. Composition comprising a polypeptide according to any one of claims 1 to 7, a nucleic acid according to claim 8, a vector according to claim 9 and/or a host cell according to claim 10.

12. A polypeptide according to any one of claims 1 to 7, a nucleic acid according to claim 8, a vector according to claim 9, a host cell according to claim 10, or a composition according to claim 11 for use in a method for treatment of the human or animal body by surgery or therapy or in diagnostic methods practiced on the human or animal body.

13. A polypeptide, a nucleic acid encoding such polypeptide, a vector comprising such nucleic acid, a host cell comprising such polypeptide, nucleic acid and/or vector, or a composition comprising such polypeptide, nucleic acid, vector, and/or host cell for use in a method for treatment or prevention of infections caused by bacteria of the genus *Salmonella,* wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1; and
ii) a derivative of SEQ ID NO:1 exhibiting at least 80*%* sequence identity with SEQ ID NO:1, and
wherein the polypeptide degrades the peptidoglycan of *Salmonella* bacteria.

14. The polypeptide, nucleic acid, vector, host cell or composition for use in a method for treatment or prevention of infections caused by bacteria of the genus *Salmonella* according to claim 13, wherein the polypeptide is a polypeptide according to any one of claims 1 to 7, wherein the nucleic acid is a nucleic acid according to claim 8, wherein the vector is a vector according to claim 9 or wherein the host cell is a host cell according to claim 10 or wherein the composition is a composition according to claim 11.

15. Use of the polypeptide according to any one of claims 1 to 7, a nucleic acid according to claim 8, a vector according to claim 9, a host cell according to claim 10 or a composition according to claim 11 as an antimicrobial in food, as an antimicrobial in feed, as an antimicrobial in cosmetics, or as disinfecting agent.

16. Non-therapeutic method of controlling the growth of bacteria of the genus *Salmonella* in animals, in particular in livestock, companion animal and/or aquaculture, the method comprising contacting said animal, in particular the livestock, companion animal and/or aquaculture, with a polypeptide, a nucleic acid encoding such polypeptide, a vector comprising such nucleic acid, a host cell comprising such polypeptide, nucleic acid and/or vector, or a composition comprising such polypeptide, nucleic acid, vector, and/or host cell, wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1; and
ii) a derivative of SEQ ID NO:1 exhibiting at least 80*%* sequence identity with SEQ ID NO:1, and
wherein the polypeptide degrades the peptidoglycan of *Salmonella* bacteria.

17. The method according to claim 16, wherein the polypeptide is a polypeptide according to any one of claims 1 to 7.

## Patentansprüche

1. Polypeptid, umfassend eine erste und eine zweite Aminosäuresequenz, wobei die erste Aminosäuresequenz ein Endolysin ist und wobei die zweite Aminosäuresequenz ein antimikrobielles Peptid, kationisches Peptid, hydrophobes Peptid, amphiphatisches Peptid oder Sushi-Peptid ist, und wobei das Polypeptid mindestens eine Sequenz ausgewählt aus der folgenden Gruppe von Sequenzen umfasst:
i) eine Aminosäuresequenz gemäß SEQ ID NO: 1;
ii) ein Derivat von SEQ ID NO:1, das mindestens 80 % Sequenzidentität mit SEQ ID NO:1 aufweist,
mit der Maßgabe, dass das Polypeptid nicht die Sequenz von SEQ ID NO: 5 umfassen darf, wenn das Polypeptid ii) umfasst, aber keines von:
a) einer Aminosäuresequenz gemäß SEQ ID NO: 3;
b) einem Derivat von SEQ ID NO:3, das mindestens 77% Sequenzidentität mit SEQ ID NO:3 aufweist;
c) einer Aminosäuresequenz gemäß SEQ ID NO: 4; mit der Maßgabe, dass das Polypeptid in diesem Fall nicht parallel die Sequenz von SEQ ID NO: 6 umfassen darf; und
d) einem Derivat von SEQ ID NO: 4, das mindestens 80 % Sequenzidentität mit SEQ ID NO: 4 aufweist, und
wobei das Polypeptid das Peptidoglycan von *Salmonella-Bakterien* abbaut.

2. Polypeptid nach Anspruch 1, wobei das Derivat von SEQ ID NO: 1 mindestens 85 %, mindestens 87,5 %, mindestens 90 %, mindestens 91 %, mindestens 92 %, mindestens 93 %, mindestens 94 %, mindestens 95 %, mindestens 96 %, mindestens 97 %, mindestens 98 %, mindestens 99 % oder mehr als 99 % Sequenzidentität mit SEQ ID NO: 1 aufweist., insbesondere wobei das Derivat von SEQ ID NO: 1 mindestens 98 %, mindestens 99 % oder mehr als 99 % Sequenzidentität mit SEQ ID NO: 1 aufweist.

3. Polypeptid nach einem der Ansprüche 1 bis 2, wobei das Derivat von SEQ ID NO: 1 eine Aminosäuresequenz gemäß SEQ ID NO: 7 ist.

4. Polypeptid nach einem der vorhergehenden Ansprüche, wobei die zweite Aminosäuresequenz ausgewählt ist aus der Gruppe bestehend aus:
i) einer Aminosäuresequenz gemäß SEQ ID NO: 3;
ii) einem Derivat von SEQ ID NO:3, das mindestens 77% Sequenzidentität mit SEQ ID NO:3 aufweist;
iii) einer Aminosäuresequenz gemäß SEQ ID NO: 4; und
iv) einem Derivat von SEQ ID NO: 4, das mindestens 80% Sequenzidentität mit SEQ ID NO: 4 aufweist.

5. Polypeptid nach Anspruch 4, wobei das Derivat von SEQ ID NO: 3 mindestens 81 %, mindestens 86 %, mindestens 90 % oder mindestens 95 % Sequenzidentität mit SEQ ID NO: 3 aufweist, oder wobei das Derivat von SEQ ID NO: 4 mindestens 83 %, mindestens 86 %, mindestens 90 %, mindestens 93 % oder mindestens 96 % Sequenzidentität mit SEQ ID NO: 4 aufweist.

6. Polypeptid nach einem der Ansprüche 1 bis 5, wobei das Derivat von SEQ ID NO: 3 ein Fragment von SEQ ID NO: 3 ist, insbesondere ein Fragment gemäß SEQ ID NO: 37.

7. Polypeptid nach Anspruch 1, wobei das Polypeptid eine Aminosäuresequenz gemäß SEQ ID NO: 126, SEQ ID NO: 127, SEQ ID NO: 132 oder SEQ ID NO: 133 umfasst.

8. Nukleinsäure, die ein Polypeptid nach einem der Ansprüche 1 bis 7 kodiert.

9. Vektor umfassend eine Nukleinsäure nach Anspruch 8.

10. Wirtszelle enthaltend ein Polypeptid nach einem der Ansprüche 1 bis 7, eine Nukleinsäure nach Anspruch 8 und/oder einen Vektor nach Anspruch 9.

11. Zusammensetzung umfassend ein Polypeptid nach einem der Ansprüche 1 bis 7, eine Nukleinsäure nach Anspruch 8, einen Vektor nach Anspruch 9 und/oder eine Wirtszelle nach Anspruch 10.

12. Polypeptid nach einem der Ansprüche 1 bis 7, Nukleinsäure nach Anspruch 8, Vektor nach Anspruch 9, Wirtszelle nach Anspruch 10 oder Zusammensetzung nach Anspruch 11 zur Verwendung in einem Verfahren zur Behandlung des menschlichen oder tierischen Körpers durch Operation oder Therapie oder in diagnostischen Verfahren, die am menschlichen oder tierischen Körper praktiziert werden.

13. Ein Polypeptid, eine Nukleinsäure, die ein solches Polypeptid kodiert, ein Vektor, der eine solche Nukleinsäure umfasst, eine Wirtszelle, die ein solches Polypeptid, eine solche Nukleinsäure und/oder einen solchen Vektor umfasst, oder eine Zusammensetzung, die ein solches Polypeptid, eine solche Nukleinsäure, einen solchen Vektor und/oder eine solche Wirtszelle umfasst, zur Verwendung in einem Verfahren zur Behandlung oder Vorbeugung von Infektionen, die durch Bakterien der Gattung *Salmonella* verursacht werden, wobei das Polypeptid eine erste und eine zweite Aminosäuresequenz umfasst, wobei die erste Aminosäuresequenz ein Endolysin ist und wobei die zweite Aminosäuresequenz ein antimikrobielles Peptid, amphiphatisches Peptid, kationisches Peptid, hydrophobes Peptid, Sushi-Peptid oder Defensin ist, und wobei das Polypeptid mindestens eine Sequenz ausgewählt aus der folgenden Gruppe von Sequenzen umfasst:
i) eine Aminosäuresequenz gemäß SEQ ID NO: 1; und
ii) ein Derivat von SEQ ID NO: 1, das mindestens 80 % Sequenzidentität mit SEQ ID NO: 1 aufweist, und
wobei das Polypeptid das Peptidoglycan von *Salmonella-Bakterien* abbaut

14. Polypeptid, Nukleinsäure, Vektor, Wirtszelle oder Zusammensetzung zur Verwendung in einem Verfahren zur Behandlung oder Prävention von Infektionen, die durch Bakterien der Gattung *Salmonella* nach Anspruch 13 verursacht werden, wobei das Polypeptid ein Polypeptid nach einem der Ansprüche 1 bis 7 ist, wobei die Nukleinsäure eine Nukleinsäure nach Anspruch 8 ist, wobei der Vektor ein Vektor nach Anspruch 9 ist oder wobei die Wirtszelle eine Wirtszelle nach Anspruch 10 ist oder wobei die Zusammensetzung eine Zusammensetzung nach Anspruch 11 ist.

15. Verwendung des Polypeptids nach einem der Ansprüche 1 bis 7, einer Nukleinsäure nach Anspruch 8, einem Vektor nach Anspruch 9, einer Wirtszelle nach Anspruch 10 oder einer Zusammensetzung nach Anspruch 11 als antimikrobielles Mittel in Lebensmitteln, als antimikrobielles Mittel in Futtermitteln, als antimikrobielles Mittel in Kosmetika oder als Desinfektionsmittel.

16. Nicht-therapeutisches Verfahren zur Bekämpfung des Wachstums von Bakterien der Gattung *Salmonella* in Tieren, insbesondere in Nutztieren, Haustieren und/oder Aquakultur, wobei das Verfahren das Kontaktieren des Tieres, insbesondere des Nutztiers, Haustiers und/oder der Aquakultur, mit einem Polypeptid, einer Nukleinsäure, die ein solches Polypeptid kodiert, einem Vektor, der eine solche Nukleinsäure umfasst, einer Wirtszelle, die ein solches Polypeptid, eine solche Nukleinsäure und/oder einen solchen Vektor umfasst, oder einer Zusammensetzung, die ein solches Polypeptid, eine solche Nukleinsäure, einen solchen Vektor und/oder eine solche Wirtszelle umfasst, umfasst, wobei das Polypeptid eine erste und eine zweite Aminosäuresequenz umfasst, wobei die erste Aminosäuresequenz ein Endolysin ist und wobei die zweite Aminosäuresequenz ein antimikrobielles Peptid, amphiphatisches Peptid, kationisches Peptid, hydrophobes Peptid, Sushi-Peptid oder Defensin ist, und wobei das Polypeptid mindestens eine Sequenz ausgewählt aus der folgenden Gruppe von Sequenzen umfasst:
i) eine Aminosäuresequenz gemäß SEQ ID NO: 1; und
ii) ein Derivat von SEQ ID NO: 1, das mindestens 80 % Sequenzidentität mit SEQ ID NO: 1 aufweist, und
wobei das Polypeptid das Peptidoglycan von *Salmonella-Bakterien* abbaut.

17. Verfahren nach Anspruch 16, wobei das Polypeptid ein Polypeptid nach einem der Ansprüche 1 bis 7 ist.

## Revendications

1. Polypeptide comprenant une première et une seconde séquences d'acides aminés, dans lequel la première séquence d'acides aminés est une endolysine, et dans lequel la seconde séquence d'acides aminés est un peptide antimicrobien, un peptide cationique, un peptide hydrophobe, un peptide amphipathique ou un peptide sushi, et lequel polypeptide comprend au moins une séquence sélectionnée dans le groupe suivant de séquences :
i) une séquence d'acides aminés selon la SEQ ID NO: 1 ;
ii) un dérivé de la SEQ ID NO: 1 présentant une identité de séquence d'au moins 80 % avec la SEQ ID NO: 1,
à condition que le polypeptide ne comprenne pas la séquence de SEQ ID NO: 5, si le polypeptide comprend ii), mais aucun de :
a) une séquence d'acides aminés selon la SEQ ID NO: 3 ;
b) un dérivé de la SEQ ID NO: 3 présentant une identité de séquence d'au moins 77 % avec la SEQ ID NO: 3 ;
c) une séquence d'acides aminés selon la SEQ ID NO: 4 ; à condition que le polypeptide ne comprenne pas dans ce cas la séquence de SEQ ID NO: 6 en parallèle ; et
d) un dérivé de la SEQ ID NO: 4 présentant une identité de séquence d'au moins 80 % avec la SEQ ID NO: 4 ; et
lequel polypeptide dégrade le peptidoglycane des bactéries *Salmonella.*

2. Polypeptide selon la revendication 1, dans lequel le dérivé de la SEQ ID NO: 1 présente une identité de séquence d'au moins 85 %, d'au moins 87,5 %, d'au moins 90 %, d'au moins 91 %, d'au moins 92 %, d'au moins 93 %, d'au moins 94 %, d'au moins 95 %, d'au moins 96 %, d'au moins 97 %, d'au moins 98 %, d'au moins 99 %, ou plus de 99 % avec la SEQ ID NO: 1, en particulier dans lequel le dérivé de la SEQ ID NO: 1 présente une identité de séquence d'au moins 98 %, d'au moins 99 %, ou plus de 99 % avec la SEQ ID NO: 1.

3. Polypeptide selon l'une quelconque des revendications 1 à 2, dans lequel le dérivé de la SEQ ID NO: 1 est une séquence d'acides aminés selon la SEQ ID NO: 7.

4. Polypeptide selon l'une quelconque des revendications précédentes, dans lequel la seconde séquence d'acides aminés est sélectionnée dans le groupe constitué par :
i) une séquence d'acides aminés selon la SEQ ID NO: 3 ;
ii) un dérivé de la SEQ ID NO: 3 présentant une identité de séquence d'au moins 77 % avec la SEQ ID NO: 3 ;
iii) une séquence d'acides aminés selon la SEQ ID NO: 4 ; et
iv) un dérivé de la SEQ ID NO: 4 présentant une identité de séquence d'au moins 80 % avec la SEQ ID NO: 4.

5. Polypeptide selon la revendication 4, dans lequel le dérivé de la SEQ ID NO: 3 présente une identité de séquence d'au moins 81 %, d'au moins 86 %, d'au moins 90 %, ou d'au moins 95 % avec la SEQ ID NO: 3, ou dans lequel le dérivé de la SEQ ID NO: 4 présente une identité de séquence d'au moins 83 %, d'au moins 86 %, d'au moins 90 %, d'au moins 93 %, ou d'au moins 96 % avec la SEQ ID NO: 4.

6. Polypeptide selon l'une quelconque des revendications 1 à 5, dans lequel ledit dérivé de la SEQ ID NO: 3 est un fragment de la SEQ ID NO: 3, en particulier un fragment selon la SEQ ID NO: 37.

7. Polypeptide selon la revendication 1, lequel polypeptide comprend une séquence d'acides aminés selon la SEQ ID NO: 126, SEQ ID NO: 127, SEQ ID NO: 132 ou SEQ ID NO: 133.

8. Acide nucléique codant pour un polypeptide selon l'une quelconque des revendications 1 à 7.

9. Vecteur comprenant un acide nucléique selon la revendication 8.

10. Cellule hôte comprenant un polypeptide selon l'une quelconque des revendications 1 à 7, un acide nucléique selon la revendication 8, et/ou un vecteur selon la revendication 9.

11. Composition comprenant un polypeptide selon l'une quelconque des revendications 1 à 7, un acide nucléique selon la revendication 8, un vecteur selon la revendication 9 et/ou une cellule hôte selon la revendication 10.

12. Polypeptide selon l'une quelconque des revendications 1 à 7, acide nucléique selon la revendication 8, vecteur selon la revendication 9, cellule hôte selon la revendication 10, ou composition selon la revendication 11, pour utilisation dans une méthode de traitement chirurgical ou thérapeutique du corps humain ou animal ou dans des méthodes diagnostiques réalisées sur le corps humain ou animal.

13. Polypeptide, acide nucléique codant pour un tel polypeptide, vecteur comprenant un tel acide nucléique, cellule hôte comprenant un tel polypeptide, acide nucléique et/ou vecteur, ou composition comprenant un tel polypeptide, acide nucléique, vecteur, et/ou cellule hôte, pour utilisation dans une méthode de traitement ou de prévention d'infections provoquées par des bactéries du genre *Salmonella,* lequel polypeptide comprend une première et seconde séquences d'acides aminés, dans lequel la première séquence d'acides aminés est une endolysine, et dans lequel la seconde séquence d'acides aminés est un peptide antimicrobien, un peptide amphipathique, un peptide cationique, un peptide hydrophobe, un peptide sushi ou la défensine, et dans lequel le polypeptide comprend au moins une séquence sélectionnée dans le groupe suivant de séquences
i) une séquence d'acides aminés selon la SEQ ID NO: 1 ; et
ii) un dérivé de la SEQ ID NO: 1 présentant une identité de séquence d'au moins 80 % avec la SEQ ID NO: 1, et
lequel polypeptide dégrade le peptidoglycane des bactéries *Salmonella.*

14. Polypeptide, acide nucléique, vecteur, cellule hôte ou composition pour utilisation dans une méthode de traitement ou de prévention d'infections provoquées par des bactéries du genre *Salmonella* selon la revendication 13, lequel polypeptide est un polypeptide selon l'une quelconque des revendications 1 à 7, lequel acide nucléique est un acide nucléique selon la revendication 8, lequel vecteur est un vecteur selon la revendication 9 ou laquelle cellule hôte est une cellule hôte selon la revendication 10 ou laquelle composition est une composition selon la revendication 11.

15. Utilisation du polypeptide selon l'une quelconque des revendications 1 à 7, d'un acide nucléique selon la revendication 8, d'un vecteur selon la revendication 9, d'une cellule hôte selon la revendication 10 ou d'une composition selon la revendication 11, comme antimicrobien dans les aliments, comme antimicrobien dans les aliments pour animaux, comme antimicrobien dans les cosmétiques, ou comme agent désinfectant.

16. Méthode non thérapeutique de contrôle de la croissance de bactéries du genre *Salmonella* chez les animaux, en particulier le bétail, l'animal de compagnie et/ou le produit en aquaculture, la méthode comprenant la mise en contact dudit animal, en particulier du bétail, de l'animal de compagnie et/ou du produit d'aquaculture, avec un polypeptide, un acide nucléique codant pour un tel polypeptide, un vecteur comprenant un tel acide nucléique, une cellule hôte comprenant un tel polypeptide, acide nucléique et/ou vecteur, ou une composition comprenant un tel polypeptide, acide nucléique, vecteur et/ou cellule hôte, dans laquelle le polypeptide comprend une première et une seconde séquences d'acides aminés, laquelle première séquence d'acides aminés est une endolysine, et laquelle seconde séquence d'acides aminés est un peptide antimicrobien, un peptide amphipathique, un peptide cationique, un peptide hydrophobe, un peptide sushi ou la défensine, et dans laquelle le polypeptide comprend au moins une séquence sélectionnée dans le groupe suivant de séquences :
i) une séquence d'acides aminés selon la SEQ ID NO: 1 ; et
ii) un dérivé de la SEQ ID NO : 1 présentant une identité de séquence d'au moins 80% avec la SEQ ID NO: 1, et
dans laquelle le polypeptide dégrade le peptidoglycane des bactéries *Salmonella.*

17. Méthode selon la revendication 16, dans laquelle le polypeptide est un polypeptide selon l'une quelconque des revendications 1 à 7.


### SEQUENCE LISTING

<110> Lysando AG
<120> New antimicrobial agents against Salmonella bacteria
<130> LYS-034 PCT
<150> EP16167543
   <151> 2016-04-28
<160> 154
<170> PatentIn version 3.5
<210> 1
   <211> 136
   <212> PRT
   <213> Unknown
<220>
   <223> S394 endolysin without N-terminal methionine
<400> 1
   Ser Phe Lys Phe Gly Lys Asn Ser Glu Lys Gln Leu Ala Thr Val Lys
   1               5                   10                  15
   Pro Glu Leu Gln Lys Val Ala Arg Arg Ala Leu Glu Leu Ser Pro Tyr
               20                  25                  30
   Asp Phe Thr Ile Val Gln Gly Ile Arg Thr Val Ala Gln Ser Ala Gln
           35                  40                  45
   Asn Ile Ala Asn Gly Thr Ser Phe Leu Lys Asp Pro Ser Lys Ser Lys
       50                  55                  60
   His Val Thr Gly Asp Ala Ile Asp Phe Ala Pro Tyr Ile Asn Gly Lys
   65                  70                  75                  80
   Ile Asp Trp Lys Asp Leu Glu Ala Phe Trp Ala Val Lys Lys Ala Phe
                   85                  90                  95
   Glu Gln Ala Gly Lys Glu Leu Gly Ile Lys Leu Arg Phe Gly Ala Asp
               100                 105                 110
   Trp Asn Ser Ser Gly Asp Tyr His Asp Glu Ile Asp Arg Gly Thr Tyr
           115                 120                 125
   Asp Gly Gly His Val Glu Leu Val
       130                 135
<210> 2
   <211> 259
   <212> PRT
   <213> unknown
<220>
   <223> KZ144 endolysin without N-terminal methionine
<400> 2
   Lys Val Leu Arg Lys Gly Asp Arg Gly Asp Glu Val Cys Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Cys Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Thr Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Ile Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Ala Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Ser Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 3
   <211> 22
   <212> PRT
   <213> Artificial sequence
<220>
   <223> MSI-78 peptide
<400> 3
   Gly Ile Gly Lys Phe Leu Lys Lys Ala Lys Lys Phe Gly Lys Ala Phe
   1               5                   10                  15
   Val Lys Ile Leu Lys Lys
               20
<210> 4
   <211> 30
   <212> PRT
   <213> Bungarus fasciatus
<220>
   <221> misc_feature
   <222> (1)..(30)
   <223> Cathelicidin-BF peptide
<400> 4
   Lys Phe Phe Arg Lys Leu Lys Lys Ser Val Lys Lys Arg Ala Lys Glu
   1               5                   10                  15
   Phe Phe Lys Lys Pro Arg Val Ile Gly Val Ser Ile Pro Phe
               20                  25                  30
<210> 5
   <211> 137
   <212> PRT
   <213> Escherichia virus T5
<400> 5
   Met Ser Phe Lys Phe Gly Lys Asn Ser Glu Lys Gln Leu Ala Thr Val
   1               5                   10                  15
   Lys Pro Glu Leu Gln Lys Val Ala Arg Arg Ala Leu Glu Leu Ser Pro
               20                  25                  30
   Tyr Asp Phe Thr Ile Val Gln Gly Ile Arg Thr Val Ala Gln Ser Ala
           35                  40                  45
   Gln Asn Ile Ala Asn Gly Thr Ser Phe Leu Lys Asp Pro Ser Lys Ser
       50                  55                  60
   Lys His Ile Thr Gly Asp Ala Ile Asp Phe Ala Pro Tyr Ile Asn Gly
   65                  70                  75                  80
   Lys Ile Asp Trp Asn Asp Leu Glu Ala Phe Trp Ala Val Lys Lys Ala
                   85                  90                  95
   Phe Glu Gln Ala Gly Lys Glu Leu Gly Ile Lys Leu Arg Phe Gly Ala
               100                 105                 110
   Asp Trp Asn Ala Ser Gly Asp Tyr His Asp Glu Ile Lys Arg Gly Thr
           115                 120                 125
   Tyr Asp Gly Gly His Val Glu Leu Val
       130                 135
<210> 6
   <211> 269
   <212> PRT
   <213> artificial
<220>
   <223> GS linker plus KZ144 endolysin w/o methionine plus His tag
<400> 6
   Gly Ser Lys Val Leu Arg Lys Gly Asp Arg Gly Asp Glu Val Cys Gln
   1               5                   10                  15
   Leu Gln Thr Leu Leu Asn Leu Cys Gly Tyr Asp Val Gly Lys Pro Asp
               20                  25                  30
   Gly Ile Phe Gly Asn Asn Thr Phe Asn Gln Val Val Lys Phe Gln Lys
           35                  40                  45
   Asp Asn Cys Leu Asp Ser Asp Gly Ile Val Gly Lys Asn Thr Trp Ala
       50                  55                  60
   Glu Leu Phe Ser Lys Tyr Ser Pro Pro Ile Pro Tyr Lys Thr Ile Pro
   65                  70                  75                  80
   Met Pro Thr Ala Asn Lys Ser Arg Ala Ala Ala Thr Pro Val Met Asn
   85 90 95
   Ala Val Glu Asn Ala Thr Gly Val Arg Ser Gln Leu Leu Leu Thr Phe
   100 105 110
   Ala Ser Ile Glu Ser Ala Phe Asp Tyr Glu Ile Lys Ala Lys Thr Ser
   115 120 125
   Ser Ala Thr Gly Trp Phe Gln Phe Leu Thr Gly Thr Trp Lys Thr Met
   130 135 140
   Ile Glu Asn Tyr Gly Met Lys Tyr Gly Val Leu Thr Asp Pro Thr Gly
   145 150 155 160
   Ala Leu Arg Lys Asp Pro Arg Ile Ser Ala Leu Met Gly Ala Glu Leu
   165 170 175
   Ile Lys Glu Asn Met Asn Ile Leu Arg Pro Val Leu Lys Arg Glu Pro
   180 185 190
   Thr Asp Thr Asp Leu Tyr Leu Ala His Phe Phe Gly Pro Gly Ala Ala
   195 200 205
   Arg Arg Phe Leu Thr Thr Gly Gln Asn Glu Leu Ala Ala Thr His Phe
   210 215 220
   Pro Lys Glu Ala Gln Ala Asn Pro Ser Ile Phe Tyr Asn Lys Asp Gly
   225 230 235 240
   Ser Pro Lys Thr Ile Gln Glu Val Tyr Asn Leu Met Asp Gly Lys Val
   245 250 255
   Ala Ala His Arg Lys Leu Glu His His His His His His
   260 265
<210> 7
   <211> 136
   <212> PRT
   <213> Escherichia virus T5
<400> 7
   Ser Phe Lys Phe Gly Lys Asn Ser Glu Lys Gln Leu Ala Thr Val Lys
   1               5                   10                  15
   Pro Glu Leu Gln Lys Val Ala Arg Arg Ala Leu Glu Leu Ser Pro Tyr
               20                  25                  30
   Asp Phe Thr Ile Val Gln Gly Ile Arg Thr Val Ala Gln Ser Ala Gln
           35                  40                  45
   Asn Ile Ala Asn Gly Thr Ser Phe Leu Lys Asp Pro Ser Lys Ser Lys
       50                  55                  60
   His Ile Thr Gly Asp Ala Ile Asp Phe Ala Pro Tyr Ile Asn Gly Lys
   65                  70                  75                  80
   Ile Asp Trp Asn Asp Leu Glu Ala Phe Trp Ala Val Lys Lys Ala Phe
                   85                  90                  95
   Glu Gln Ala Gly Lys Glu Leu Gly Ile Lys Leu Arg Phe Gly Ala Asp
               100                 105                 110
   Trp Asn Ala Ser Gly Asp Tyr His Asp Glu Ile Lys Arg Gly Thr Tyr
           115                 120                 125
   Asp Gly Gly His Val Glu Leu Val
       130                 135
<210> 8
   <211> 260
   <212> PRT
   <213> Artificial
<220>
   <223> Consensus Sequence
<220>
   <221> MISC_FEATURE
   <222> (1)..(1)
   <223> Xaa can be any naturally occurring amino acid or absent; in particular it can be methionine
<220>
   <221> MISC_FEATURE
   <222> (14)..(14)
   <223> Xaa can be any naturally occurring amino acid, in particular serine, arginine or aspargine
<220>
   <221> MISC_FEATURE
   <222> (23) .. (23)
   <223> Xaa can be any naturally occurring amino acid, in particular serine, arginine or aspargine
<220>
   <221> MISC_FEATURE
   <222> (50).. (50)
   <223> Xaa can be any naturally occurring amino acid, in particular serine, arginine or aspargine
<220>
   <221> MISC_FEATURE
   <222> (82) .. (82)
   <223> Xaa can be any naturally occurring amino acid, in particular threonine or isoleucine
<220>
   <221> MISC_FEATURE
   <222> (122)..(122)
   <223> Xaa can be any naturally occurring amino acid, in particular isoleucine or methionine
<220>
   <221> MISC_FEATURE
   <222> (149)..(149)
   <223> Xaa can be any naturally occurring amino acid, in particular methionine or proline
<220>
   <221> MISC_FEATURE
   <222> (154)..(154)
   <223> Xaa can be any naturally occurring amino acid, in particular leucine or threonine
<220>
   <221> MISC_FEATURE
   <222> (160).. (160)
   <223> Xaa can be any naturally occurring amino acid, in particular alanine or threonine
<220>
   <221> MISC_FEATURE
   <222> (167)..(167)
   <223> Xaa can be any naturally occurring amino acid, in particular isoleucine or leucine
<220>
   <221> MISC_FEATURE
   <222> (179)..(179)
   <223> Xaa can be any naturally occurring amino acid, in particular asparagine or phenylalanine
<220>
   <221> MISC_FEATURE
   <222> (180)..(180)
   <223> Xaa can be any naturally occurring amino acid, in particular methionine or glutamic acid
<220>
   <221> MISC_FEATURE
   <222> (186)..(186)
   <223> Xaa can be any naturally occurring amino acid, in particular valine or tyrosine
<220>
   <221> MISC_FEATURE
   <222> (206) .. (206)
   <223> Xaa can be any naturally occurring amino acid, in particular alanine, asparagine or valine
<220>
   <221> MISC_FEATURE
   <222> (212) . . (212)
   <223> Xaa can be any naturally occurring amino acid, in particular threonine or asparagine
<220>
   <221> MISC_FEATURE
   <222> (224)..(224)
   <223> Xaa can be any naturally occurring amino acid, in particular proline or glutamine
<220>
   <221> MISC_FEATURE
   <222> (230) . . (230)
   <223> Xaa can be any naturally occurring amino acid, in particular asparagine or tyrosine
<220>
   <221> MISC_FEATURE
   <222> (232) .. (232)
   <223> Xaa can be any naturally occurring amino acid, in particular serine or threonine
<400> 8
   Xaa Lys Val Leu Arg Lys Gly Asp Arg Gly Asp Glu Val Xaa Gln Leu
   1               5                   10                  15
   Gln Thr Leu Leu Asn Leu Xaa Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Xaa Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Xaa Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Xaa Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Xaa Lys Tyr Gly Val Xaa Thr Asp Pro Thr Gly Xaa
   145                 150                 155                 160
   Leu Arg Lys Asp Pro Arg Xaa Ser Ala Leu Met Gly Ala Glu Leu Ile
                   165                 170                 175
   Lys Glu Xaa Xaa Asn Ile Leu Arg Pro Xaa Leu Lys Arg Glu Pro Thr
   180 185 190
   Asp Thr Asp Leu Tyr Leu Ala His Phe Phe Gly Pro Gly Xaa Ala Arg
   195 200 205
   Arg Phe Leu Xaa Thr Gly Gln Asn Glu Leu Ala Ala Thr His Phe Xaa
   210 215 220
   Lys Glu Ala Gln Ala Xaa Pro Xaa Ile Phe Tyr Asn Lys Asp Gly Ser
   225 230 235 240
   Pro Lys Thr Ile Gln Glu Val Tyr Asn Leu Met Asp Gly Lys Val Ala
   245 250 255
   Ala His Arg Lys
   260
<210> 9
   <211> 259
   <212> PRT
   <213> artificial sequence
<220>
   <223> mutated KZ144 with C14S and C50S, without N-terminal methionine
<400> 9
   Lys Val Leu Arg Lys Gly Asp Arg Gly Asp Glu Val Ser Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Ser Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Thr Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Ile Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Met Lys Tyr Gly Val Leu Thr Asp Pro Thr Gly Ala Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Ala Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Ser Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 10
   <211> 259
   <212> PRT
   <213> artificial sequence
<220>
   <223> mutated KZ144 with T82I, A206V and S232, without N-terminal methionine
<400> 10
   Lys Val Leu Arg Lys Gly Asp Arg Gly Asp Glu Val Cys Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Cys Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Ile Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 11
   <211> 259
   <212> PRT
   <213> artificial sequence
<220>
   <223> mutated KZ144 with T82I, A206V, S232T, I122M and A160T, without **N-terminal methionine**
<400> 11
   Lys Val Leu Arg Lys Gly Asp Arg Gly Asp Glu Val Cys Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Cys Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
   245 250 255
   His Arg Lys
<210> 12
   <211> 259
   <212> PRT
   <213> artificial sequence
<220>
   <223> mutated KZ144 with C14S, C50S, I122M and A160T, without N-terminal methionine
<400> 12
   Lys Val Leu Arg Lys Gly Asp Arg Gly Asp Glu Val Ser Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Ser Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Thr Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Met Lys Tyr Gly Val Leu Thr Asp Pro Thr Gly Thr Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Ala Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Ser Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 13
   <211> 259
   <212> PRT
   <213> artificial sequence
<220>
   <223> mutated KZ144 with C14S, C23S and C50S, without N-terminal methionine
<400> 13
   Lys Val Leu Arg Lys Gly Asp Arg Gly Asp Glu Val Ser Gln Leu Gln
   1               5                   10                  15
   Thr Leu Leu Asn Leu Ser Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Thr Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Ile Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Ala Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Ser Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 14
   <211> 259
   <212> PRT
   <213> artificial sequence
<220>
   <223> mutated KZ144 with T82I, A206V, S232T, I122M, A160T, C14S and C50S, without N-terminal methionine
<400> 14
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 15
   <211> 259
   <212> PRT
   <213> artificial sequence
<220>
   <223> mutated KZ144 with T82I, A206N, S232T, I122M, A160T, C14S and C50S, without N-terminal methionine
<400> 15
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Asn Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
   245 250 255
   His Arg Lys
<210> 16
   <211> 259
   <212> PRT
   <213> Artificial Sequence
<220>
   <223> mutated KZ144 with N230Y, T82I, A206V, S232T, I122M, A160T, C14S and C50S, without N-terminal methionine
<400> 16
   Lys Val Leu Arg Lys Gly Asp Arg Gly Asp Glu Val Ser Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Ser Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Ile Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Met Lys Tyr Gly Val Leu Thr Asp Pro Thr Gly Thr Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Tyr Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 17
   <211> 259
   <212> PRT
   <213> Artificial Sequence
<220>
   <223> mutated KZ144 with M180E, T82I, A206V, S232T, I122M, A160T, C14S and C50S, without N-terminal methionine
<400> 17
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Glu Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 18
   <211> 259
   <212> PRT
   <213> Artificial Sequence
<220>
   <223> mutated KZ144 with M149P, T82I, A206V, S232T, I122M, A160T, C14S and C50S, without N-terminal methionine
<400> 18
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 19
   <211> 259
   <212> PRT
   <213> Artificial Sequence
<220>
   <223> mutated KZ144 with V186Y, T82I, A206V, S232T, I122M, A160T, C14S and C50S, without N-terminal methionine
<400> 19
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Tyr Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 20
   <211> 259
   <212> PRT
   <213> Artificial Sequence
<220>
   <223> mutated KZ144 with T82I, A206V, S232T, I122M, A160T, C14R and C50S, without N-terminal methionine
<400> 20
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 21
   <211> 259
   <212> PRT
   <213> Artificial Sequence
<220>
   <223> mutated KZ144 with T82I, A206V, S232T, I122M, C14S and C50S, without N-terminal methionine
<400> 21
   Lys Val Leu Arg Lys Gly Asp Arg Gly Asp Glu Val Cys Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Met Lys Tyr Gly Val Leu Thr Asp Pro Thr Gly Ala Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Val Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 22
   <211> 259
   <212> PRT
   <213> Artificial Sequence
<220>
   <223> mutated KZ144 with T82I, A206V, S232T, I122M, A160T, C14S and C50N, without N-terminal methionine
<400> 22
   Lys Val Leu Arg Lys Gly Asp Arg Gly Asp Glu Val Ser Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
           35                  40                  45
   Asn Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 23
   <211> 259
   <212> PRT
   <213> Artificial Sequence
<220>
   <223> mutated KZ144 with C14R, C50S, T82I, I122M, M149P, A206V and S232T; without N-terminal methionine
<400> 23
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 24
   <211> 259
   <212> PRT
   <213> Artificial Sequence
<220>
   <223> mutated KZ144 with C14R, C50S, T82I, I122M, M149P, A160T, A206V and S232T; without N-terminal methionine
<400> 24
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Val Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 25
   <211> 259
   <212> PRT
   <213> Artificial Sequence
<220>
   <223> mutated KZ144 with T82I, I122M, M149P, A206V, S232T, C14R and C50N, without N-terminal methionine
<400> 25
   Lys Val Leu Arg Lys Gly Asp Arg Gly Asp Glu Val Arg Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Asn Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Ile Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 26
   <211> 259
   <212> PRT
   <213> Artificial Sequence
<220>
   <223> mutated KZ144 with T82I, I122M, M149P, I167L, A206V, S232T, C14R and C50N, without N-terminal methionine
<400> 26
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Asn Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Ala Leu
   145                 150                 155                 160
   Arg Lys Asp Pro Arg Leu Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 27
   <211> 259
   <212> PRT
   <213> Artificial Sequence
<220>
   <223> mutated KZ144 with T82I, I122M, M149P, N179F, A206V, S232T, C14R and C50N, without N-terminal methionine
<400> 27
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Asn Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Ala Leu
   145                 150                 155                 160
   Arg Lys Asp Pro Arg Leu Ser Ala Leu Met Gly Ala Glu Leu Ile Lys
                   165                 170                 175
   Glu Phe Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 28
   <211> 259
   <212> PRT
   <213> Artificial Sequence
<220>
   <223> mutated KZ144 with T82I, I122M, M149P, A206V, T212N, S232T, C14R and C50N, without N-terminal methionine
<400> 28
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Asn Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Asn Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 29
   <211> 259
   <212> PRT
   <213> Artificial Sequence
<220>
   <223> mutated KZ144 with T82I, I122M, M149P, A206V, P224Q, S232T, C14R and C50N, without N-terminal methionine
<400> 29
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Asn Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Pro Lys Tyr Gly Val Leu Thr Asp Pro Thr Gly Ala Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Val Ala Arg Arg
   195 200 205
   Phe Leu Thr Thr Gly Gln Asn Glu Leu Ala Ala Thr His Phe Gln Lys
   210 215 220
   Glu Ala Gln Ala Asn Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 30
   <211> 259
   <212> PRT
   <213> Artificial Sequence
<220>
   <223> mutated KZ144 with T82I, I122M, M149P, L154T, A206V, S232T, C14R and C50N, without N-terminal methionine
<400> 30
   Lys Val Leu Arg Lys Gly Asp Arg Gly Asp Glu Val Arg Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
           35                  40                  45
   Asn Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Thr Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 31
   <211> 137
   <212> PRT
   <213> Bacteriophage S-394
<400> 31
   Met Ser Phe Lys Phe Gly Lys Asn Ser Glu Lys Gln Leu Ala Thr Val
   1 5 10 15
   Lys Pro Glu Leu Gln Lys Val Ala Arg Arg Ala Leu Glu Leu Ser Pro
   20 25 30
   Tyr Asp Phe Thr Ile Val Gln Gly Ile Arg Thr Val Ala Gln Ser Ala
   35 40 45
   Gln Asn Ile Ala Asn Gly Thr Ser Phe Leu Lys Asp Pro Ser Lys Ser
   50 55 60
   Lys His Val Thr Gly Asp Ala Ile Asp Phe Ala Pro Tyr Ile Asn Gly
   65 70 75 80
   Lys Ile Asp Trp Lys Asp Leu Glu Ala Phe Trp Ala Val Lys Lys Ala
   85 90 95
   Phe Glu Gln Ala Gly Lys Glu Leu Gly Ile Lys Leu Arg Phe Gly Ala
   100 105 110
   Asp Trp Asn Ser Ser Gly Asp Tyr His Asp Glu Ile Asp Arg Gly Thr
   115 120 125
   Tyr Asp Gly Gly His Val Glu Leu Val
   130 135
<210> 32
   <211> 260
   <212> PRT
   <213> unknown
<220>
   <223> phiKZgp144
<400> 32
   Met Lys Val Leu Arg Lys Gly Asp Arg Gly Asp Glu Val Cys Gln Leu
   1 5 10 15
   Gln Thr Leu Leu Asn Leu Cys Gly Tyr Asp Val Gly Lys Pro Asp Gly
   20 25 30
   Ile Phe Gly Asn Asn Thr Phe Asn Gln Val Val Lys Phe Gln Lys Asp
   35 40 45
   Asn Cys Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Thr Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Ile Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Ala Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Ser Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 33
   <211> 260
   <212> PRT
   <213> artificial sequence
<220>
   <223> mutated KZ144 with C14S, C23S and C50S
<400> 33
   Met Lys Val Leu Arg Lys Gly Asp Arg Gly Asp Glu Val Ser Gln Leu
   1               5                   10                  15
   Gln Thr Leu Leu Asn Leu Ser Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Thr Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Ile Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Ala Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Ser Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 34
   <211> 327
   <212> PRT
   <213> unknown
<220>
   <223> OBPgp279
<400> 34
   Lys Asn Ser Glu Lys Asn Ala Ser Ile Ile Met Ser Ile Gln Arg Thr
   1               5                   10                  15
   Leu Ala Ser Leu Ser Leu Tyr Gly Gly Arg Ile Asp Gly Leu Phe Gly
               20                  25                  30
   Glu Lys Cys Arg Gly Ala Ile Ile Leu Met Leu Asn Lys Val Tyr Pro
           35                  40                  45
   Asn Phe Ser Thr Asn Lys Leu Pro Ser Asn Thr Tyr Glu Ala Glu Ser
       50                  55                  60
   Val Phe Thr Phe Leu Gln Thr Ala Leu Ala Gly Val Gly Leu Tyr Thr
   65                  70                  75                  80
   Ile Thr Ile Asp Gly Lys Trp Gly Gly Thr Ser Gln Gly Ala Ile Asp
                   85                  90                  95
   Ala Leu Val Lys Ser Tyr Arg Gln Ile Thr Glu Ala Glu Arg Ala Gly
               100                 105                 110
   Ser Thr Leu Pro Leu Gly Leu Ala Thr Val Met Ser Lys His Met Ser
           115                 120                 125
   Ile Glu Gln Leu Arg Ala Met Leu Pro Thr Asp Arg Gln Gly Tyr Ala
       130                 135                 140
   Glu Val Tyr Ile Asp Pro Leu Asn Glu Thr Met Asp Ile Phe Glu Ile
   145                 150                 155                 160
   Asn Thr Pro Leu Arg Ile Ala His Phe Met Ala Gln Ile Leu His Glu
                   165                 170                 175
   Thr Ala Cys Phe Lys Tyr Thr Glu Glu Leu Ala Ser Gly Lys Ala Tyr
   180 185 190
   Glu Gly Arg Ala Asp Leu Gly Asn Thr Arg Pro Gly Asp Gly Pro Leu
   195 200 205
   Phe Lys Gly Arg Gly Leu Leu Gln Ile Thr Gly Arg Leu Asn Tyr Val
   210 215 220
   Lys Cys Gln Val Tyr Leu Arg Glu Lys Leu Lys Asp Pro Thr Phe Asp
   225 230 235 240
   Ile Thr Ser Ser Val Thr Cys Ala Gln Gln Leu Ser Glu Ser Pro Leu
   245 250 255
   Leu Ala Ala Leu Ala Ser Gly Tyr Phe Trp Arg Phe Ile Lys Pro Lys
   260 265 270
   Leu Asn Glu Thr Ala Asp Lys Asp Asp Ile Tyr Trp Val Ser Val Tyr
   275 280 285
   Val Asn Gly Tyr Ala Lys Gln Ala Asn Pro Tyr Tyr Pro Asn Arg Asp
   290 295 300
   Lys Glu Pro Asn His Met Lys Glu Arg Val Gln Met Leu Ala Val Thr
   305 310 315 320
   Lys Lys Ala Leu Gly Ile Val
   325
<210> 35
   <211> 235
   <212> PRT
   <213> unknown
<220>
   <223> PVP-SE1gp146
<400> 35
   Asn Ala Ala Ile Ala Glu Ile Gln Arg Met Leu Ile Glu Gly Gly Phe
   1               5                   10                  15
   Ser Val Gly Lys Ser Gly Ala Asp Gly Leu Tyr Gly Pro Ala Thr Lys
               20                  25                  30
   Ala Ala Leu Gln Lys Cys Ile Ala Gln Ala Thr Ser Gly Asn Asn Lys
           35                  40                  45
   Gly Gly Thr Leu Lys Leu Thr Gln Ala Gln Leu Asp Lys Ile Phe Pro
       50                  55                  60
   Val Gly Ala Ser Ser Gly Arg Asn Ala Lys Phe Leu Lys Pro Leu Asn
   65                  70                  75                  80
   Asp Leu Phe Glu Lys Thr Glu Ile Asn Thr Val Asn Arg Val Ala Gly
                   85                  90                  95
   Phe Leu Ser Gln Ile Gly Val Glu Ser Ala Glu Phe Arg Tyr Val Arg
               100                 105                 110
   Glu Leu Gly Asn Asp Ala Tyr Phe Asp Lys Tyr Asp Thr Gly Pro Ile
           115                 120                 125
   Ala Glu Arg Leu Gly Asn Thr Pro Gln Lys Asp Gly Asp Gly Ala Lys
       130                 135                 140
   Tyr Lys Gly Arg Gly Leu Ile Gln Val Thr Gly Leu Ala Asn Tyr Lys
   145                 150                 155                 160
   Ala Cys Gly Lys Ala Leu Gly Leu Asp Leu Val Asn His Pro Glu Leu
                   165                 170                 175
   Leu Glu Gln Pro Glu Tyr Ala Val Ala Ser Ala Gly Trp Tyr Trp Asp
               180                 185                 190
   Thr Arg Asn Ile Asn Ala Ala Cys Asp Ala Asp Asp Ile Val Lys Ile
           195                 200                 205
   Thr Lys Leu Val Asn Gly Gly Thr Asn His Leu Ala Glu Arg Thr Ala
       210                 215                 220
   Tyr Tyr Lys Lys Ala Lys Ser Val Leu Thr Ser
   225                 230                 235
<210> 36
   <211> 300
   <212> PRT
   <213> artificial
<220>
   <223> SEQ ID NO: 124 of WO2015/155244
<400> 36
   Met Lys Phe Phe Arg Lys Leu Lys Lys Ser Val Lys Lys Arg Ala Lys
   1               5                   10                  15
   Glu Phe Phe Lys Lys Pro Arg Val Ile Gly Val Ser Ile Pro Phe Gly
               20                  25                  30
   Ser Lys Val Leu Arg Lys Gly Asp Arg Gly Asp Glu Val Cys Gln Leu
   35 40 45
   Gln Thr Leu Leu Asn Leu Cys Gly Tyr Asp Val Gly Lys Pro Asp Gly
   50 55 60
   Ile Phe Gly Asn Asn Thr Phe Asn Gln Val Val Lys Phe Gln Lys Asp
   65 70 75 80
   Asn Cys Leu Asp Ser Asp Gly Ile Val Gly Lys Asn Thr Trp Ala Glu
   85 90 95
   Leu Phe Ser Lys Tyr Ser Pro Pro Ile Pro Tyr Lys Thr Ile Pro Met
   100 105 110
   Pro Thr Ala Asn Lys Ser Arg Ala Ala Ala Thr Pro Val Met Asn Ala
   115 120 125
   Val Glu Asn Ala Thr Gly Val Arg Ser Gln Leu Leu Leu Thr Phe Ala
   130 135 140
   Ser Ile Glu Ser Ala Phe Asp Tyr Glu Ile Lys Ala Lys Thr Ser Ser
   145 150 155 160
   Ala Thr Gly Trp Phe Gln Phe Leu Thr Gly Thr Trp Lys Thr Met Ile
   165 170 175
   Glu Asn Tyr Gly Met Lys Tyr Gly Val Leu Thr Asp Pro Thr Gly Ala
   180 185 190
   Leu Arg Lys Asp Pro Arg Ile Ser Ala Leu Met Gly Ala Glu Leu Ile
   195 200 205
   Lys Glu Asn Met Asn Ile Leu Arg Pro Val Leu Lys Arg Glu Pro Thr
   210 215 220
   Asp Thr Asp Leu Tyr Leu Ala His Phe Phe Gly Pro Gly Ala Ala Arg
   225 230 235 240
   Arg Phe Leu Thr Thr Gly Gln Asn Glu Leu Ala Ala Thr His Phe Pro
   245 250 255
   Lys Glu Ala Gln Ala Asn Pro Ser Ile Phe Tyr Asn Lys Asp Gly Ser
   260 265 270
   Pro Lys Thr Ile Gln Glu Val Tyr Asn Leu Met Asp Gly Lys Val Ala
   275 280 285
   Ala His Arg Lys Leu Glu His His His His His His
   290 295 300
<210> 37
   <211> 17
   <212> PRT
   <213> Artificial Sequence
<220>
   <223> MSI-78 (4-20) peptide
<400> 37
   Lys Phe Leu Lys Lys Ala Lys Lys Phe Gly Lys Ala Phe Val Lys Ile
   1 5 10 15
   Leu
<210> 38
   <211> 6
   <212> PRT
   <213> artificial
<220>
   <223> synthetic sequence
<400> 38
   Lys Arg Lys Lys Arg Lys
   1 5
<210> 39
   <211> 5
   <212> PRT
   <213> artificial
<220>
   <223> synethtic sequence
<220>
   <221> misc_feature
   <222> (3)..(3)
   <223> Xaa can be any naturally occurring amino acid
<400> 39
   Lys Arg Xaa Lys Arg
   1 5
<210> 40
   <211> 5
   <212> **PRT**
   <213> artificial
<220>
   <223> synthetic sequence
<400> 40
   Lys Arg Ser Lys Arg
   1               5
<210> 41
   <211> 5
   <212> PRT
   <213> artificial
<220>
   <223> synthetic sequence
<400> 41
   Lys Arg Gly Ser Gly
   1               5
<210> 42
   <211> 9
   <212> PRT
   <213> artificial
<220>
   <223> synthetic sequence
<400> 42
   Lys Arg Lys Lys Arg Lys Lys Arg Lys
   1               5
<210> 43
   <211> 9
   <212> PRT
   <213> artificial
<220>
   <223> synthetic sequence
<400> 43
   Arg Arg Arg Arg Arg Arg Arg Arg Arg
   1               5
<210> 44
   <211> 8
   <212> PRT
   <213> artificial
<220>
   <223> synthetic sequence
<400> 44
   Lys Lys Lys Lys Lys Lys Lys Lys
   1               5
<210> 45
   <211> 10
   <212> PRT
   <213> artificial
<220>
   <223> synthetic sequence
<400> 45
   Lys Arg Lys Lys Arg Lys Lys Arg Lys Lys
   1               5                   10
<210> 46
   <211> 12
   <212> PRT
   <213> artificial
<220>
   <223> synthetic sequence
<400> 46
   Lys Arg Lys Lys Arg Lys Lys Arg Lys Lys Arg Lys
   1               5                   10
<210> 47
   <211> 14
   <212> PRT
   <213> artificial
<220>
   <223> synthetic sequence
<400> 47
   Lys Arg Lys Lys Arg Lys Lys Arg Lys Lys Arg Lys Lys Arg
   1               5                   10
<210> 48
   <211> 16
   <212> PRT
   <213> artificial
<220>
   <223> synthetic sequence
<400> 48
   Lys Lys Lys Lys Lys Lys Lys Lys Lys Lys Lys Lys Lys Lys Lys Lys
   1               5                   10                  15
<210> 49
   <211> 18
   <212> PRT
   <213> artificial
<220>
   <223> synthetic sequence
<400> 49
   Lys Arg Lys Lys Arg Lys Lys Arg Lys Lys Arg Lys Lys Arg Lys Lys
   1               5                   10                  15
   Arg Lys
<210> 50
   <211> 19
   <212> PRT
   <213> artificial
<220>
   <223> synthetic sequence
<400> 50
   Lys Arg Lys Lys Arg Lys Lys Arg Lys Lys Arg Lys Lys Arg Lys Lys
   1               5                   10                  15
   Arg Lys Lys
<210> 51
   <211> 19
   <212> PRT
   <213> artificial
<220>
   <223> synthetic sequence
<400> 51
   Arg Arg Arg Arg Arg Arg Arg Arg Arg Arg Arg Arg Arg Arg Arg Arg
   1               5                   10                  15
   Arg Arg Arg
<210> 52
   <211> 19
   <212> PRT
   <213> artificial
<220>
   <223> synthetic sequence
<400> 52
   Lys Lys Lys Lys Lys Lys Lys Lys Lys Lys Lys Lys Lys Lys Lys Lys
   1               5                   10                  15
   Lys Lys Lys
<210> 53
   <211> 20
   <212> PRT
   <213> artificial
<220>
   <223> synthetic sequence
<400> 53
   Lys Arg Lys Lys Arg Lys Lys Arg Lys Arg Ser Lys Arg Lys Lys Arg
   1               5                   10                  15
   Lys Lys Arg Lys
               20
<210> 54
   <211> 21
   <212> PRT
   <213> artificial
<220>
   <223> synthetic sequence
<400> 54
   Lys Arg Lys Lys Arg Lys Lys Arg Lys Arg Ser Lys Arg Lys Lys Arg
   1               5                   10                  15
   Lys Lys Arg Lys Lys
               20
<210> 55
   <211> 21
   <212> PRT
   <213> artificial
<220>
   <223> synthetic sequence
<400> 55
   Lys Arg Lys Lys Arg Lys Lys Arg Lys Lys Arg Lys Lys Arg Lys Lys
   1               5                   10                  15
   Arg Lys Lys Arg Lys
               20
<210> 56
   <211> 22
   <212> PRT
   <213> artificial
<220>
   <223> synthetic sequence
<400> 56
   Lys Arg Lys Lys Arg Lys Lys Arg Lys Arg Gly Ser Gly Lys Arg Lys
   1 5 10 15
   Lys Arg Lys Lys Arg Lys
   20
<210> 57
   <211> 24
   <212> PRT
   <213> artificial
<220>
   <223> synthetic sequence
<400> 57
   Lys Arg Lys Lys Arg Lys Lys Arg Lys Arg Gly Ser Gly Ser Gly Lys
   1 5 10 15
   Arg Lys Lys Arg Lys Lys Arg Lys
   20
<210> 58
   <211> 25
   <212> PRT
   <213> artificial
<220>
   <223> synthetic sequence
<400> 58
   Lys Arg Lys Lys Arg Lys Lys Arg Lys Lys Arg Lys Lys Arg Lys Lys
   1 5 10 15
   Arg Lys Lys Arg Lys Lys Arg Lys Lys
   20 25
<210> 59
   <211> 31
   <212> PRT
   <213> artificial
<220>
   <223> synthetic sequence
<400> 59
   Lys Arg Lys Lys Arg Lys Lys Arg Lys Arg Ser Lys Arg Lys Lys Arg
   1 5 10 15
   Lys Lys Arg Lys Arg Ser Lys Arg Lys Lys Arg Lys Lys Arg Lys
   20 25 30
<210> 60
   <211> 38
   <212> PRT
   <213> artificial
<220>
   <223> synthetic sequence
<400> 60
   Lys Arg Lys Lys Arg Lys Lys Arg Lys Arg Gly Ser Gly Ser Gly Lys
   1               5                   10                  15
   Arg Lys Lys Arg Lys Lys Arg Lys Gly Ser Gly Ser Gly Lys Arg Lys
               20                  25                  30
   Lys Arg Lys Lys Arg Lys
           35
<210> 61
   <211> 39
   <212> PRT
   <213> artificial
<220>
   <223> synthetic sequence
<400> 61
   Lys Arg Lys Lys Arg Lys Lys Arg Lys Lys Arg Lys Lys Arg Lys Lys
   1               5                   10                  15
   Arg Lys Lys Arg Lys Lys Arg Lys Lys Arg Lys Lys Arg Lys Lys Arg
               20                  25                  30
   Lys Lys Arg Lys Lys Arg Lys
           35
<210> 62
   <211> 42
   <212> PRT
   <213> artificial
<220>
   <223> synthetic sequence
<400> 62
   Lys Arg Lys Lys Arg Lys Lys Arg Lys Arg Ser Lys Arg Lys Lys Arg
   1               5                   10                  15
   Lys Lys Arg Lys Arg Ser Lys Arg Lys Lys Arg Lys Lys Arg Lys Arg
   20 25 30
   Ser Lys Arg Lys Lys Arg Lys Lys Arg Lys
   35 40
<210> 63
   <211> 37
   <212> PRT
   <213> Homo sapiens
<400> 63
   Leu Leu Gly Asp Phe Phe Arg Lys Ser Lys Glu Lys Ile Gly Lys Glu
   1 5 10 15
   Phe Lys Arg Ile Val Gln Arg Ile Lys Asp Phe Leu Arg Asn Leu Val
   20 25 30
   Pro Arg Thr Glu Ser
   35
<210> 64
   <211> 29
   <212> PRT
   <213> unknown
<220>
   <223> SMAP-29 sheep
<400> 64
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly
   20 25
<210> 65
   <211> 13
   <212> PRT
   <213> unknown
<220>
   <223> Indolicidine bovine
<400> 65
   Ile Leu Pro Trp Lys Trp Pro Trp Trp Pro Trp Arg Arg
   1 5 10
<210> 66
   <211> 18
   <212> PRT
   <213> unknown
<220>
   <223> Protegrin Porcine
<400> 66
   Arg Gly Gly Arg Leu Cys Tyr Cys Arg Arg Arg Phe Cys Val Cys Val
   1 5 10 15
   Gly Arg
<210> 67
   <211> 31
   <212> PRT
   <213> unknown
<220>
   <223> Cecropin P1 Mammal (pig)
<400> 67
   Ser Trp Leu Ser Lys Thr Ala Lys Lys Leu Glu Asn Ser Ala Lys Lys
   1 5 10 15
   Arg Ile Ser Glu Gly Ile Ala Ile Ala Ile Gln Gly Gly Pro Arg
   20 25 30
<210> 68
   <211> 23
   <212> PRT
   <213> unknown
<220>
   <223> Magainin frog
<400> 68
   Gly Ile Gly Lys Phe Leu His Ser Ala Lys Lys Phe Gly Lys Ala Phe
   1 5 10 15
   Val Gly Glu Ile Met Asn Ser
   20
<210> 69
   <211> 25
   <212> PRT
   <213> unknown
<220>
   <223> Pleurocidin fish
<400> 69
   Gly Trp Gly Ser Phe Phe Lys Lys Ala Ala His Val Gly Lys His Val
   1 5 10 15
   Gly Lys Ala Ala Leu Thr His Tyr Leu
   20 25
<210> 70
   <211> 36
   <212> PRT
   <213> Aedes aegypti
<400> 70
   Gly Gly Leu Lys Lys Leu Gly Lys Lys Leu Glu Gly Ala Gly Lys Arg
   1 5 10 15
   Val Phe Asn Ala Ala Glu Lys Ala Leu Pro Val Val Ala Gly Ala Lys
   20 25 30
   Ala Leu Arg Lys
   35
<210> 71
   <211> 40
   <212> **PRT**
   <213> Drosophila melanogaster
<400> 71
   Gly Trp Leu Lys Lys Ile Gly Lys Lys Ile Glu Arg Val Gly Gln His
   1 5 10 15
   Thr Arg Asp Ala Thr Ile Gln Gly Leu Gly Ile Pro Gln Gln Ala Ala
   20 25 30
   Asn Val Ala Ala Thr Ala Arg Gly
   35 40
<210> 72
   <211> 21
   <212> PRT
   <213> unknown
<220>
   <223> Buforin II vertebrate
<400> 72
   Thr Arg Ser Ser Arg Ala Gly Leu Gln Phe Pro Val Gly Arg Val His
   1 5 10 15
   Arg Leu Leu Arg Lys
   20
<210> 73
   <211> 39
   <212> **PRT**
   <213> unknown
<220>
   <223> Sarcotoxin IA Fly
<400> 73
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Ala Gln Gln Ala Ala
               20                  25                  30
   Asn Val Ala Ala Thr Ala Arg
           35
<210> 74
   <211> 17
   <212> **PRT**
   <213> Apis mellifera
<400> 74
   Ala Asn Arg Pro Val Tyr Ile Pro Pro Pro Arg Pro Pro His Pro Arg
   1               5                   10                  15
   Leu
<210> 75
   <211> 24
   <212> PRT
   <213> unknown
<220>
   <223> Ascaphine 5 Frog
<400> 75
   Gly Ile Lys Asp Trp Ile Lys Gly Ala Ala Lys Lys Leu Ile Lys Thr
   1               5                   10                  15
   Val Ala Ser His Ile Ala Asn Gln
               20
<210> 76
   <211> 22
   <212> PRT
   <213> unknown
<220>
   <223> Nigrocine 2 Frog
<400> 76
   Gly Leu Leu Ser Lys Val Leu Gly Val Gly Lys Lys Val Leu Cys Gly
   1 5 10 15
   Val Ser Gly Leu Val Cys
   20
<210> 77
   <211> 24
   <212> PRT
   <213> unknown
<220>
   <223> Pseudin 1 Rana Frog
<400> 77
   Gly Leu Asn Thr Leu Lys Lys Val Phe Gln Gly Leu His Glu Ala Ile
   1 5 10 15
   Lys Leu Ile Asn Asn His Val Gln
   20
<210> 78
   <211> 18
   <212> PRT
   <213> unknown
<220>
   <223> Ranalexin Frog
<400> 78
   Phe Leu Gly Gly Leu Ile Val Pro Ala Met Ile Cys Ala Val Thr Lys
   1 5 10 15
   Lys Cys
<210> 79
   <211> 26
   <212> PRT
   <213> unknown
<220>
   <223> Melittin bee
<400> 79
   Gly Ile Gly Ala Val Leu Lys Val Leu Thr Thr Gly Leu Pro Ala Leu
   1 5 10 15
   Ile Ser Trp Ile Lys Arg Lys Arg Gln Gln
   20 25
<210> 80
   <211> 25
   <212> PRT
   <213> unknown
<220>
   <223> Lycotoxin 1 Spider
<400> 80
   Ile Trp Leu Thr Ala Leu Lys Phe Leu Gly Lys His Ala Ala Lys Lys
   1               5                   10                  15
   Leu Ala Lys Gln Gln Leu Ser Lys Leu
               20                  25
<210> 81
   <211> 19
   <212> PRT
   <213> unknown
<220>
   <223> Parasin 1 Fish
<400> 81
   Lys Gly Arg Gly Lys Gln Gly Gly Lys Val Arg Ala Lys Ala Lys Thr
   1               5                   10                  15
   Arg Ser Ser
<210> 82
   <211> 39
   <212> PRT
   <213> unknown
<220>
   <223> Buforin I Toad
<400> 82
   Ala Gly Arg Gly Lys Gln Gly Gly Lys Val Arg Ala Lys Ala Lys Thr
   1               5                   10                  15
   Arg Ser Ser Arg Ala Gly Leu Gln Phe Pro Val Gly Arg Val His Arg
               20                  25                  30
   Leu Leu Arg Lys Gly Asn Tyr
           35
<210> 83
   <211> 34
   <212> PRT
   <213> unknown
<220>
   <223> Dermaseptin 1 Frog
<400> 83
   Ala Leu Trp Lys Thr Met Leu Lys Lys Leu Gly Thr Met Ala Leu His
   1               5                   10                  15
   Ala Gly Lys Ala Ala Leu Gly Ala Ala Ala Asp Thr Ile Ser Gln Gly
               20                  25                  30
   Thr Gln
<210> 84
   <211> 12
   <212> PRT
   <213> unknown
<220>
   <223> Bactenecin 1 Cow
<400> 84
   Arg Leu Cys Arg Ile Val Val Ile Arg Val Cys Arg
   1               5                   10
<210> 85
   <211> 21
   <212> PRT
   <213> unknown
<220>
   <223> Thanatin Insect
<400> 85
   Gly Ser Lys Lys Pro Val Pro Ile Ile Tyr Cys Asn Arg Arg Thr Gly
   1               5                   10                  15
   Lys Cys Gln Arg Met
               20
<210> 86
   <211> 19
   <212> PRT
   <213> unknown
<220>
   <223> Brevinin 1T Rana frogs
<400> 86
   Val Asn Pro Ile Ile Leu Gly Val Leu Pro Lys Val Cys Leu Ile Thr
   1               5                   10                  15
   Lys Lys Cys
<210> 87
   <211> 26
   <212> PRT
   <213> unknown
<220>
   <223> Ranateurin 1 Rana frog
<400> 87
   Ser Met Leu Ser Val Leu Lys Asn Leu Gly Lys Val Gly Leu Gly Phe
   1               5                   10                  15
   Val Ala Cys Lys Ile Asn Ile Lys Gln Cys
               20                  25
<210> 88
   <211> 46
   <212> PRT
   <213> unknown
<220>
   <223> Esculentin 1 Rana frogs
<400> 88
   Gly Ile Phe Ser Lys Leu Gly Arg Lys Lys Ile Lys Asn Leu Leu Ile
   1               5                   10                  15
   Ser Gly Leu Lys Asn Val Gly Lys Glu Val Gly Met Asp Val Val Arg
               20                  25                  30
   Thr Gly Ile Lys Ile Ala Gly Cys Lys Ile Lys Gly Glu Cys
           35                  40                  45
<210> 89
   <211> 17
   <212> PRT
   <213> Limulus polyphemus
<400> 89
   Arg Trp Cys Phe Arg Val Cys Tyr Arg Gly Ile Cys Tyr Arg Lys Cys
   1               5                   10                  15
   Arg
<210> 90
   <211> 25
   <212> PRT
   <213> unknown
<220>
   <223> Androctonin Scorpion
<400> 90
   Arg Ser Val Cys Arg Gln Ile Lys Ile Cys Arg Arg Arg Gly Gly Cys
   1               5                   10                  15
   Tyr Tyr Lys Cys Thr Asn Arg Pro Tyr
               20                  25
<210> 91
   <211> 30
   <212> PRT
   <213> Homo sapiens
<400> 91
   Asp Cys Tyr Cys Arg Ile Pro Ala Cys Ile Ala Gly Glu Arg Arg Tyr
   1               5                   10                  15
   Gly Thr Cys Ile Tyr Gln Gly Arg Leu Trp Ala Phe Cys Cys
               20                  25                  30
<210> 92
   <211> 38
   <212> PRT
   <213> unknown
<220>
   <223> beta-defensin cow
<400> 92
   Asn Pro Val Ser Cys Val Arg Asn Lys Gly Ile Cys Val Pro Ile Arg
   1               5                   10                  15
   Cys Pro Gly Ser Met Lys Gln Ile Gly Thr Cys Val Gly Arg Ala Val
               20                  25                  30
   Lys Cys Cys Arg Lys Lys
           35
<210> 93
   <211> 18
   <212> PRT
   <213> unknown
<220>
   <223> theta-defensin monkey
<400> 93
   Gly Phe Cys Arg Cys Leu Cys Arg Arg Gly Val Cys Arg Cys Ile Cys
   1               5                   10                  15
   Thr Arg
<210> 94
   <211> 40
   <212> PRT
   <213> unknown
<220>
   <223> defensin (sapecin A) insect
<400> 94
   Ala Thr Cys Asp Leu Leu Ser Gly Thr Gly Ile Asn His Ser Ala Cys
   1               5                   10                  15
   Ala Ala His Cys Leu Leu Arg Gly Asn Arg Gly Gly Tyr Cys Asn Gly
               20                  25                  30
   Lys Ala Val Cys Val Cys Arg Asn
           35                  40
<210> 95
   <211> 46
   <212> PRT
   <213> unknown
<220>
   <223> Thionin (crambin) plant
<400> 95
   Thr Thr Cys Cys Pro Ser Ile Val Ala Arg Ser Asn Phe Asn Val Cys
   1               5                   10                  15
   Arg Ile Pro Gly Thr Pro Glu Ala Ile Cys Ala Thr Tyr Thr Gly Cys
               20                  25                  30
   Ile Ile Ile Pro Gly Ala Thr Cys Pro Gly Asp Tyr Ala Asn
           35                  40                  45
<210> 96
   <211> 50
   <212> PRT
   <213> unknown
<220>
   <223> defensin from radish
<400> 96
   Gln Lys Leu Cys Gln Arg Pro Ser Gly Thr Trp Ser Gly Val Cys Gly
   1               5                   10                  15
   Asn Asn Asn Ala Cys Lys Asn Gln Cys Ile Arg Leu Glu Lys Ala Arg
               20                  25                  30
   His Gly Ser Cys Asn Tyr Val Phe Pro Ala His Cys Ile Cys Tyr Phe
           35                  40                  45
   Pro Cys
       50
<210> 97
   <211> 44
   <212> PRT
   <213> Drosophila melanogaster
<400> 97
   Asp Cys Leu Ser Gly Arg Tyr Lys Gly Pro Cys Ala Val Trp Asp Asn
   1               5                   10                  15
   Glu Thr Cys Arg Arg Val Cys Lys Glu Glu Gly Arg Ser Ser Gly His
               20                  25                  30
   Cys Ser Pro Ser Leu Lys Cys Trp Cys Glu Gly Cys
           35                  40
<210> 98
   <211> 25
   <212> PRT
   <213> Homo sapiens
<400> 98
   Asp Thr His Phe Pro Ile Cys Ile Phe Cys Cys Gly Cys Cys His Arg
   1               5                   10                  15
   Ser Lys Cys Gly Met Cys Cys Lys Thr
               20                  25
<210> 99
   <211> 44
   <212> PRT
   <213> unknown
<220>
   <223> Bac 5 Cow
<400> 99
   Arg Phe Arg Pro Pro Ile Arg Arg Pro Pro Ile Arg Pro Pro Phe Tyr
   1               5                   10                  15
   Pro Pro Phe Arg Pro Pro Ile Arg Pro Pro Ile Phe Pro Pro Ile Arg
               20                  25                  30
   Pro Pro Phe Arg Pro Pro Leu Gly Arg Pro Phe Pro
           35                  40
<210> 100
   <211> 39
   <212> PRT
   <213> unknown
<220>
   <223> PR-39 Pig
<400> 100
   Arg Arg Arg Pro Arg Pro Pro Tyr Leu Pro Arg Pro Arg Pro Pro Pro
   1               5                   10                  15
   Phe Phe Pro Pro Arg Leu Pro Pro Arg Ile Pro Pro Gly Phe Pro Pro
               20                  25                  30
   Arg Phe Pro Pro Arg Phe Pro
           35
<210> 101
   <211> 20
   <212> PRT
   <213> unknown
<220>
   <223> Pyrrhocoricin Insect
<400> 101
   Val Asp Lys Gly Ser Tyr Leu Pro Arg Pro Thr Pro Pro Arg Pro Ile
   1               5                   10                  15
   Tyr Asn Arg Asn
               20
<210> 102
   <211> 24
   <212> PRT
   <213> Homo sapiens
<400> 102
   Asp Ser His Ala Lys Arg His His Gly Tyr Lys Arg Lys Phe His Glu
   1 5 10 15
   Lys His His Ser His Arg Gly Tyr
   20
<210> 103
   <211> 19
   <212> PRT
   <213> Unknown
<220>
   <223> ECP19
<400> 103
   Arg Pro Pro Gln Phe Thr Arg Ala Gln Trp Phe Ala Ile Gln His Ile
   1 5 10 15
   Ser Leu Asn
<210> 104
   <211> 23
   <212> PRT
   <213> Unknown
<220>
   <223> MSI-594
<400> 104
   Gly Ile Gly Lys Phe Leu Lys Lys Ala Lys Lys Gly Ile Gly Ala Val
   1 5 10 15
   Leu Lys Val Leu Thr Thr Gly
   20
<210> 105
   <211> 35
   <212> PRT
   <213> Unknown
<220>
   <223> TL-ColM
<400> 105
   Met Glu Thr Leu Thr Val His Ala Pro Ser Pro Ser Thr Asn Leu Pro
   1 5 10 15
   Ser Tyr Gly Asn Gly Ala Phe Ser Leu Ser Ala Pro His Val Pro Gly
   20 25 30
   Ala Gly Pro
   35
<210> 106
   <211> 18
   <212> PRT
   <213> Unknown
<220>
   <223> SBO
<400> 106
   Lys Leu Lys Lys Ile Ala Gln Lys Ile Lys Asn Phe Phe Ala Lys Leu
   1 5 10 15
   Val Ala
<210> 107
   <211> 26
   <212> PRT
   <213> unknown
<220>
   <223> Macedocin
<400> 107
   Gly Lys Asn Gly Val Phe Lys Thr Ile Ser His Glu Cys His Leu Asn
   1 5 10 15
   Thr Trp Ala Phe Leu Ala Thr Cys Cys Ser
   20 25
<210> 108
   <211> 22
   <212> PRT
   <213> unknown
<220>
   <223> Macedocin (Trunc)
<400> 108
   Gly Lys Asn Gly Val Phe Lys Thr Ile Ser His Glu Cys His Leu Asn
   1               5                   10                  15
   Thr Trp Ala Phe Leu Ala
               20
<210> 109
   <211> 28
   <212> PRT
   <213> unknown
<220>
   <223> D16
<400> 109
   Ala Cys Lys Leu Lys Ser Leu Leu Lys Thr Leu Ser Lys Ala Lys Lys
   1               5                   10                  15
   Lys Lys Leu Lys Thr Leu Leu Lys Ala Leu Ser Lys
               20                  25
<210> 110
   <211> 17
   <212> PRT
   <213> unknown
<220>
   <223> CPF-C1
<400> 110
   Gly Phe Gly Ser Leu Leu Gly Lys Ala Leu Arg Leu Gly Ala Asn Val
   1               5                   10                  15
   Leu
<210> 111
   <211> 34
   <212> PRT
   <213> unknown
<220>
   <223> TL-ColM
<400> 111
   Glu Thr Leu Thr Val His Ala Pro Ser Pro Ser Thr Asn Leu Pro Ser
   1 5 10 15
   Tyr Gly Asn Gly Ala Phe Ser Leu Ser Ala Pro His Val Pro Gly Ala
   20 25 30
   Gly Pro
<210> 112
   <211> 26
   <212> PRT
   <213> unknown
<220>
   <223> TM-174E
<400> 112
   Leu Ile Ser Lys Gly Trp Pro Tyr Leu Leu Val Val Val Leu Gly Ala
   1 5 10 15
   Thr Ile Tyr Phe Trp Gly Asn Ser Asn Gly
   20 25
<210> 113
   <211> 45
   <212> PRT
   <213> unknown
<220>
   <223> ECP45
<400> 113
   Arg Pro Pro Gln Phe Thr Arg Ala Gln Trp Phe Ala Ile Gln His Ile
   1 5 10 15
   Ser Leu Asn Pro Pro Arg Cys Thr Ile Ala Met Arg Ala Ile Asn Asn
   20 25 30
   Tyr Arg Trp Arg Cys Lys Asn Gln Asn Thr Phe Leu Arg
   35 40 45
<210> 114
   <211> 50
   <212> PRT
   <213> unknown
<220>
   <223> ColicinE3_1-51 (S37F)
<400> 114
   Ser Gly Gly Asp Gly Arg Gly His Asn Thr Gly Ala His Ser Thr Ser
   1               5                   10                  15
   Gly Asn Ile Asn Gly Gly Pro Thr Gly Leu Gly Val Gly Gly Gly Ala
               20                  25                  30
   Ser Asp Gly Phe Gly Trp Ser Ser Glu Asn Asn Pro Trp Gly Gly Gly
           35                  40                  45
   Ser Gly
       50
<210> 115
   <211> 68
   <212> PRT
   <213> unknown
<220>
   <223> ColicinE3_1-69 (S37F)
<400> 115
   Ser Gly Gly Asp Gly Arg Gly His Asn Thr Gly Ala His Ser Thr Ser
   1               5                   10                  15
   Gly Asn Ile Asn Gly Gly Pro Thr Gly Leu Gly Val Gly Gly Gly Ala
               20                  25                  30
   Ser Asp Gly Phe Gly Trp Ser Ser Glu Asn Asn Pro Trp Gly Gly Gly
           35                  40                  45
   Ser Gly Ser Gly Ile His Trp Gly Gly Gly Ser Gly His Gly Asn Gly
       50                  55                  60
   Gly Gly Asn Gly
   65
<210> 116
   <211> 52
   <212> PRT
   <213> unknown
<220>
   <223> ColicinD_1-53
<400> 116
   Ser Asp Tyr Glu Gly Ser Gly Pro Thr Glu Gly Ile Asp Tyr Gly His
   1               5                   10                  15
   Ser Met Val Val Trp Pro Ser Thr Gly Leu Ile Ser Gly Gly Asp Val
               20                  25                  30
   Lys Pro Gly Gly Ser Ser Gly Ile Ala Pro Ser Met Pro Pro Gly Trp
           35                  40                  45
   Gly Asp Tyr Ser
       50
<210> 117
   <211> 34
   <212> PRT
   <213> Limulus polyphemus
<400> 117
   Gly Phe Lys Leu Lys Gly Met Ala Arg Ile Ser Cys Leu Pro Asn Gly
   1               5                   10                  15
   Gln Trp Ser Asn Phe Pro Pro Lys Cys Ile Arg Glu Cys Ala Met Val
               20                  25                  30
   Ser Ser
<210> 118
   <211> 18
   <212> PRT
   <213> artificial
<220>
   <223> synthetic sequence
<400> 118
   Gly Phe Phe Ile Pro Ala Val Ile Leu Pro Ser Ile Ala Phe Leu Ile
   1               5                   10                  15
   Val Pro
<210> 119
   <211> 5
   <212> PRT
   <213> artificial
<220>
   <223> synthetic sequence
<400> 119
   Phe Phe Val Ala Pro
   1 5
<210> 120
   <211> 13
   <212> PRT
   <213> unknown
<220>
   <223> alpha4-helix of T4 lysozyme
<400> 120
   Pro Asn Arg Ala Lys Arg Val Ile Thr Thr Phe Arg Thr
   1 5 10
<210> 121
   <211> 27
   <212> PRT
   <213> artificial
<220>
   <223> synthetic sequence
<400> 121
   Lys Arg Trp Val Lys Arg Val Lys Arg Val Lys Arg Trp Val Lys Arg
   1 5 10 15
   Val Val Arg Val Val Lys Arg Trp Val Lys Arg
   20 25
<210> 122
   <211> 25
   <212> PRT
   <213> Artificial Sequence
<220>
   <223> synthetic sequence; MW2
<400> 122
   Gly Lys Pro Gly Trp Leu Ile Lys Val Ala Leu Lys Phe Lys Lys Leu
   1 5 10 15
   Ile Arg Arg Pro Leu Lys Arg Leu Ala
   20 25
<210> 123
   <211> 5
   <212> PRT
   <213> Artificial
<220>
   <223> Linker sequence
<400> 123
   Gly Gly Gly Gly Ser
   1               5
<210> 124
   <211> 6
   <212> PRT
   <213> artificial sequence
<220>
   <223> His-Tag (6x)
<400> 124
   His His His His His His
   1               5
<210> 125
   <211> 166
   <212> PRT
   <213> Artificial sequence
<220>
   <223> Fusion MSI-78-GGGGS-S394 w/o methionine
<400> 125
   Gly Ile Gly Lys Phe Leu Lys Lys Ala Lys Lys Phe Gly Lys Ala Phe
   1               5                   10                  15
   Val Lys Ile Leu Lys Lys Gly Gly Gly Gly Ser Gly Ser Met Ser Phe
               20                  25                  30
   Lys Phe Gly Lys Asn Ser Glu Lys Gln Leu Ala Thr Val Lys Pro Glu
           35                  40                  45
   Leu Gln Lys Val Ala Arg Arg Ala Leu Glu Leu Ser Pro Tyr Asp Phe
       50                  55                  60
   Thr Ile Val Gln Gly Ile Arg Thr Val Ala Gln Ser Ala Gln Asn Ile
   65 70 75 80
   Ala Asn Gly Thr Ser Phe Leu Lys Asp Pro Ser Lys Ser Lys His Val
   85 90 95
   Thr Gly Asp Ala Ile Asp Phe Ala Pro Tyr Ile Asn Gly Lys Ile Asp
   100 105 110
   Trp Lys Asp Leu Glu Ala Phe Trp Ala Val Lys Lys Ala Phe Glu Gln
   115 120 125
   Ala Gly Lys Glu Leu Gly Ile Lys Leu Arg Phe Gly Ala Asp Trp Asn
   130 135 140
   Ser Ser Gly Asp Tyr His Asp Glu Ile Asp Arg Gly Thr Tyr Asp Gly
   145 150 155 160
   Gly His Val Glu Leu Val
   165
<210> 126
   <211> 167
   <212> PRT
   <213> Artificial sequence
<220>
   <223> Fusion MSI-78-GGGGS-S394
<400> 126
   Met Gly Ile Gly Lys Phe Leu Lys Lys Ala Lys Lys Phe Gly Lys Ala
   1 5 10 15
   Phe Val Lys Ile Leu Lys Lys Gly Gly Gly Gly Ser Gly Ser Met Ser
   20 25 30
   Phe Lys Phe Gly Lys Asn Ser Glu Lys Gln Leu Ala Thr Val Lys Pro
   35 40 45
   Glu Leu Gln Lys Val Ala Arg Arg Ala Leu Glu Leu Ser Pro Tyr Asp
   50 55 60
   Phe Thr Ile Val Gln Gly Ile Arg Thr Val Ala Gln Ser Ala Gln Asn
   65 70 75 80
   Ile Ala Asn Gly Thr Ser Phe Leu Lys Asp Pro Ser Lys Ser Lys His
   85 90 95
   Val Thr Gly Asp Ala Ile Asp Phe Ala Pro Tyr Ile Asn Gly Lys Ile
   100 105 110
   Asp Trp Lys Asp Leu Glu Ala Phe Trp Ala Val Lys Lys Ala Phe Glu
   115 120 125
   Gln Ala Gly Lys Glu Leu Gly Ile Lys Leu Arg Phe Gly Ala Asp Trp
   130 135 140
   Asn Ser Ser Gly Asp Tyr His Asp Glu Ile Asp Arg Gly Thr Tyr Asp
   145 150 155 160
   Gly Gly His Val Glu Leu Val
   165
<210> 127
   <211> 175
   <212> **PRT**
   <213> Artificial sequence
<220>
   <223> Fusion MSI-78-GGGGS-S394-His6
<400> 127
   Met Gly Ile Gly Lys Phe Leu Lys Lys Ala Lys Lys Phe Gly Lys Ala
   1 5 10 15
   Phe Val Lys Ile Leu Lys Lys Gly Gly Gly Gly Ser Gly Ser Met Ser
   20 25 30
   Phe Lys Phe Gly Lys Asn Ser Glu Lys Gln Leu Ala Thr Val Lys Pro
   35 40 45
   Glu Leu Gln Lys Val Ala Arg Arg Ala Leu Glu Leu Ser Pro Tyr Asp
   50 55 60
   Phe Thr Ile Val Gln Gly Ile Arg Thr Val Ala Gln Ser Ala Gln Asn
   65 70 75 80
   Ile Ala Asn Gly Thr Ser Phe Leu Lys Asp Pro Ser Lys Ser Lys His
   85 90 95
   Val Thr Gly Asp Ala Ile Asp Phe Ala Pro Tyr Ile Asn Gly Lys Ile
   100 105 110
   Asp Trp Lys Asp Leu Glu Ala Phe Trp Ala Val Lys Lys Ala Phe Glu
   115 120 125
   Gln Ala Gly Lys Glu Leu Gly Ile Lys Leu Arg Phe Gly Ala Asp Trp
       130                 135                 140
   Asn Ser Ser Gly Asp Tyr His Asp Glu Ile Asp Arg Gly Thr Tyr Asp
   145                 150                 155                 160
   Gly Gly His Val Glu Leu Val Leu Glu His His His His His His
                   165                 170                 175
<210> 128
   <211> 291
   <212> PRT
   <213> Artificial Sequence
<220>
   <223> Fusion Cathelicidin-BF-KZ144(C14S, C23S, C50S) w/o methionine
<400> 128
   Lys Phe Phe Arg Lys Leu Lys Lys Ser Val Lys Lys Arg Ala Lys Glu
   1               5                   10                  15
   Phe Phe Lys Lys Pro Arg Val Ile Gly Val Ser Ile Pro Phe Gly Ser
               20                  25                  30
   Lys Val Leu Arg Lys Gly Asp Arg Gly Asp Glu Val Ser Gln Leu Gln
           35                  40                  45
   Thr Leu Leu Asn Leu Ser Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Ser Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Thr Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Ile Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Ala Leu
               180                 185                 190
   Arg Lys Asp Pro Arg Ile Ser Ala Leu Met Gly Ala Glu Leu Ile Lys
           195                 200                 205
   Glu Asn Met Asn Ile Leu Arg Pro Val Leu Lys Arg Glu Pro Thr Asp
       210                 215                 220
   Thr Asp Leu Tyr Leu Ala His Phe Phe Gly Pro Gly Ala Ala Arg Arg
   225                 230                 235                 240
   Phe Leu Thr Thr Gly Gln Asn Glu Leu Ala Ala Thr His Phe Pro Lys
                   245                 250                 255
   Glu Ala Gln Ala Asn Pro Ser Ile Phe Tyr Asn Lys Asp Gly Ser Pro
               260                 265                 270
   Lys Thr Ile Gln Glu Val Tyr Asn Leu Met Asp Gly Lys Val Ala Ala
           275                 280                 285
   His Arg Lys
       290
<210> 129
   <211> 292
   <212> PRT
   <213> Artificial Sequence
<220>
   <223> Fusion Cathelicidin-BF-KZ144(C14S, C23S, C50S)
<400> 129
   Met Lys Phe Phe Arg Lys Leu Lys Lys Ser Val Lys Lys Arg Ala Lys
   1               5                   10                  15
   Glu Phe Phe Lys Lys Pro Arg Val Ile Gly Val Ser Ile Pro Phe Gly
               20                  25                  30
   Ser Lys Val Leu Arg Lys Gly Asp Arg Gly Asp Glu Val Ser Gln Leu
           35                  40                  45
   Gln Thr Leu Leu Asn Leu Ser Gly Tyr Asp Val Gly Lys Pro Asp Gly
       50                  55                  60
   Ile Phe Gly Asn Asn Thr Phe Asn Gln Val Val Lys Phe Gln Lys Asp
   65                  70                  75                  80
   Asn Ser Leu Asp Ser Asp Gly Ile Val Gly Lys Asn Thr Trp Ala Glu
                   85                  90                  95
   Leu Phe Ser Lys Tyr Ser Pro Pro Ile Pro Tyr Lys Thr Ile Pro Met
               100                 105                 110
   Pro Thr Ala Asn Lys Ser Arg Ala Ala Ala Thr Pro Val Met Asn Ala
           115                 120                 125
   Val Glu Asn Ala Thr Gly Val Arg Ser Gln Leu Leu Leu Thr Phe Ala
       130                 135                 140
   Ser Ile Glu Ser Ala Phe Asp Tyr Glu Ile Lys Ala Lys Thr Ser Ser
   145                 150                 155                 160
   Ala Thr Gly Trp Phe Gln Phe Leu Thr Gly Thr Trp Lys Thr Met Ile
                   165                 170                 175
   Glu Asn Tyr Gly Met Lys Tyr Gly Val Leu Thr Asp Pro Thr Gly Ala
               180                 185                 190
   Leu Arg Lys Asp Pro Arg Ile Ser Ala Leu Met Gly Ala Glu Leu Ile
           195                 200                 205
   Lys Glu Asn Met Asn Ile Leu Arg Pro Val Leu Lys Arg Glu Pro Thr
       210                 215                 220
   Asp Thr Asp Leu Tyr Leu Ala His Phe Phe Gly Pro Gly Ala Ala Arg
   225                 230                 235                 240
   Arg Phe Leu Thr Thr Gly Gln Asn Glu Leu Ala Ala Thr His Phe Pro
                   245                 250                 255
   Lys Glu Ala Gln Ala Asn Pro Ser Ile Phe Tyr Asn Lys Asp Gly Ser
               260                 265                 270
   Pro Lys Thr Ile Gln Glu Val Tyr Asn Leu Met Asp Gly Lys Val Ala
           275                 280                 285
   Ala His Arg Lys
       290
<210> 130
   <211> 300
   <212> PRT
   <213> Artificial Sequence
<220>
   <223> Fusion Cathelicidin-BF-KZ144(C14S, C23S, C50S)-His6
<400> 130
   Met Lys Phe Phe Arg Lys Leu Lys Lys Ser Val Lys Lys Arg Ala Lys
   1               5                   10                  15
   Glu Phe Phe Lys Lys Pro Arg Val Ile Gly Val Ser Ile Pro Phe Gly
               20                  25                  30
   Ser Lys Val Leu Arg Lys Gly Asp Arg Gly Asp Glu Val Ser Gln Leu
           35                  40                  45
   Gln Thr Leu Leu Asn Leu Ser Gly Tyr Asp Val Gly Lys Pro Asp Gly
       50                  55                  60
   Ile Phe Gly Asn Asn Thr Phe Asn Gln Val Val Lys Phe Gln Lys Asp
   65                  70                  75                  80
   Asn Ser Leu Asp Ser Asp Gly Ile Val Gly Lys Asn Thr Trp Ala Glu
                   85                  90                  95
   Leu Phe Ser Lys Tyr Ser Pro Pro Ile Pro Tyr Lys Thr Ile Pro Met
               100                 105                 110
   Pro Thr Ala Asn Lys Ser Arg Ala Ala Ala Thr Pro Val Met Asn Ala
           115                 120                 125
   Val Glu Asn Ala Thr Gly Val Arg Ser Gln Leu Leu Leu Thr Phe Ala
       130                 135                 140
   Ser Ile Glu Ser Ala Phe Asp Tyr Glu Ile Lys Ala Lys Thr Ser Ser
   145                 150                 155                 160
   Ala Thr Gly Trp Phe Gln Phe Leu Thr Gly Thr Trp Lys Thr Met Ile
                   165                 170                 175
   Glu Asn Tyr Gly Met Lys Tyr Gly Val Leu Thr Asp Pro Thr Gly Ala
               180                 185                 190
   Leu Arg Lys Asp Pro Arg Ile Ser Ala Leu Met Gly Ala Glu Leu Ile
           195                 200                 205
   Lys Glu Asn Met Asn Ile Leu Arg Pro Val Leu Lys Arg Glu Pro Thr
       210                 215                 220
   Asp Thr Asp Leu Tyr Leu Ala His Phe Phe Gly Pro Gly Ala Ala Arg
   225                 230                 235                 240
   Arg Phe Leu Thr Thr Gly Gln Asn Glu Leu Ala Ala Thr His Phe Pro
   245 250 255
   Lys Glu Ala Gln Ala Asn Pro Ser Ile Phe Tyr Asn Lys Asp Gly Ser
   260 265 270
   Pro Lys Thr Ile Gln Glu Val Tyr Asn Leu Met Asp Gly Lys Val Ala
   275 280 285
   Ala His Arg Lys Leu Glu His His His His His His
   290 295 300
<210> 131
   <211> 169
   <212> PRT
   <213> Artificial Sequence
<220>
   <223> Fusion Cathelicidin-BF-S394 w/o methionine
<400> 131
   Lys Phe Phe Arg Lys Leu Lys Lys Ser Val Lys Lys Arg Ala Lys Glu
   1 5 10 15
   Phe Phe Lys Lys Pro Arg Val Ile Gly Val Ser Ile Pro Phe Gly Ser
   20 25 30
   Met Ser Phe Lys Phe Gly Lys Asn Ser Glu Lys Gln Leu Ala Thr Val
   35 40 45
   Lys Pro Glu Leu Gln Lys Val Ala Arg Arg Ala Leu Glu Leu Ser Pro
   50 55 60
   Tyr Asp Phe Thr Ile Val Gln Gly Ile Arg Thr Val Ala Gln Ser Ala
   65 70 75 80
   Gln Asn Ile Ala Asn Gly Thr Ser Phe Leu Lys Asp Pro Ser Lys Ser
   85 90 95
   Lys His Val Thr Gly Asp Ala Ile Asp Phe Ala Pro Tyr Ile Asn Gly
   100 105 110
   Lys Ile Asp Trp Lys Asp Leu Glu Ala Phe Trp Ala Val Lys Lys Ala
   115 120 125
   Phe Glu Gln Ala Gly Lys Glu Leu Gly Ile Lys Leu Arg Phe Gly Ala
   130 135 140
   Asp Trp Asn Ser Ser Gly Asp Tyr His Asp Glu Ile Asp Arg Gly Thr
   145 150 155 160
   Tyr Asp Gly Gly His Val Glu Leu Val
   165
<210> 132
   <211> 170
   <212> PRT
   <213> Artificial Sequence
<220>
   <223> Fusion Cathelicidin-BF-S394
<400> 132
   Met Lys Phe Phe Arg Lys Leu Lys Lys Ser Val Lys Lys Arg Ala Lys
   1 5 10 15
   Glu Phe Phe Lys Lys Pro Arg Val Ile Gly Val Ser Ile Pro Phe Gly
   20 25 30
   Ser Met Ser Phe Lys Phe Gly Lys Asn Ser Glu Lys Gln Leu Ala Thr
   35 40 45
   Val Lys Pro Glu Leu Gln Lys Val Ala Arg Arg Ala Leu Glu Leu Ser
   50 55 60
   Pro Tyr Asp Phe Thr Ile Val Gln Gly Ile Arg Thr Val Ala Gln Ser
   65 70 75 80
   Ala Gln Asn Ile Ala Asn Gly Thr Ser Phe Leu Lys Asp Pro Ser Lys
   85 90 95
   Ser Lys His Val Thr Gly Asp Ala Ile Asp Phe Ala Pro Tyr Ile Asn
   100 105 110
   Gly Lys Ile Asp Trp Lys Asp Leu Glu Ala Phe Trp Ala Val Lys Lys
   115 120 125
   Ala Phe Glu Gln Ala Gly Lys Glu Leu Gly Ile Lys Leu Arg Phe Gly
   130 135 140
   Ala Asp Trp Asn Ser Ser Gly Asp Tyr His Asp Glu Ile Asp Arg Gly
   145 150 155 160
   Thr Tyr Asp Gly Gly His Val Glu Leu Val
   165 170
<210> 133
   <211> 178
   <212> PRT
   <213> Artificial Sequence
<220>
   <223> Fusion Cathelicidin-BF-S394-His6
<400> 133
   Met Lys Phe Phe Arg Lys Leu Lys Lys Ser Val Lys Lys Arg Ala Lys
   1               5                   10                  15
   Glu Phe Phe Lys Lys Pro Arg Val Ile Gly Val Ser Ile Pro Phe Gly
               20                  25                  30
   Ser Met Ser Phe Lys Phe Gly Lys Asn Ser Glu Lys Gln Leu Ala Thr
           35                  40                  45
   Val Lys Pro Glu Leu Gln Lys Val Ala Arg Arg Ala Leu Glu Leu Ser
       50                  55                  60
   Pro Tyr Asp Phe Thr Ile Val Gln Gly Ile Arg Thr Val Ala Gln Ser
   65                  70                  75                  80
   Ala Gln Asn Ile Ala Asn Gly Thr Ser Phe Leu Lys Asp Pro Ser Lys
                   85                  90                  95
   Ser Lys His Val Thr Gly Asp Ala Ile Asp Phe Ala Pro Tyr Ile Asn
               100                 105                 110
   Gly Lys Ile Asp Trp Lys Asp Leu Glu Ala Phe Trp Ala Val Lys Lys
           115                 120                 125
   Ala Phe Glu Gln Ala Gly Lys Glu Leu Gly Ile Lys Leu Arg Phe Gly
       130                 135                 140
   Ala Asp Trp Asn Ser Ser Gly Asp Tyr His Asp Glu Ile Asp Arg Gly
   145                 150                 155                 160
   Thr Tyr Asp Gly Gly His Val Glu Leu Val Leu Glu His His His His
                   165                 170                 175
   His His
<210> 134
   <211> 288
   <212> PRT
   <213> Artificial Sequence
<220>
   <223> Fusion MSI-78--KZ144(C14S, C23S, C50S) w/o methionine
<400> 134
   Gly Ile Gly Lys Phe Leu Lys Lys Ala Lys Lys Phe Gly Lys Ala Phe
   1               5                   10                  15
   Val Lys Ile Leu Lys Lys Gly Gly Gly Gly Ser Gly Ser Lys Val Leu
               20                  25                  30
   Arg Lys Gly Asp Arg Gly Asp Glu Val Ser Gln Leu Gln Thr Leu Leu
           35                  40                  45
   Asn Leu Ser Gly Tyr Asp Val Gly Lys Pro Asp Gly Ile Phe Gly Asn
       50                  55                  60
   Asn Thr Phe Asn Gln Val Val Lys Phe Gln Lys Asp Asn Ser Leu Asp
   65                  70                  75                  80
   Ser Asp Gly Ile Val Gly Lys Asn Thr Trp Ala Glu Leu Phe Ser Lys
                   85                  90                  95
   Tyr Ser Pro Pro Ile Pro Tyr Lys Thr Ile Pro Met Pro Thr Ala Asn
               100                 105                 110
   Lys Ser Arg Ala Ala Ala Thr Pro Val Met Asn Ala Val Glu Asn Ala
           115                 120                 125
   Thr Gly Val Arg Ser Gln Leu Leu Leu Thr Phe Ala Ser Ile Glu Ser
       130                 135                 140
   Ala Phe Asp Tyr Glu Ile Lys Ala Lys Thr Ser Ser Ala Thr Gly Trp
   145                 150                 155                 160
   Phe Gln Phe Leu Thr Gly Thr Trp Lys Thr Met Ile Glu Asn Tyr Gly
                   165                 170                 175
   Met Lys Tyr Gly Val Leu Thr Asp Pro Thr Gly Ala Leu Arg Lys Asp
               180                 185                 190
   Pro Arg Ile Ser Ala Leu Met Gly Ala Glu Leu Ile Lys Glu Asn Met
           195                 200                 205
   Asn Ile Leu Arg Pro Val Leu Lys Arg Glu Pro Thr Asp Thr Asp Leu
       210                 215                 220
   Tyr Leu Ala His Phe Phe Gly Pro Gly Ala Ala Arg Arg Phe Leu Thr
   225                 230                 235                 240
   Thr Gly Gln Asn Glu Leu Ala Ala Thr His Phe Pro Lys Glu Ala Gln
                   245                 250                 255
   Ala Asn Pro Ser Ile Phe Tyr Asn Lys Asp Gly Ser Pro Lys Thr Ile
               260                 265                 270
   Gln Glu Val Tyr Asn Leu Met Asp Gly Lys Val Ala Ala His Arg Lys
           275                 280                 285
<210> 135
   <211> 289
   <212> PRT
   <213> Artificial Sequence
<220>
   <223> Fusion MSI-78--KZ144(C14S, C23S, C50S)
<400> 135
   Met Gly Ile Gly Lys Phe Leu Lys Lys Ala Lys Lys Phe Gly Lys Ala
   1               5                   10                  15
   Phe Val Lys Ile Leu Lys Lys Gly Gly Gly Gly Ser Gly Ser Lys Val
               20                  25                  30
   Leu Arg Lys Gly Asp Arg Gly Asp Glu Val Ser Gln Leu Gln Thr Leu
           35                  40                  45
   Leu Asn Leu Ser Gly Tyr Asp Val Gly Lys Pro Asp Gly Ile Phe Gly
       50                  55                  60
   Asn Asn Thr Phe Asn Gln Val Val Lys Phe Gln Lys Asp Asn Ser Leu
   65                  70                  75                  80
   Asp Ser Asp Gly Ile Val Gly Lys Asn Thr Trp Ala Glu Leu Phe Ser
                   85                  90                  95
   Lys Tyr Ser Pro Pro Ile Pro Tyr Lys Thr Ile Pro Met Pro Thr Ala
               100                 105                 110
   Asn Lys Ser Arg Ala Ala Ala Thr Pro Val Met Asn Ala Val Glu Asn
           115                 120                 125
   Ala Thr Gly Val Arg Ser Gln Leu Leu Leu Thr Phe Ala Ser Ile Glu
       130                 135                 140
   Ser Ala Phe Asp Tyr Glu Ile Lys Ala Lys Thr Ser Ser Ala Thr Gly
   145                 150                 155                 160
   Trp Phe Gln Phe Leu Thr Gly Thr Trp Lys Thr Met Ile Glu Asn Tyr
   165 170 175
   Gly Met Lys Tyr Gly Val Leu Thr Asp Pro Thr Gly Ala Leu Arg Lys
   180 185 190
   Asp Pro Arg Ile Ser Ala Leu Met Gly Ala Glu Leu Ile Lys Glu Asn
   195 200 205
   Met Asn Ile Leu Arg Pro Val Leu Lys Arg Glu Pro Thr Asp Thr Asp
   210 215 220
   Leu Tyr Leu Ala His Phe Phe Gly Pro Gly Ala Ala Arg Arg Phe Leu
   225 230 235 240
   Thr Thr Gly Gln Asn Glu Leu Ala Ala Thr His Phe Pro Lys Glu Ala
   245 250 255
   Gln Ala Asn Pro Ser Ile Phe Tyr Asn Lys Asp Gly Ser Pro Lys Thr
   260 265 270
   Ile Gln Glu Val Tyr Asn Leu Met Asp Gly Lys Val Ala Ala His Arg
   275 280 285
   Lys
<210> 136
   <211> 297
   <212> PRT
   <213> Artificial Sequence
<220>
   <223> Fusion MSI-78-KZ144(C14S, C23S, C50S)-His6
<400> 136
   Met Gly Ile Gly Lys Phe Leu Lys Lys Ala Lys Lys Phe Gly Lys Ala
   1 5 10 15
   Phe Val Lys Ile Leu Lys Lys Gly Gly Gly Gly Ser Gly Ser Lys Val
   20 25 30
   Leu Arg Lys Gly Asp Arg Gly Asp Glu Val Ser Gln Leu Gln Thr Leu
   35 40 45
   Leu Asn Leu Ser Gly Tyr Asp Val Gly Lys Pro Asp Gly Ile Phe Gly
   50 55 60
   Asn Asn Thr Phe Asn Gln Val Val Lys Phe Gln Lys Asp Asn Ser Leu
   65                  70                  75                  80
   Asp Ser Asp Gly Ile Val Gly Lys Asn Thr Trp Ala Glu Leu Phe Ser
                   85                  90                  95
   Lys Tyr Ser Pro Pro Ile Pro Tyr Lys Thr Ile Pro Met Pro Thr Ala
               100                 105                 110
   Asn Lys Ser Arg Ala Ala Ala Thr Pro Val Met Asn Ala Val Glu Asn
           115                 120                 125
   Ala Thr Gly Val Arg Ser Gln Leu Leu Leu Thr Phe Ala Ser Ile Glu
       130                 135                 140
   Ser Ala Phe Asp Tyr Glu Ile Lys Ala Lys Thr Ser Ser Ala Thr Gly
   145                 150                 155                 160
   Trp Phe Gln Phe Leu Thr Gly Thr Trp Lys Thr Met Ile Glu Asn Tyr
                   165                 170                 175
   Gly Met Lys Tyr Gly Val Leu Thr Asp Pro Thr Gly Ala Leu Arg Lys
               180                 185                 190
   Asp Pro Arg Ile Ser Ala Leu Met Gly Ala Glu Leu Ile Lys Glu Asn
           195                 200                 205
   Met Asn Ile Leu Arg Pro Val Leu Lys Arg Glu Pro Thr Asp Thr Asp
       210                 215                 220
   Leu Tyr Leu Ala His Phe Phe Gly Pro Gly Ala Ala Arg Arg Phe Leu
   225                 230                 235                 240
   Thr Thr Gly Gln Asn Glu Leu Ala Ala Thr His Phe Pro Lys Glu Ala
                   245                 250                 255
   Gln Ala Asn Pro Ser Ile Phe Tyr Asn Lys Asp Gly Ser Pro Lys Thr
               260                 265                 270
   Ile Gln Glu Val Tyr Asn Leu Met Asp Gly Lys Val Ala Ala His Arg
           275                 280                 285
   Lys Leu Glu His His His His His His
       290                 295
<210> 137
   <211> 359
   <212> PRT
   <213> Artificial Sequence
<220>
   <223> Fusion Cathelicidin-BF-OBPgp279 w/o methionine
<400> 137
   Lys Phe Phe Arg Lys Leu Lys Lys Ser Val Lys Lys Arg Ala Lys Glu
   1               5                   10                  15
   Phe Phe Lys Lys Pro Arg Val Ile Gly Val Ser Ile Pro Phe Gly Ser
               20                  25                  30
   Lys Asn Ser Glu Lys Asn Ala Ser Ile Ile Met Ser Ile Gln Arg Thr
           35                  40                  45
   Leu Ala Ser Leu Ser Leu Tyr Gly Gly Arg Ile Asp Gly Leu Phe Gly
       50                  55                  60
   Glu Lys Cys Arg Gly Ala Ile Ile Leu Met Leu Asn Lys Val Tyr Pro
   65                  70                  75                  80
   Asn Phe Ser Thr Asn Lys Leu Pro Ser Asn Thr Tyr Glu Ala Glu Ser
                   85                  90                  95
   Val Phe Thr Phe Leu Gln Thr Ala Leu Ala Gly Val Gly Leu Tyr Thr
               100                 105                 110
   Ile Thr Ile Asp Gly Lys Trp Gly Gly Thr Ser Gln Gly Ala Ile Asp
           115                 120                 125
   Ala Leu Val Lys Ser Tyr Arg Gln Ile Thr Glu Ala Glu Arg Ala Gly
       130                 135                 140
   Ser Thr Leu Pro Leu Gly Leu Ala Thr Val Met Ser Lys His Met Ser
   145                 150                 155                 160
   Ile Glu Gln Leu Arg Ala Met Leu Pro Thr Asp Arg Gln Gly Tyr Ala
                   165                 170                 175
   Glu Val Tyr Ile Asp Pro Leu Asn Glu Thr Met Asp Ile Phe Glu Ile
               180                 185                 190
   Asn Thr Pro Leu Arg Ile Ala His Phe Met Ala Gln Ile Leu His Glu
           195                 200                 205
   Thr Ala Cys Phe Lys Tyr Thr Glu Glu Leu Ala Ser Gly Lys Ala Tyr
       210                 215                 220
   Glu Gly Arg Ala Asp Leu Gly Asn Thr Arg Pro Gly Asp Gly Pro Leu
   225                 230                 235                 240
   Phe Lys Gly Arg Gly Leu Leu Gln Ile Thr Gly Arg Leu Asn Tyr Val
                   245                 250                 255
   Lys Cys Gln Val Tyr Leu Arg Glu Lys Leu Lys Asp Pro Thr Phe Asp
               260                 265                 270
   Ile Thr Ser Ser Val Thr Cys Ala Gln Gln Leu Ser Glu Ser Pro Leu
           275                 280                 285
   Leu Ala Ala Leu Ala Ser Gly Tyr Phe Trp Arg Phe Ile Lys Pro Lys
       290                 295                 300
   Leu Asn Glu Thr Ala Asp Lys Asp Asp Ile Tyr Trp Val Ser Val Tyr
   305                 310                 315                 320
   Val Asn Gly Tyr Ala Lys Gln Ala Asn Pro Tyr Tyr Pro Asn Arg Asp
                   325                 330                 335
   Lys Glu Pro Asn His Met Lys Glu Arg Val Gln Met Leu Ala Val Thr
               340                 345                 350
   Lys Lys Ala Leu Gly Ile Val
           355
<210> 138
   <211> 360
   <212> PRT
   <213> Artificial Sequence
<220>
   <223> Fusion Cathelicidin-BF-OBPgp279
<400> 138
   Met Lys Phe Phe Arg Lys Leu Lys Lys Ser Val Lys Lys Arg Ala Lys
   1               5                   10                  15
   Glu Phe Phe Lys Lys Pro Arg Val Ile Gly Val Ser Ile Pro Phe Gly
               20                  25                  30
   Ser Lys Asn Ser Glu Lys Asn Ala Ser Ile Ile Met Ser Ile Gln Arg
           35                  40                  45
   Thr Leu Ala Ser Leu Ser Leu Tyr Gly Gly Arg Ile Asp Gly Leu Phe
       50                  55                  60
   Gly Glu Lys Cys Arg Gly Ala Ile Ile Leu Met Leu Asn Lys Val Tyr
   65                  70                  75                  80
   Pro Asn Phe Ser Thr Asn Lys Leu Pro Ser Asn Thr Tyr Glu Ala Glu
                   85                  90                  95
   Ser Val Phe Thr Phe Leu Gln Thr Ala Leu Ala Gly Val Gly Leu Tyr
               100                 105                 110
   Thr Ile Thr Ile Asp Gly Lys Trp Gly Gly Thr Ser Gln Gly Ala Ile
           115                 120                 125
   Asp Ala Leu Val Lys Ser Tyr Arg Gln Ile Thr Glu Ala Glu Arg Ala
       130                 135                 140
   Gly Ser Thr Leu Pro Leu Gly Leu Ala Thr Val Met Ser Lys His Met
   145                 150                 155                 160
   Ser Ile Glu Gln Leu Arg Ala Met Leu Pro Thr Asp Arg Gln Gly Tyr
                   165                 170                 175
   Ala Glu Val Tyr Ile Asp Pro Leu Asn Glu Thr Met Asp Ile Phe Glu
               180                 185                 190
   Ile Asn Thr Pro Leu Arg Ile Ala His Phe Met Ala Gln Ile Leu His
           195                 200                 205
   Glu Thr Ala Cys Phe Lys Tyr Thr Glu Glu Leu Ala Ser Gly Lys Ala
       210                 215                 220
   Tyr Glu Gly Arg Ala Asp Leu Gly Asn Thr Arg Pro Gly Asp Gly Pro
   225                 230                 235                 240
   Leu Phe Lys Gly Arg Gly Leu Leu Gln Ile Thr Gly Arg Leu Asn Tyr
                   245                 250                 255
   Val Lys Cys Gln Val Tyr Leu Arg Glu Lys Leu Lys Asp Pro Thr Phe
               260                 265                 270
   Asp Ile Thr Ser Ser Val Thr Cys Ala Gln Gln Leu Ser Glu Ser Pro
           275                 280                 285
   Leu Leu Ala Ala Leu Ala Ser Gly Tyr Phe Trp Arg Phe Ile Lys Pro
       290                 295                 300
   Lys Leu Asn Glu Thr Ala Asp Lys Asp Asp Ile Tyr Trp Val Ser Val
   305                 310                 315                 320
   Tyr Val Asn Gly Tyr Ala Lys Gln Ala Asn Pro Tyr Tyr Pro Asn Arg
   325 330 335
   Asp Lys Glu Pro Asn His Met Lys Glu Arg Val Gln Met Leu Ala Val
   340 345 350
   Thr Lys Lys Ala Leu Gly Ile Val
   355 360
<210> 139
   <211> 368
   <212> PRT
   <213> Artificial Sequence
<220>
   <223> Fusion Cathelicidin-BF-OBPgp279-His6
<400> 139
   Met Lys Phe Phe Arg Lys Leu Lys Lys Ser Val Lys Lys Arg Ala Lys
   1               5                   10                  15
   Glu Phe Phe Lys Lys Pro Arg Val Ile Gly Val Ser Ile Pro Phe Gly
               20                  25                  30
   Ser Lys Asn Ser Glu Lys Asn Ala Ser Ile Ile Met Ser Ile Gln Arg
           35                  40                  45
   Thr Leu Ala Ser Leu Ser Leu Tyr Gly Gly Arg Ile Asp Gly Leu Phe
       50                  55                  60
   Gly Glu Lys Cys Arg Gly Ala Ile Ile Leu Met Leu Asn Lys Val Tyr
   65                  70                  75                  80
   Pro Asn Phe Ser Thr Asn Lys Leu Pro Ser Asn Thr Tyr Glu Ala Glu
                   85                  90                  95
   Ser Val Phe Thr Phe Leu Gln Thr Ala Leu Ala Gly Val Gly Leu Tyr
               100                 105                 110
   Thr Ile Thr Ile Asp Gly Lys Trp Gly Gly Thr Ser Gln Gly Ala Ile
           115                 120                 125
   Asp Ala Leu Val Lys Ser Tyr Arg Gln Ile Thr Glu Ala Glu Arg Ala
       130                 135                 140
   Gly Ser Thr Leu Pro Leu Gly Leu Ala Thr Val Met Ser Lys His Met
   145                 150                 155                 160
   Ser Ile Glu Gln Leu Arg Ala Met Leu Pro Thr Asp Arg Gln Gly Tyr
   165 170 175
   Ala Glu Val Tyr Ile Asp Pro Leu Asn Glu Thr Met Asp Ile Phe Glu
   180 185 190
   Ile Asn Thr Pro Leu Arg Ile Ala His Phe Met Ala Gln Ile Leu His
   195 200 205
   Glu Thr Ala Cys Phe Lys Tyr Thr Glu Glu Leu Ala Ser Gly Lys Ala
   210 215 220
   Tyr Glu Gly Arg Ala Asp Leu Gly Asn Thr Arg Pro Gly Asp Gly Pro
   225 230 235 240
   Leu Phe Lys Gly Arg Gly Leu Leu Gln Ile Thr Gly Arg Leu Asn Tyr
   245 250 255
   Val Lys Cys Gln Val Tyr Leu Arg Glu Lys Leu Lys Asp Pro Thr Phe
   260 265 270
   Asp Ile Thr Ser Ser Val Thr Cys Ala Gln Gln Leu Ser Glu Ser Pro
   275 280 285
   Leu Leu Ala Ala Leu Ala Ser Gly Tyr Phe Trp Arg Phe Ile Lys Pro
   290 295 300
   Lys Leu Asn Glu Thr Ala Asp Lys Asp Asp Ile Tyr Trp Val Ser Val
   305 310 315 320
   Tyr Val Asn Gly Tyr Ala Lys Gln Ala Asn Pro Tyr Tyr Pro Asn Arg
   325 330 335
   Asp Lys Glu Pro Asn His Met Lys Glu Arg Val Gln Met Leu Ala Val
   340 345 350
   Thr Lys Lys Ala Leu Gly Ile Val Leu Glu His His His His His His
   355 360 365
<210> 140
   <211> 268
   <212> PRT
   <213> Artificial Sequence
<220>
   <223> Fusion Cathelicidin-BF-PVP-SE1gp146 w/o methionine
<400> 140
   Lys Phe Phe Arg Lys Leu Lys Lys Ser Val Lys Lys Arg Ala Lys Glu
   1               5                   10                  15
   Phe Phe Lys Lys Pro Arg Val Ile Gly Val Ser Ile Pro Phe Gly Ser
               20                  25                  30
   Met Asn Ala Ala Ile Ala Glu Ile Gln Arg Met Leu Ile Glu Gly Gly
           35                  40                  45
   Phe Ser Val Gly Lys Ser Gly Ala Asp Gly Leu Tyr Gly Pro Ala Thr
       50                  55                  60
   Lys Ala Ala Leu Gln Lys Cys Ile Ala Gln Ala Thr Ser Gly Asn Asn
   65                  70                  75                  80
   Lys Gly Gly Thr Leu Lys Leu Thr Gln Ala Gln Leu Asp Lys Ile Phe
                   85                  90                  95
   Pro Val Gly Ala Ser Ser Gly Arg Asn Ala Lys Phe Leu Lys Pro Leu
               100                 105                 110
   Asn Asp Leu Phe Glu Lys Thr Glu Ile Asn Thr Val Asn Arg Val Ala
           115                 120                 125
   Gly Phe Leu Ser Gln Ile Gly Val Glu Ser Ala Glu Phe Arg Tyr Val
       130                 135                 140
   Arg Glu Leu Gly Asn Asp Ala Tyr Phe Asp Lys Tyr Asp Thr Gly Pro
   145                 150                 155                 160
   Ile Ala Glu Arg Leu Gly Asn Thr Pro Gln Lys Asp Gly Asp Gly Ala
                   165                 170                 175
   Lys Tyr Lys Gly Arg Gly Leu Ile Gln Val Thr Gly Leu Ala Asn Tyr
               180                 185                 190
   Lys Ala Cys Gly Lys Ala Leu Gly Leu Asp Leu Val Asn His Pro Glu
           195                 200                 205
   Leu Leu Glu Gln Pro Glu Tyr Ala Val Ala Ser Ala Gly Trp Tyr Trp
       210                 215                 220
   Asp Thr Arg Asn Ile Asn Ala Ala Cys Asp Ala Asp Asp Ile Val Lys
   225                 230                 235                 240
   Ile Thr Lys Leu Val Asn Gly Gly Thr Asn His Leu Ala Glu Arg Thr
                   245                 250                 255
   Ala Tyr Tyr Lys Lys Ala Lys Ser Val Leu Thr Ser
   260 265
<210> 141
   <211> 269
   <212> PRT
   <213> Artificial Sequence
<220>
   <223> Fusion Cathelicidin-BF-PVP-SE1gp146
<400> 141
   Met Lys Phe Phe Arg Lys Leu Lys Lys Ser Val Lys Lys Arg Ala Lys
   1               5                   10                  15
   Glu Phe Phe Lys Lys Pro Arg Val Ile Gly Val Ser Ile Pro Phe Gly
               20                  25                  30
   Ser Met Asn Ala Ala Ile Ala Glu Ile Gln Arg Met Leu Ile Glu Gly
           35                  40                  45
   Gly Phe Ser Val Gly Lys Ser Gly Ala Asp Gly Leu Tyr Gly Pro Ala
       50                  55                  60
   Thr Lys Ala Ala Leu Gln Lys Cys Ile Ala Gln Ala Thr Ser Gly Asn
   65                  70                  75                  80
   Asn Lys Gly Gly Thr Leu Lys Leu Thr Gln Ala Gln Leu Asp Lys Ile
                   85                  90                  95
   Phe Pro Val Gly Ala Ser Ser Gly Arg Asn Ala Lys Phe Leu Lys Pro
               100                 105                 110
   Leu Asn Asp Leu Phe Glu Lys Thr Glu Ile Asn Thr Val Asn Arg Val
           115                 120                 125
   Ala Gly Phe Leu Ser Gln Ile Gly Val Glu Ser Ala Glu Phe Arg Tyr
       130                 135                 140
   Val Arg Glu Leu Gly Asn Asp Ala Tyr Phe Asp Lys Tyr Asp Thr Gly
   145                 150                 155                 160
   Pro Ile Ala Glu Arg Leu Gly Asn Thr Pro Gln Lys Asp Gly Asp Gly
                   165                 170                 175
   Ala Lys Tyr Lys Gly Arg Gly Leu Ile Gln Val Thr Gly Leu Ala Asn
               180                 185                 190
   Tyr Lys Ala Cys Gly Lys Ala Leu Gly Leu Asp Leu Val Asn His Pro
           195                 200                 205
   Glu Leu Leu Glu Gln Pro Glu Tyr Ala Val Ala Ser Ala Gly Trp Tyr
   210 215 220
   Trp Asp Thr Arg Asn Ile Asn Ala Ala Cys Asp Ala Asp Asp Ile Val
   225 230 235 240
   Lys Ile Thr Lys Leu Val Asn Gly Gly Thr Asn His Leu Ala Glu Arg
   245 250 255
   Thr Ala Tyr Tyr Lys Lys Ala Lys Ser Val Leu Thr Ser
   260 265
<210> 142
   <211> 277
   <212> PRT
   <213> Artificial Sequence
<220>
   <223> Fusion Cathelicidin-BF-PVP-SE1gp146-His6
<400> 142
   Met Lys Phe Phe Arg Lys Leu Lys Lys Ser Val Lys Lys Arg Ala Lys
   1 5 10 15
   Glu Phe Phe Lys Lys Pro Arg Val Ile Gly Val Ser Ile Pro Phe Gly
   20 25 30
   Ser Met Asn Ala Ala Ile Ala Glu Ile Gln Arg Met Leu Ile Glu Gly
   35 40 45
   Gly Phe Ser Val Gly Lys Ser Gly Ala Asp Gly Leu Tyr Gly Pro Ala
   50 55 60
   Thr Lys Ala Ala Leu Gln Lys Cys Ile Ala Gln Ala Thr Ser Gly Asn
   65 70 75 80
   Asn Lys Gly Gly Thr Leu Lys Leu Thr Gln Ala Gln Leu Asp Lys Ile
   85 90 95
   Phe Pro Val Gly Ala Ser Ser Gly Arg Asn Ala Lys Phe Leu Lys Pro
   100 105 110
   Leu Asn Asp Leu Phe Glu Lys Thr Glu Ile Asn Thr Val Asn Arg Val
   115 120 125
   Ala Gly Phe Leu Ser Gln Ile Gly Val Glu Ser Ala Glu Phe Arg Tyr
   130 135 140
   Val Arg Glu Leu Gly Asn Asp Ala Tyr Phe Asp Lys Tyr Asp Thr Gly
   145                 150                 155                 160
   Pro Ile Ala Glu Arg Leu Gly Asn Thr Pro Gln Lys Asp Gly Asp Gly
                   165                 170                 175
   Ala Lys Tyr Lys Gly Arg Gly Leu Ile Gln Val Thr Gly Leu Ala Asn
               180                 185                 190
   Tyr Lys Ala Cys Gly Lys Ala Leu Gly Leu Asp Leu Val Asn His Pro
           195                 200                 205
   Glu Leu Leu Glu Gln Pro Glu Tyr Ala Val Ala Ser Ala Gly Trp Tyr
       210                 215                 220
   Trp Asp Thr Arg Asn Ile Asn Ala Ala Cys Asp Ala Asp Asp Ile Val
   225                 230                 235                 240
   Lys Ile Thr Lys Leu Val Asn Gly Gly Thr Asn His Leu Ala Glu Arg
                   245                 250                 255
   Thr Ala Tyr Tyr Lys Lys Ala Lys Ser Val Leu Thr Ser Leu Glu His
               260                 265                 270
   His His His His His
           275
<210> 143
   <211> 164
   <212> PRT
   <213> Artificial Sequence
<220>
   <223> Fusion Lycotoxin1-S394 w/o methionine
<400> 143
   Ile Trp Leu Thr Ala Leu Lys Phe Leu Gly Lys His Ala Ala Lys Lys
   1               5                   10                  15
   Leu Ala Lys Gln Gln Leu Ser Lys Leu Gly Ser Met Ser Phe Lys Phe
               20                  25                  30
   Gly Lys Asn Ser Glu Lys Gln Leu Ala Thr Val Lys Pro Glu Leu Gln
           35                  40                  45
   Lys Val Ala Arg Arg Ala Leu Glu Leu Ser Pro Tyr Asp Phe Thr Ile
       50                  55                  60
   Val Gln Gly Ile Arg Thr Val Ala Gln Ser Ala Gln Asn Ile Ala Asn
   65 70 75 80
   Gly Thr Ser Phe Leu Lys Asp Pro Ser Lys Ser Lys His Val Thr Gly
   85 90 95
   Asp Ala Ile Asp Phe Ala Pro Tyr Ile Asn Gly Lys Ile Asp Trp Lys
   100 105 110
   Asp Leu Glu Ala Phe Trp Ala Val Lys Lys Ala Phe Glu Gln Ala Gly
   115 120 125
   Lys Glu Leu Gly Ile Lys Leu Arg Phe Gly Ala Asp Trp Asn Ser Ser
   130 135 140
   Gly Asp Tyr His Asp Glu Ile Asp Arg Gly Thr Tyr Asp Gly Gly His
   145 150 155 160
   Val Glu Leu Val
<210> 144
   <211> 165
   <212> PRT
   <213> Artificial Sequence
<220>
   <223> Fusion Lycotoxin1-S394
<400> 144
   Met Ile Trp Leu Thr Ala Leu Lys Phe Leu Gly Lys His Ala Ala Lys
   1               5                   10                  15
   Lys Leu Ala Lys Gln Gln Leu Ser Lys Leu Gly Ser Met Ser Phe Lys
               20                  25                  30
   Phe Gly Lys Asn Ser Glu Lys Gln Leu Ala Thr Val Lys Pro Glu Leu
           35                  40                  45
   Gln Lys Val Ala Arg Arg Ala Leu Glu Leu Ser Pro Tyr Asp Phe Thr
       50                  55                  60
   Ile Val Gln Gly Ile Arg Thr Val Ala Gln Ser Ala Gln Asn Ile Ala
   65                  70                  75                  80
   Asn Gly Thr Ser Phe Leu Lys Asp Pro Ser Lys Ser Lys His Val Thr
                   85                  90                  95
   Gly Asp Ala Ile Asp Phe Ala Pro Tyr Ile Asn Gly Lys Ile Asp Trp
               100                 105                 110
   Lys Asp Leu Glu Ala Phe Trp Ala Val Lys Lys Ala Phe Glu Gln Ala
           115                 120                 125
   Gly Lys Glu Leu Gly Ile Lys Leu Arg Phe Gly Ala Asp Trp Asn Ser
       130                 135                 140
   Ser Gly Asp Tyr His Asp Glu Ile Asp Arg Gly Thr Tyr Asp Gly Gly
   145                 150                 155                 160
   His Val Glu Leu Val
                   165
<210> 145
   <211> 173
   <212> PRT
   <213> Artificial Sequence
<220>
   <223> Fusion Lycotoxin1-S394-His6
<400> 145
   Met Ile Trp Leu Thr Ala Leu Lys Phe Leu Gly Lys His Ala Ala Lys
   1               5                   10                  15
   Lys Leu Ala Lys Gln Gln Leu Ser Lys Leu Gly Ser Met Ser Phe Lys
               20                  25                  30
   Phe Gly Lys Asn Ser Glu Lys Gln Leu Ala Thr Val Lys Pro Glu Leu
           35                  40                  45
   Gln Lys Val Ala Arg Arg Ala Leu Glu Leu Ser Pro Tyr Asp Phe Thr
       50                  55                  60
   Ile Val Gln Gly Ile Arg Thr Val Ala Gln Ser Ala Gln Asn Ile Ala
   65                  70                  75                  80
   Asn Gly Thr Ser Phe Leu Lys Asp Pro Ser Lys Ser Lys His Val Thr
                   85                  90                  95
   Gly Asp Ala Ile Asp Phe Ala Pro Tyr Ile Asn Gly Lys Ile Asp Trp
               100                 105                 110
   Lys Asp Leu Glu Ala Phe Trp Ala Val Lys Lys Ala Phe Glu Gln Ala
           115                 120                 125
   Gly Lys Glu Leu Gly Ile Lys Leu Arg Phe Gly Ala Asp Trp Asn Ser
       130                 135                 140
   Ser Gly Asp Tyr His Asp Glu Ile Asp Arg Gly Thr Tyr Asp Gly Gly
   145                 150                 155                 160
   His Val Glu Leu Val Leu Glu His His His His His His
                   165                 170
<210> 146
   <211> 364
   <212> PRT
   <213> Artificial Sequence
<220>
   <223> Fusion MSI-78-OBPgp279 w/o methionine
<400> 146
   Gly Ile Gly Lys Phe Leu Lys Lys Ala Lys Lys Phe Gly Lys Ala Phe
   1               5                   10                  15
   Val Lys Ile Leu Lys Lys Gly Gly Gly Gly Ser Gly Ser Lys Asn Ser
               20                  25                  30
   Glu Lys Asn Ala Ser Ile Ile Met Ser Ile Gln Arg Thr Leu Ala Ser
           35                  40                  45
   Leu Ser Leu Tyr Gly Gly Arg Ile Asp Gly Leu Phe Gly Glu Lys Cys
       50                  55                  60
   Arg Gly Ala Ile Ile Leu Met Leu Asn Lys Val Tyr Pro Asn Phe Ser
   65                  70                  75                  80
   Thr Asn Lys Leu Pro Ser Asn Thr Tyr Glu Ala Glu Ser Val Phe Thr
                   85                  90                  95
   Phe Leu Gln Thr Ala Leu Ala Gly Val Gly Leu Tyr Thr Ile Thr Ile
               100                 105                 110
   Asp Gly Lys Trp Gly Gly Thr Ser Gln Gly Ala Ile Asp Ala Leu Val
           115                 120                 125
   Lys Ser Tyr Arg Gln Ile Thr Glu Ala Glu Arg Ala Gly Ser Thr Leu
       130                 135                 140
   Pro Leu Gly Leu Ala Thr Val Met Ser Lys His Met Ser Ile Glu Gln
   145                 150                 155                 160
   Leu Arg Ala Met Leu Pro Thr Asp Arg Gln Gly Tyr Ala Glu Val Tyr
                   165                 170                 175
   Ile Asp Pro Leu Asn Glu Thr Met Asp Ile Phe Glu Ile Asn Thr Pro
               180                 185                 190
   Leu Arg Ile Ala His Phe Met Ala Gln Ile Leu His Glu Thr Ala Cys
           195                 200                 205
   Phe Lys Tyr Thr Glu Glu Leu Ala Ser Gly Lys Ala Tyr Glu Gly Arg
       210                 215                 220
   Ala Asp Leu Gly Asn Thr Arg Pro Gly Asp Gly Pro Leu Phe Lys Gly
   225                 230                 235                 240
   Arg Gly Leu Leu Gln Ile Thr Gly Arg Leu Asn Tyr Val Lys Cys Gln
                   245                 250                 255
   Val Tyr Leu Arg Glu Lys Leu Lys Asp Pro Thr Phe Asp Ile Thr Ser
               260                 265                 270
   Ser Val Thr Cys Ala Gln Gln Leu Ser Glu Ser Pro Leu Leu Ala Ala
           275                 280                 285
   Leu Ala Ser Gly Tyr Phe Trp Arg Phe Ile Lys Pro Lys Leu Asn Glu
       290                 295                 300
   Thr Ala Asp Lys Asp Asp Ile Tyr Trp Val Ser Val Tyr Val Asn Gly
   305                 310                 315                 320
   Tyr Ala Lys Gln Ala Asn Pro Tyr Tyr Pro Asn Arg Asp Lys Glu Pro
                   325                 330                 335
   Asn His Met Lys Glu Arg Val Gln Met Leu Ala Val Thr Lys Lys Ala
               340                 345                 350
   Leu Gly Ile Val Leu Glu His His His His His His
           355                 360
<210> 147
   <211> 357
   <212> PRT
   <213> Artificial Sequence
<220>
   <223> Fusion MSI-78-OBPgp279
<400> 147
   Met Gly Ile Gly Lys Phe Leu Lys Lys Ala Lys Lys Phe Gly Lys Ala
   1               5                   10                  15
   Phe Val Lys Ile Leu Lys Lys Gly Gly Gly Gly Ser Gly Ser Lys Asn
               20                  25                  30
   Ser Glu Lys Asn Ala Ser Ile Ile Met Ser Ile Gln Arg Thr Leu Ala
           35                  40                  45
   Ser Leu Ser Leu Tyr Gly Gly Arg Ile Asp Gly Leu Phe Gly Glu Lys
       50                  55                  60
   Cys Arg Gly Ala Ile Ile Leu Met Leu Asn Lys Val Tyr Pro Asn Phe
   65                  70                  75                  80
   Ser Thr Asn Lys Leu Pro Ser Asn Thr Tyr Glu Ala Glu Ser Val Phe
                   85                  90                  95
   Thr Phe Leu Gln Thr Ala Leu Ala Gly Val Gly Leu Tyr Thr Ile Thr
               100                 105                 110
   Ile Asp Gly Lys Trp Gly Gly Thr Ser Gln Gly Ala Ile Asp Ala Leu
           115                 120                 125
   Val Lys Ser Tyr Arg Gln Ile Thr Glu Ala Glu Arg Ala Gly Ser Thr
       130                 135                 140
   Leu Pro Leu Gly Leu Ala Thr Val Met Ser Lys His Met Ser Ile Glu
   145                 150                 155                 160
   Gln Leu Arg Ala Met Leu Pro Thr Asp Arg Gln Gly Tyr Ala Glu Val
                   165                 170                 175
   Tyr Ile Asp Pro Leu Asn Glu Thr Met Asp Ile Phe Glu Ile Asn Thr
               180                 185                 190
   Pro Leu Arg Ile Ala His Phe Met Ala Gln Ile Leu His Glu Thr Ala
           195                 200                 205
   Cys Phe Lys Tyr Thr Glu Glu Leu Ala Ser Gly Lys Ala Tyr Glu Gly
       210                 215                 220
   Arg Ala Asp Leu Gly Asn Thr Arg Pro Gly Asp Gly Pro Leu Phe Lys
   225                 230                 235                 240
   Gly Arg Gly Leu Leu Gln Ile Thr Gly Arg Leu Asn Tyr Val Lys Cys
                   245                 250                 255
   Gln Val Tyr Leu Arg Glu Lys Leu Lys Asp Pro Thr Phe Asp Ile Thr
               260                 265                 270
   Ser Ser Val Thr Cys Ala Gln Gln Leu Ser Glu Ser Pro Leu Leu Ala
   275 280 285
   Ala Leu Ala Ser Gly Tyr Phe Trp Arg Phe Ile Lys Pro Lys Leu Asn
   290 295 300
   Glu Thr Ala Asp Lys Asp Asp Ile Tyr Trp Val Ser Val Tyr Val Asn
   305 310 315 320
   Gly Tyr Ala Lys Gln Ala Asn Pro Tyr Tyr Pro Asn Arg Asp Lys Glu
   325 330 335
   Pro Asn His Met Lys Glu Arg Val Gln Met Leu Ala Val Thr Lys Lys
   340 345 350
   Ala Leu Gly Ile Val
   355
<210> 148
   <211> 365
   <212> PRT
   <213> Artificial Sequence
<220>
   <223> Fusion MSI-78-OBPgp279-His6
<400> 148
   Met Gly Ile Gly Lys Phe Leu Lys Lys Ala Lys Lys Phe Gly Lys Ala
   1 5 10 15
   Phe Val Lys Ile Leu Lys Lys Gly Gly Gly Gly Ser Gly Ser Lys Asn
   20 25 30
   Ser Glu Lys Asn Ala Ser Ile Ile Met Ser Ile Gln Arg Thr Leu Ala
   35 40 45
   Ser Leu Ser Leu Tyr Gly Gly Arg Ile Asp Gly Leu Phe Gly Glu Lys
   50 55 60
   Cys Arg Gly Ala Ile Ile Leu Met Leu Asn Lys Val Tyr Pro Asn Phe
   65 70 75 80
   Ser Thr Asn Lys Leu Pro Ser Asn Thr Tyr Glu Ala Glu Ser Val Phe
   85 90 95
   Thr Phe Leu Gln Thr Ala Leu Ala Gly Val Gly Leu Tyr Thr Ile Thr
   100 105 110
   Ile Asp Gly Lys Trp Gly Gly Thr Ser Gln Gly Ala Ile Asp Ala Leu
           115                 120                 125
   Val Lys Ser Tyr Arg Gln Ile Thr Glu Ala Glu Arg Ala Gly Ser Thr
       130                 135                 140
   Leu Pro Leu Gly Leu Ala Thr Val Met Ser Lys His Met Ser Ile Glu
   145                 150                 155                 160
   Gln Leu Arg Ala Met Leu Pro Thr Asp Arg Gln Gly Tyr Ala Glu Val
                   165                 170                 175
   Tyr Ile Asp Pro Leu Asn Glu Thr Met Asp Ile Phe Glu Ile Asn Thr
               180                 185                 190
   Pro Leu Arg Ile Ala His Phe Met Ala Gln Ile Leu His Glu Thr Ala
           195                 200                 205
   Cys Phe Lys Tyr Thr Glu Glu Leu Ala Ser Gly Lys Ala Tyr Glu Gly
       210                 215                 220
   Arg Ala Asp Leu Gly Asn Thr Arg Pro Gly Asp Gly Pro Leu Phe Lys
   225                 230                 235                 240
   Gly Arg Gly Leu Leu Gln Ile Thr Gly Arg Leu Asn Tyr Val Lys Cys
                   245                 250                 255
   Gln Val Tyr Leu Arg Glu Lys Leu Lys Asp Pro Thr Phe Asp Ile Thr
               260                 265                 270
   Ser Ser Val Thr Cys Ala Gln Gln Leu Ser Glu Ser Pro Leu Leu Ala
           275                 280                 285
   Ala Leu Ala Ser Gly Tyr Phe Trp Arg Phe Ile Lys Pro Lys Leu Asn
       290                 295                 300
   Glu Thr Ala Asp Lys Asp Asp Ile Tyr Trp Val Ser Val Tyr Val Asn
   305                 310                 315                 320
   Gly Tyr Ala Lys Gln Ala Asn Pro Tyr Tyr Pro Asn Arg Asp Lys Glu
                   325                 330                 335
   Pro Asn His Met Lys Glu Arg Val Gln Met Leu Ala Val Thr Lys Lys
               340                 345                 350
   Ala Leu Gly Ile Val Leu Glu His His His His His His
           355                 360                 365
<210> 149
   <211> 408
   <212> **DNA**
   <213> Bacteriophage S394
<400> 149
   agcttcaaat tcggcaaaaa cagcgaaaaa cagctggcaa ccgttaaacc ggaactgcag        60
   aaagttgcac gtcgtgcact ggaactgagc ccgtatgatt ttaccattgt tcagggtatt       120
   cgtaccgttg cacagagcgc acagaatatt gcaaatggca ccagctttct gaaagatccg       180
   agcaaaagca aacatgttac cggtgatgca attgattttg caccgtatat taacggcaaa       240
   atcgattgga aagatctgga agcattttgg gcagtgaaaa aagcatttga acaggcaggt       300
   aaagaactgg gtattaaact gcgttttggt gcagattgga atagcagcgg tgattatcat       360
   gatgaaattg atcgtggcac ctatgatggt ggtcatgttg aactggtt                    408
<210> 150
   <211> 66
   <212> **DNA**
   <213> Artificial sequence
<220>
   <223> MSI-78 **DNA**
<400> 150
   ggcattggca aatttctgaa aaaagcgaaa aaatttggca aagcgtttgt gaaaattctg        60
   aaaaaa                                                                   66
<210> 151
   <211> 90
   <212> DNA
   <213> Artificial Sequence
<220>
   <223> nucleic acid encoding Cathelicidin-BF peptide
<400> 151
   aaattttttc gcaaactgaa aaaaagcgtg aaaaaacgcg cgaaagaatt ttttaaaaaa        60
   ccgcgcgtga ttggcgtgag cattccgttt                                         90
<210> 152
   <211> 777
   <212> DNA
   <213> Artificial Sequence
<220>
   <223> mutated KZ144 with C14S, C23S and C50S, without N-terminal methionine
<400> 152
   aaagtattac gcaaaggcga taggggtgat gaggtaagtc aactccagac actcttaaat        60
   ttaagtggct atgatgttgg aaagccagat ggtatttttg gaaataacac ctttaatcag       120
   gtagttaaat ttcaaaaaga taatagtcta gatagtgatg gtattgtagg taagaatact       180
   tgggctgaat tattcagtaa atattctcca cctattcctt ataaaactat ccctatgcca       240
   actgcaaata aatcacgtgc agctgcaact ccagttatga atgcagtaga aaatgctact       300
   ggcgttcgta gccagttgct actaacattt gcttctattg aatcagcatt cgattacgaa       360
   ataaaagcta agacttcatc agctactggt tggttccaat tccttactgg aacatggaaa       420
   acaatgattg aaaattatgg catgaagtat ggcgtactta ctgatccaac tggggcatta       480
   cgtaaagatc cacgtataag tgctttaatg ggtgccgaac taattaaaga gaatatgaat       540
   attcttcgtc ctgtccttaa acgtgaacca actgatactg atctttattt agctcacttc       600
   tttgggcctg gtgcagcccg tcgtttcctg accactggcc agaatgaatt agctgctacc       660
   catttcccaa aagaagctca ggcaaaccca tctatttttt ataacaaaga tgggtcacct       720
   aaaaccattc aagaagttta taacttaatg gatggtaaag ttgcagcaca tagaaaa          777
<210> 153
   <211> 531
   <212> DNA
   <213> Artificial Sequence
<220>
   <223> Fusion MSI-78-GGGGS-S394-His6
<400> 153
   catatgggca ttggcaaatt tctgaaaaaa gcgaaaaaat ttggcaaagc gtttgtgaaa        60
   attctgaaaa aaggcggcgg cggcagcgga tccatgagct tcaaattcgg caaaaacagc       120
   gaaaaacagc tggcaaccgt taaaccggaa ctgcagaaag ttgcacgtcg tgcactggaa       180
   ctgagcccgt atgattttac cattgttcag ggtattcgta ccgttgcaca gagcgcacag       240
   aatattgcaa atggcaccag ctttctgaaa gatccgagca aaagcaaaca tgttaccggt       300
   gatgcaattg attttgcacc gtatattaac ggcaaaatcg attggaaaga tctggaagca       360
   ttttgggcag tgaaaaaagc atttgaacag gcaggtaaag aactgggtat taaactgcgt       420
   tttggtgcag attggaatag cagcggtgat tatcatgatg aaattgatcg tggcacctat       480
   gatggtggtc atgttgaact ggttctcgag caccaccacc accaccactg a                531
<210> 154
   <211> 906
   <212> DNA
   <213> Artificial sequence
<220>
   <223> Fusion Cathelicidin-BF-KZ144(C14S, C23S, C50S)-His6
<400> 154
   catatgaaat tttttcgcaa actgaaaaaa agcgtgaaaa aacgcgcgaa agaatttttt      60
   aaaaaaccgc gcgtgattgg cgtgagcatt ccgtttggat ccaaagtatt acgcaaaggc     120
   gataggggtg atgaggtaag tcaactccag acactcttaa atttaagtgg ctatgatgtt     180
   ggaaagccag atggtatttt tggaaataac acctttaatc aggtagttaa atttcaaaaa     240
   gataatagtc tagatagtga tggtattgta ggtaagaata cttgggctga attattcagt     300
   aaatattctc cacctattcc ttataaaact atccctatgc caactgcaaa taaatcacgt     360
   gcagctgcaa ctccagttat gaatgcagta gaaaatgcta ctggcgttcg tagccagttg     420
   ctactaacat ttgcttctat tgaatcagca ttcgattacg aaataaaagc taagacttca     480
   tcagctactg gttggttcca attccttact ggaacatgga aaacaatgat tgaaaattat     540
   ggcatgaagt atggcgtact tactgatcca actggggcat tacgtaaaga tccacgtata     600
   agtgctttaa tgggtgccga actaattaaa gagaatatga atattcttcg tcctgtcctt     660
   aaacgtgaac caactgatac tgatctttat ttagctcact tctttgggcc tggtgcagcc     720
   cgtcgtttcc tgaccactgg ccagaatgaa ttagctgcta cccatttccc aaaagaagct     780
   caggcaaacc catctatttt ttataacaaa gatgggtcac ctaaaaccat tcaagaagtt     840
   tataacttaa tggatggtaa agttgcagca catagaaaac tcgagcacca ccaccaccac     900
   cactga                                                                906